# EUROPEAN PATENT SPECIFICATION

(11) **EP 3 585 163 B1**
(45) Date of publication and mention of the grant of the patent: **09.03.2022**
(21) Application number: 18808600.3
(22) Date of filing: 09.11.2018
(51) Int. Cl.: A01K 67/027, C07K 14/47

(54) **NON-HUMAN ANIMALS COMPRISING SLC30A8 MUTATION AND METHODS OF USE**
NICHTMENSCHLICHE TIERE MIT SLC30A8-MUTATION UND VERFAHREN ZUR VERWENDUNG
ANIMAUX NON HUMAINS COMPRENANT UNE MUTATION DE SLC30A8 ET PROCÉDÉS D'UTILISATION

(30) Priority: 10.11.2017 US 201762584228 P; 03.05.2018 US 201862666337 P; 26.06.2018 US 201862689945 P
(43) Date of publication of application: 01.01.2020
(73) Proprietor: Regeneron Pharmaceuticals, Inc., Tarrytown, NY 10591 (US)
(72) Inventor: KLEINER, Sandra, Tarrytown NY 10591 (US); ROJAS, Jose, F., Tarrytown NY 10591 (US); GROMADA, Jesper, Concord, Massachusetts 01742 (US)
(74) Representative: Witthoff Jaekel Steinecke Patentanwälte PartG mbB
(86) International application number: PCT/US2018/060052
(87) International publication number: WO 2019/094735

(56) References cited:
- WO-A1-2016/100857
- WO-A2-2008/096009
- GABRIELA DA SILVA XAVIER ET AL: "Animal Models of GWAS-Identified Type 2 Diabetes Genes", JOURNAL OF DIABETES RESEARCH, vol. 2013, 1 January 2013 (2013-01-01), pages 1-12, XP055539548, ISSN: 2314-6745, DOI: 10.1155/2013/906590
- MARIE-CHRISTINE BIRLING ET AL: "Modeling human disease in rodents by CRISPR/Cas9 genome editing", MAMMALIAN GENOME, vol. 28, no. 7-8, 4 July 2017 (2017-07-04) , pages 291-301, XP055539696, US ISSN: 0938-8990, DOI: 10.1007/s00335-017-9703-x
- SANDRA KLEINER ET AL: "Mice harboring the human SLC30A8 R138X loss-of-function mutation have increased insulin secretory capacity", PROCEEDINGS OF THE NATIONAL ACADEMY OF SCIENCES OF THE UNITED STATES OF AMERICA, vol. 115, no. 32, 23 July 2018 (2018-07-23), pages E7642-E7649, XP055539058, US ISSN: 0027-8424, DOI: 10.1073/pnas.1721418115

## Description

### CROSS-REFERENCE TO RELATED APPLICATIONS

This application claims the benefit of US Application No. 62/584,228, filed November 10, 2017, US Application No. 62/666,337, filed May 3, 2018, and US Application No. 62/689,945, filed June 26, 2018.

### REFERENCE TO A SEQUENCE LISTING SUBMITTED AS A TEXT FILE VIA EFS WEB

The Sequence Listing written in file 523060SEQLIST.txt is 39.2 kilobytes, was created on November 5, 2018.

### BACKGROUND

Diabetes is a disorder characterized by metabolic defects in insulin-responsive tissues and insulin-producing pancreatic beta cells resulting in a failure to maintain appropriate blood sugar levels in the body. Diabetes in humans can be defined as a disorder corresponding to a fasting plasma glucose concentration greater than 125 mg/dL, or a plasma glucose concentration greater than 199 mg/dL two hours after ingestion of a 75 g oral glucose load. Two major forms of diabetes are type 1 diabetes (T1D) and type 2 diabetes (T2D). T1D is an autoimmune disorder resulting in destruction of beta-pancreatic cells and an absolute deficiency of insulin, the hormone that regulates glucose utilization. By contrast, T2D can occur with normal or even elevated levels of insulin from the inability of tissues to respond appropriately to insulin. Most T2D patients have impaired insulin sensitivity. Insulin secretion cannot compensate for the resistance of peripheral tissues to respond to insulin. Many T2D patients are obese. Type 1.5 diabetes (late autoimmune onset in adults) shows some characteristics of T1D and T2D.

Zinc is required for insulin biosynthesis and processing. Two zinc ions are complexed in a hexameric form of proinsulin, which is ultimately processed to insulin.

*SLC30A8* encodes a zinc transporter that is primarily expressed in the pancreatic islet, where it transports zinc into insulin-containing secretory granules. Heterozygous loss-of-function (LOF) mutations in *SLC30A8* protect from type 2 diabetes in humans. Several *Slc30a8* knockout mouse lines have been characterized, but they do not fully recapitulate the protective human phenotype. Rather, *Slc30a8*-deficient mice have reduced plasma insulin levels and impaired glucose tolerance.

### SUMMARY

The present invention relates to the embodiments as characterized in the appended claims. In particular, non-human animals comprising a mutated *Slc30a8* locus are provided, as well as methods of making and using such non-human animals, wherein the non-human animal is a rodent. Non-human animal, *i.e.* rodent genomes or cells comprising a mutated *Slc30a8* locus are also provided.

Such non-human animal genomes, non-human animal cells, or non-human animals comprise a mutated *Slc30a8* gene, wherein the mutated *Slc30a8* gene encodes a truncated SLC30A8 protein and results in the non-human animal having an enhanced capacity for insulin secretion relative to a non-human animal without the mutation.

In particular, provided are non-human animals whose genome comprises an endogenous *Slc30a8* locus comprising a mutated *Slc30a8* gene, wherein the mutated *Slc30a8* gene encodes a truncated SLC30A8 protein and results in the non-human animal having an enhanced capacity for insulin secretion relative to a non-human animal without the mutation.

In some such non-human animals, the non-human animals exhibit enhanced capacity for insulin secretion in response to hyperglycemia. Optionally, the non-human animal has increased insulin secretion in response to hyperglycemia induced by insulin receptor inhibition relative to the non-human animal without the mutation. Optionally, the increased insulin secretion in response to hyperglycemia induced by insulin receptor inhibition is not associated with increased beta-cell proliferation or beta-cell mass relative to the non-human animal without the mutation.

In some such non-human animals, the enhanced capacity for insulin secretion is observed when the non-human animals are fed a high-fat diet. Optionally, the non-human animal has increased insulin secretion relative to the non-human animal without the mutation when fed a high-fat diet, wherein the increased insulin secretion is associated with increased beta-cell proliferation or beta-cell mass relative to the non-human animal without the mutation. Optionally, the increased beta-cell proliferation is insulin-receptor-dependent (e.g., dependent on an insulin receptor in the beta cells).

In such non-human animals, non-human animal cells, or non-human animal genomes, the mutated *Slc30a8* gene has a premature termination codon. In such non-human animals, non-human animal cells, or non-human animal genomes, the mutated *Slc30a8* gene comprises a mutation is in the third exon of the *Slc30a8* gene, which is at the 3' end of the third exon of the *Slc30a8* gene.

In such non-human animals, non-human animal cells, or non-human animal genomes, the mutated *Slc30a8* gene comprises a nonsense mutation. Optionally, the nonsense mutation is in a codon corresponding to the codon encoding R138 in SEQ ID NO: 14 when the SLC30A8 protein encoded by the mutated *Slc30a8* gene is optimally aligned with SEQ ID NO: 14. Optionally, the nonsense mutation is at a position corresponding to residue 412 in SEQ ID NO: 21 when the coding sequence of the mutated *Slc30a8* gene is optimally aligned with SEQ ID NO: 21.

In such non-human animals, non-human animal cells, or non-human animal genomes, the mutated *Slc30a8* gene is endogenous to the non-human animal. In some such non-human animals, non-human animal cells, or non-human animal genomes, the non-human animal is a rat or a mouse. Optionally, the non-human animal is a mouse. Optionally, the mutated *Slc30a8* gene encodes a SLC30A8 protein comprising the sequence set forth in SEQ ID NO: 13. Optionally, the mutated *Slc30a8* gene comprises the coding sequence set forth in SEQ ID NO: 22 or degenerates thereof that encode the same amino acid sequence.

Some such non-human animals have increased insulin secretion in response to hyperglycemia induced by insulin receptor inhibition relative to the non-human animal without the mutation. In some such non-human animals, the increased insulin secretion is not associated with increased beta-cell proliferation or beta-cell mass relative to the non-human animal without the mutation. Some such non-human animals have decreased mitochondrial gene expression relative to the non-human animal without the mutation. Some such non-human animals have increased *Hvcn1* expression relative to the non-human animal without the mutation. In some such non-human animals, the non-human animal has normal glucose homeostasis and glucose-induced insulin secretion on a control chow diet relative to the non-human animal without the mutation. In some such non-human animals, the non-human animal has a normal metabolic phenotype on a control chow diet relative to the non-human animal without the mutation.

Some such non-human animals have one or more of the following characteristics relative to the non-human animal without the mutation: (a) increased glucose-induced insulin secretion when fed the high-fat diet; (b) increased pancreatic beta-cell proliferation when fed the high-fat diet; (c) increased number of pancreatic beta cells when fed the high-fat diet; and (d) increased fed plasma insulin levels after blockade of the insulin receptor. Optionally, the non-human animal has all of the following characteristics relative to the non-human animal without the mutation: (a) increased glucose-induced insulin secretion when fed the high-fat diet; (b) increased pancreatic beta-cell proliferation when fed the high-fat diet; (c) increased number of pancreatic beta cells when fed the high-fat diet; and (d) increased fed plasma insulin levels after blockade of the insulin receptor.

Some such non-human animals have one or more of the following characteristics relative to the non-human animal without the mutation: (a) increased circulating insulin levels after fed the high-fat diet for 20 weeks; (b) increased number of pancreatic beta cells after fed the high-fat diet for 20 weeks; (c) decreased proinsulin-to-insulin ratio when fed the high-fat diet; and (d) increased fed plasma insulin levels after blockade of the insulin receptor. Optionally, the non-human animal has all of the following characteristics relative to the non-human animal without the mutation: (a) increased circulating insulin levels after fed the high-fat diet for 20 weeks; (b) increased number of pancreatic beta cells after fed the high-fat diet for 20 weeks; (c) decreased proinsulin-to-insulin ratio when fed the high-fat diet; and (d) increased fed plasma insulin levels after blockade of the insulin receptor.

In some such non-human animals, *Slc30a8* mRNA expression levels in the islets of the non-human animal are at least 25% of *Slc30a8* mRNA expression levels in the islets of the non-human animal without the mutation.

Some such non-human animals, non-human animal cells, or non-human animal genomes are heterozygous for the mutation. Some such non-human animals, non-human animal cells, or non-human animal genomes are homozygous for the mutation. Some such non-human animals, non-human animal cells, or non-human animal genomes are male. Some such non-human animals, non-human animal cells, or non-human animal genomes are female.

In another aspect, provided are methods for making any of the above non-human animals, *i.e.* rodents. Some such methods comprise: (a) contacting the genome of a non-human animal pluripotent cell that is not a one-cell stage embryo with: (i) an exogenous repair template comprising an insert nucleic acid flanked by a 5' homology arm that hybridizes to a 5' target sequence at the *Slc30a8* locus and a 3' homology arm that hybridizes to a 3' target sequence at the *Slc30a8* locus, wherein the insert nucleic acid comprises the mutation; and (ii) a Cas9 protein; and (iii) a guide RNA that hybridizes to a guide RNA recognition sequence within the *Slc30a8* locus, wherein the *Slc30a8* gene is modified to comprise the mutation; and (b) introducing the modified non-human animal pluripotent cell into a host embryo; and (c) producing from the host embryo implanted into a surrogate mother a genetically modified F0 generation non-human animal in which the *Slc30a8* gene is modified to comprise the mutation, wherein the mutation results in the F0 generation non-human animal having an enhanced capacity for insulin secretion relative to a non-human animal without the mutation when fed the high-fat diet. Some such methods comprise: (a) contacting the genome of a non-human animal pluripotent cell that is not a one-cell stage embryo with: (i) an exogenous repair template comprising an insert nucleic acid flanked by a 5' homology arm that hybridizes to a 5' target sequence at the *Slc30a8* locus and a 3' homology arm that hybridizes to a 3' target sequence at the *Slc30a8* locus, wherein the insert nucleic acid comprises the mutation; and (ii) a Cas9 protein; and (iii) a guide RNA that hybridizes to a guide RNA recognition sequence within the *Slc30a8* locus, wherein the *Slc30a8* gene is modified to comprise the mutation; and (b) introducing the modified non-human animal pluripotent cell into a host embryo; and (c) gestating the host embryo in a surrogate mother to produce a genetically modified F0 generation non-human animal in which the *Slc30a8* gene is modified to comprise the mutation, wherein the mutation results in the F0 generation non-human animal having an enhanced capacity for insulin secretion relative to a non-human animal without the mutation when fed the high-fat diet. Optionally, the pluripotent cell is an embryonic stem cell. Optionally, the exogenous repair template is a large targeting vector that is at least 10 kb in length, or wherein the exogenous repair template is a large targeting vector in which the sum total of the 5' homology arm and the 3' homology arm is at least 10 kb in length.

Some such methods comprise: (a) contacting the genome of a non-human animal one-cell stage embryo with: (i) an exogenous repair template comprising an insert nucleic acid flanked by a 5' homology arm that hybridizes to a 5' target sequence at the *Slc30a8* locus and a 3' homology arm that hybridizes to a 3' target sequence at the *Slc30a8* locus, wherein the insert nucleic acid comprises the mutation; (ii) a Cas9 protein; and (iii) a guide RNA that that hybridizes to a guide RNA recognition sequence within the *Slc30a8* locus, wherein the *Slc30a8* gene is modified to comprise the mutation; and (b) producing from the modified non-human animal one-cell stage embryo implanted into a surrogate mother a genetically modified F0 generation non-human animal in which the *Slc30a8* gene is modified to comprise the mutation, wherein the mutation results in the F0 generation non-human animal having an enhanced capacity for insulin secretion relative to a non-human animal without the mutation when fed the high-fat diet. Some such methods comprise: (a) contacting the genome of a non-human animal one-cell stage embryo with: (i) an exogenous repair template comprising an insert nucleic acid flanked by a 5' homology arm that hybridizes to a 5' target sequence at the *Slc30a8* locus and a 3' homology arm that hybridizes to a 3' target sequence at the *Slc30a8* locus, wherein the insert nucleic acid comprises the mutation; (ii) a Cas9 protein; and (iii) a guide RNA that that hybridizes to a guide RNA recognition sequence within the *Slc30a8* locus, wherein the *Slc30a8* gene is modified to comprise the mutation; and (b) gestating the modified non-human animal one-cell stage embryo in a surrogate mother to produce a genetically modified F0 generation non-human animal in which the *Slc30a8* gene is modified to comprise the mutation, wherein the mutation results in the F0 generation non-human animal having an enhanced capacity for insulin secretion relative to a non-human animal without the mutation when fed the high-fat diet.

In some such methods, step (a) further comprises contacting the genome of the non-human animal pluripotent cell or the non-human one-cell stage embryo with a second guide RNA that hybridizes to a second guide RNA recognition sequence within the *Slc30a8* locus.

In some such methods, the exogenous repair template a single-stranded oligodeoxynucleotide. Optionally, the exogenous repair template is between about 80 nucleotides to about 200 nucleotides in length.

In another aspect, provided are methods of screening compounds for activity for ameliorating or exacerbating type-2-diabetes. Some such methods comprise: (a) contacting any of the above subject non-human animals, *i.e.* rodents with the compound; and (b) measuring one or more of the following in the subject non-human animal relative to a control non-human animal not contacted with the compound, wherein the control non-human animal comprises the same *Slc30a8* mutation as the subject non-human animal: (1) glucose-induced insulin secretion when fed the high-fat diet; (2) pancreatic beta-cell proliferation levels when fed the high-fat diet; (3) number of pancreatic beta cells when fed the high-fat diet; and (4) fed plasma insulin levels after blockade of the insulin receptor, whereby activity for ameliorating type 2 diabetes is identified by one or more of the following in the subject non-human animal compared with the control non-human animal: (1) increased glucose-induced insulin secretion when fed the high-fat diet; (2) increased pancreatic beta-cell proliferation when fed the high-fat diet; (3) increased number of pancreatic beta cells when fed the high-fat diet; and (4) increased fed plasma insulin levels after blockade of the insulin receptor, and whereby activity for exacerbating type 2 diabetes is identified by one or more of the following in the subject non-human animal compared with the control non-human animal: (1) decreased glucose-induced insulin secretion when fed the high-fat diet; (2) decreased pancreatic beta-cell proliferation when fed the high-fat diet; (3) decreased number of pancreatic beta cells when fed the high-fat diet; and (4) decreased fed plasma insulin levels after blockade of the insulin receptor.

Some such methods comprise: (a) contacting any of the above subject non-human animals with the compound; and (b) measuring one or more of the following in the subject non-human animal relative to a control non-human animal not contacted with the compound, wherein the control non-human animal comprises the same *Slc30a8* mutation as the subject non-human animal: (1) capacity to secrete insulin in response to hyperglycemia; (2) insulin clearance; (3) mitochondrial gene expression; and (4) *Hvcn1* expression, whereby activity for ameliorating type 2 diabetes is identified by one or more of the following in the subject non-human animal compared with the control non-human animal: (1) increased capacity to secrete insulin in response to hyperglycemia; (2) increased insulin clearance; (3) decreased mitochondrial gene expression; and (4) increased *Hvcn1* expression, and whereby activity for exacerbating type 2 diabetes is identified by one or more of the following in the subject non-human animal compared with the control non-human animal: (1) decreased capacity to secrete insulin in response to hyperglycemia; (2) decreased insulin clearance; (3) increased mitochondrial gene expression; and (4) decreased *Hvcn1* expression.

In another aspect, provided are non-human animal cells, *i.e.* rodent cells whose genome comprises an endogenous *Slc30a8* locus comprising a mutated *Slc30a8* gene, wherein the mutated *Slc30a8* gene encodes a truncated SLC30A8 protein, and wherein a non-human animal comprising the mutated *Slc30a8* gene has an enhanced capacity for insulin secretion relative to a non-human animal without the mutation.

In another aspect, provided are non-human animal genomes comprising an endogenous *Slc30a8* locus comprising a mutated *Slc30a8* gene, wherein the mutated *Slc30a8* gene encodes a truncated SLC30A8 protein, and wherein a non-human animal comprising the mutated *Slc30a8* gene has an enhanced capacity for insulin secretion relative to a non-human animal without the mutation.

In another aspect, provided are targeting vectors for generating a mutated *Slc30a8* gene at an endogenous *Slc30a8* locus in a non-human animal, wherein the targeting vector comprises a 5' homology arm targeting a 5' target sequence at the endogenous *Slc30a8* locus and a 3' homology arm targeting a 3' target sequence at the endogenous *Slc30a8* locus, wherein the targeting vector comprises a mutation in the *Slc30a8* gene, wherein the mutated *Slc30a8* gene encodes a truncated SLC30A8 protein, and wherein a non-human animal comprising the mutated *Slc30a8* gene has an enhanced capacity for insulin secretion relative to a non-human animal without the mutation.

### BRIEF DESCRIPTION OF THE FIGURES

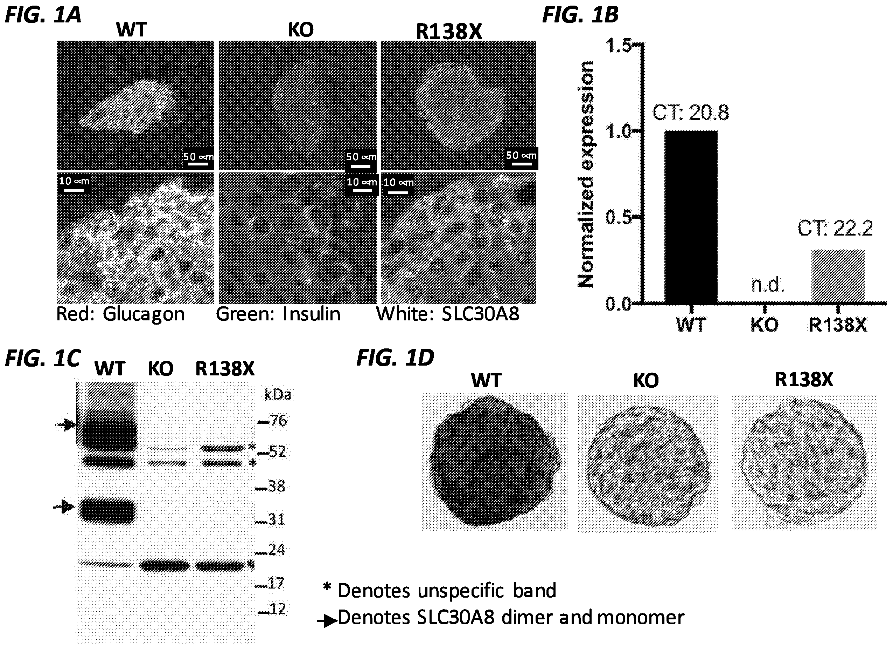

**Figures 1A-1D** show analysis of *Slc30a8* RNA and protein in islets from male R138X mice on chow diet. **Figure 1A** shows *Slc30a8* RNA in situ hybridization of pancreatic islets isolated from wild type (WT), homozygous knockout (KO), and homozygous R138X mice. KO islets were used as negative control. Red: glucagon RNA, green: insulin RNA, white: *Slc30a8* RNA. **Figure 1B** shows quantification of islet *Slc30a8* RNA levels using TAQMAN^{®} analysis. **Figure 1C** shows Western blot of islets isolated from chow-fed WT, KO and R138X mice. KO islets were used as negative control. Arrow: SLC30A8 protein; *denotes unspecific bands. **Figure ID** shows dithizone staining of pancreatic islets isolated from WT, KO, and R138X mice.

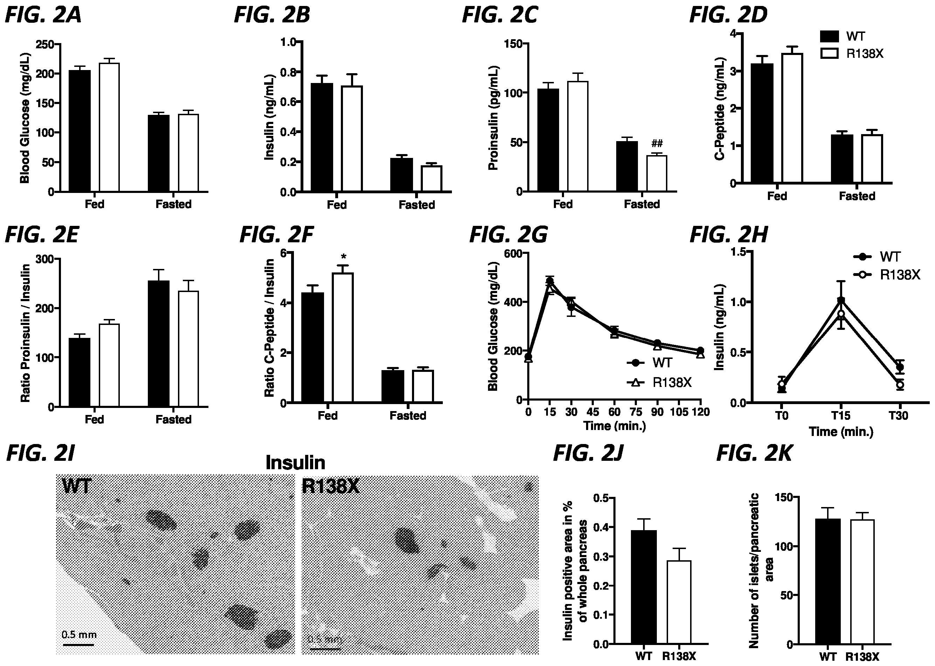

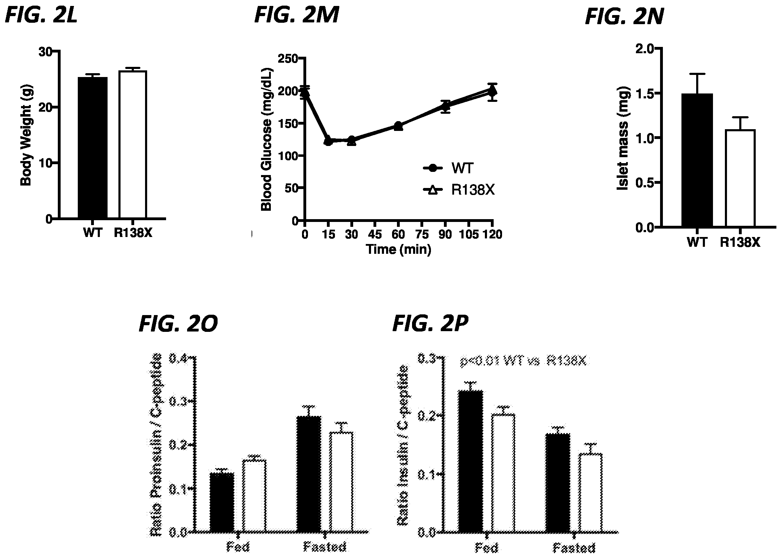

**Figures 2A-2P** show the metabolic phenotype of male homozygous R138X mice on chow diet. **Figures 2A-2D** and **2L** show body weight **(****Figure 2L****)**, blood glucose (**Figure 2A**), plasma insulin **(****Figure 2B****),** proinsulin **(****Figure 2C****),** and C-peptide **(****Figure 2D****)** levels in fed and fasted WT and R138X mice. **Figures 2E-2F** show proinsulin/insulin ratio **(****Figure 2E****)** and C-peptide/insulin ratio **(****Figure 2F****)** in fed and fasted WT and R138X mice. **Figure 2*****G*** shows oral glucose tolerance test in WT and R138X mice. Data are displayed as blood glucose over time. **Figure 2H** shows plasma insulin levels in WT or R138X mice after an overnight fast (time 0) and at the indicated times after an intraperitoneal injection of glucose. Data are displayed as blood glucose levels over time. **Figure 2I** shows histology for insulin in pancreas isolated from WT and R138X mice. **Figure 2J** shows quantification of pancreatic insulin staining. **Figure 2K** shows quantification of islet number. Values represent the means ± SEM (n=6-20 mice per genotype). **Figure 2M** shows results of an insulin tolerance test in 4 hr fasted WT and R138X mice. Data are displayed as blood glucose levels over time (n=6 / genotype). **Figure 2N** shows further quantification of pancreatic insulin staining (islet mass; n=6 / genotype). Values represent the means ± SEM. **Figures 2O-2P** show proinsulin/C-peptide ratio (**Figure 2O**) and insulin/C-peptide ratio **(****Figure 2P****)** in fed and fasted WT and R138X mice on a chow diet. N=18-20 mice per genotype.

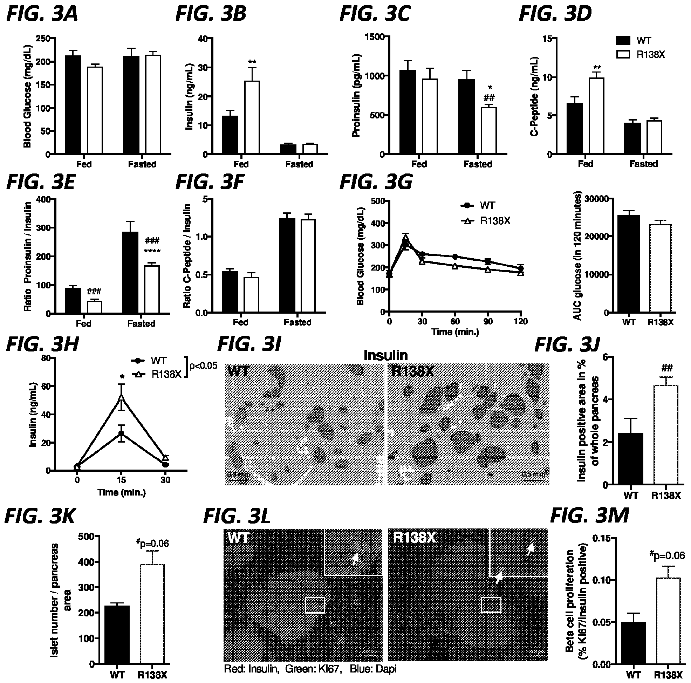

**Figures 3A-3M** show the metabolic phenotype of male homozygous R138X mice on HFD diet. **Figures 3A-3F** show blood glucose **(****Figure 3A****),** plasma insulin **(****Figure 3B****),** proinsulin **(****Figure 3C****),** and C-peptide **(****Figure 3D****)** levels in fed and fasted WT and R138X mice. **Figures 3E-3F** show proinsulin/insulin ratio **(****Figure 3E****)** and C-peptide/insulin ratio **(****Figure 3F****)** in fed and fasted WT and R138X mice. **Figure 3G** shows oral glucose tolerance test in WT and R138X mice. Data are displayed as blood glucose over time. Area under the curve is shown on the right. **Figure 3H** shows blood insulin levels in WT or R138X mice after an overnight fast (time 0) and at the indicated times after an intraperitoneal injection of glucose. Data are displayed as blood glucose levels over time. **Figure 31** shows histology for insulin in pancreas isolated from WT and R138X mice. **Figure 3J** shows quantification of pancreatic insulin staining. **Figure 3K** shows quantification of islet number. **Figure 3L** shows immunohistochemistry for Ki67 (green) and insulin (red). **Figure 3M** shows quantification of Ki67 and insulin double-positive cells. Values represent the means ± SEM (n=4-17 mice per genotype).

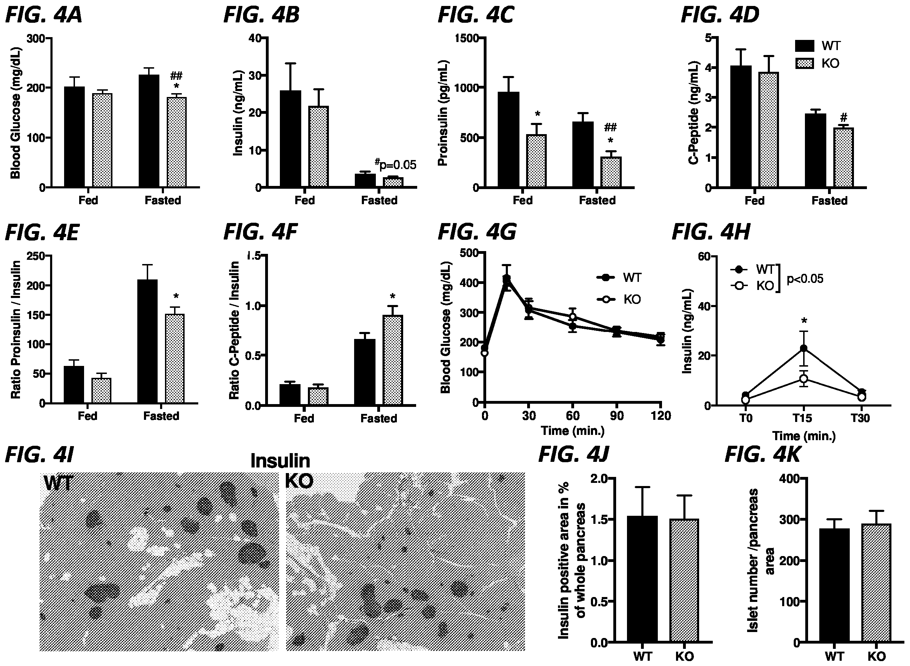

**Figures 4A-4K** show metabolic phenotype of male homozygous *Slc30a8* KO mice on HFD diet. **Figures 4A-4D** show blood glucose **(****Figure 4A****),** plasma insulin **(****Figure 4B****),** proinsulin **(****Figure 4C****),** and C-peptide **(****Figure 4D****)** levels in fed and fasted WT and *Slc30a8* KO mice. **Figures 4E-4F** show proinsulin/insulin ratio **(****Figure 4E****)** and C-peptide/insulin ratio **(****Figure 4F****)** in fed and fasted WT and *Slc30a8* KO mice. **Figure 4G** shows oral glucose tolerance test in WT and *Slc30a8* KO mice. Data are displayed as blood glucose levels over time. **Figure 4H** shows blood insulin levels in WT or *Slc30a8* KO mice after an overnight fast (time 0) and at the indicated times after an intraperitoneal injection of glucose. Data are displayed as glucose levels over time. **Figure 4I** shows histology for insulin in pancreas isolated from WT and *Slc30a8* KO mice. **Figure 4J** shows quantification of pancreatic insulin staining. **Figure 4K** shows quantification of islet number. Values represent the means ± SEM (n=6-17 mice per genotype).

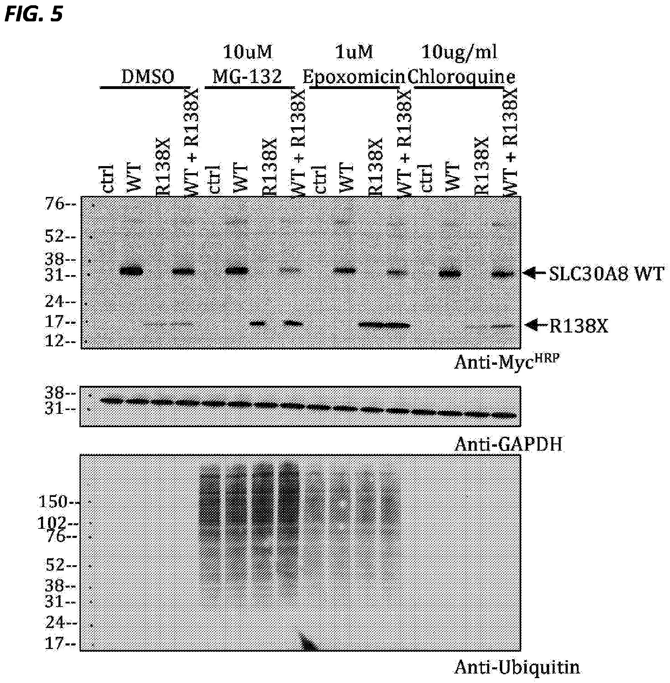

**Figure 5** shows *in vitro* expression and stabilization of human R138X protein by proteasomal inhibition. Western blot analysis of HEK293 lysates is shown after overexpression of either myc-tagged SLC30A8 WT or R138X protein alone or together. Cells were treated for 6 hr with indicated compounds and probed with the respective antibodies.

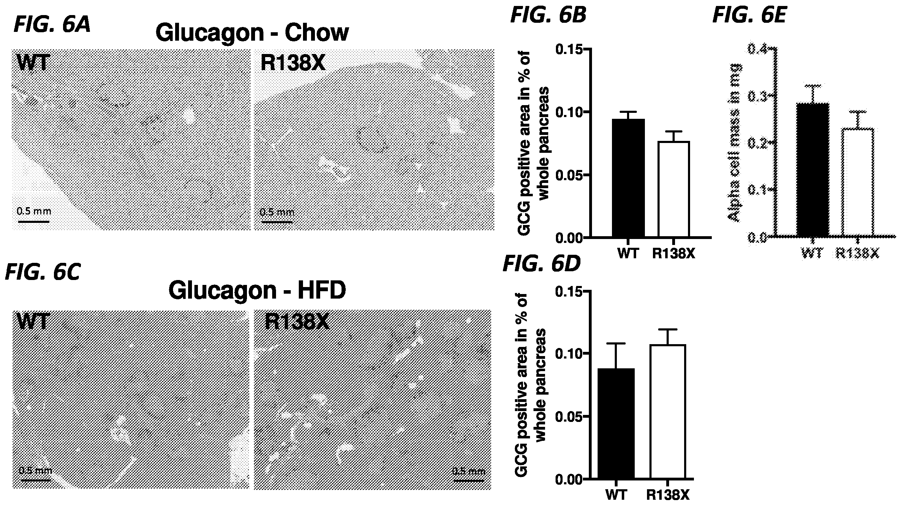

**Figures 6A-6E** show glucagon histology in chow and HFD conditions is unchanged in male homozygous R138X mice. **Figure 6A** shows histology for glucagon in pancreas isolated from chow-fed WT and R138X mice. **Figures 6B** and **6E** show quantification of pancreatic glucagon staining shown in **Figure 6A****.** Values represent the means ± SEM (n= 6 per genotype). **Figure 6C** shows histology for glucagon in pancreas isolated from HFD-fed WT and R138X mice. **Figure 6D** shows quantification of pancreatic glucagon staining shown in **Figure 6C** (n=4-17 mice per genotype).

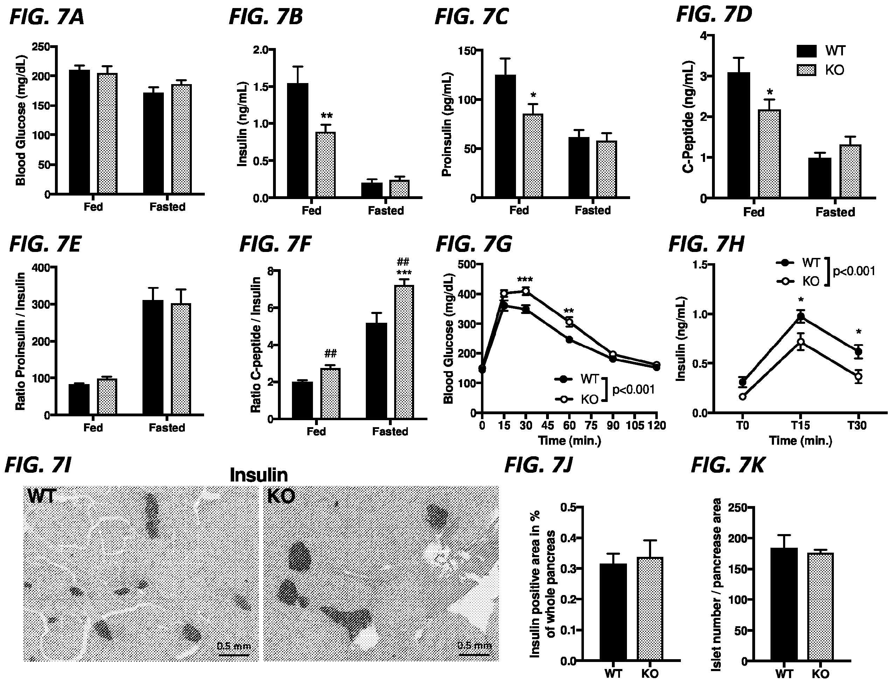

**Figures 7A-7K** show metabolic phenotype of male homozygous *Slc30a8* KO mice on chow diet. **Figures 7A-7D** show blood glucose **(****Figure 7A****),** plasma insulin (**Figure 7B****),** proinsulin **(****Figure 7C****),** and C-peptide (**Figure 7D****)** levels in fed and fasted WT and *Slc30a8* KO mice. **Figures 7E-7F** show proinsulin/insulin ratio **(****Figure 7E****)** and C-peptide/insulin ratio **(****Figure 7F****)** in fed and fasted WT and *Slc30a8* KO mice. **Figure 7G** shows oral glucose tolerance test in WT and *Slc30a8* KO mice. Data are displayed as blood glucose levels over time. **Figure 7H** shows plasma insulin levels in WT or *Slc30a8* KO mice after an overnight fast (time 0) and at the indicated times after an intraperitoneal injection of glucose. Data are displayed as blood glucose levels over time. **Figure 71** shows histology for insulin in pancreas isolated from WT and *Slc30a8* KO mice. **Figure 7J** shows quantification of pancreatic insulin staining. **Figure 7K** shows quantification of islet number. Values represent the means ± SEM (n=6-10 mice per genotype).

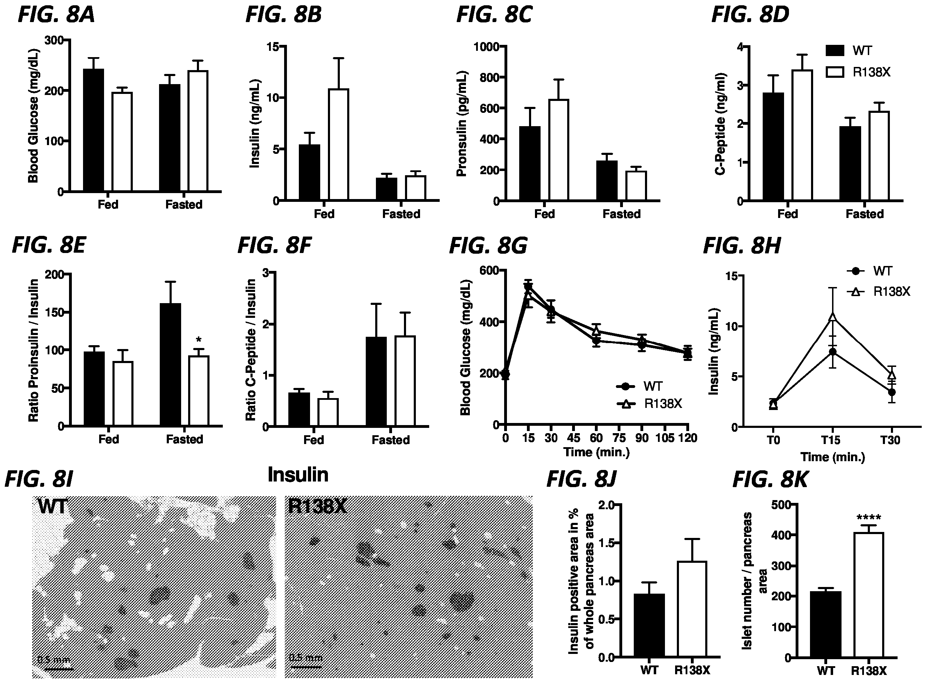

**Figures 8A-8K** show metabolic phenotype of female homozygous R138X mice on HFD. **Figures 8A-8D** show blood glucose **(****Figure 8A****),** plasma insulin (**Figure 8B****),** proinsulin **(****Figure 8C****),** and C-peptide (**Figure 8D****)** levels in fed and fasted WT and R138X mice. **Figures 8E-8F** show proinsulin/insulin ratio **(****Figure 8E****)** and C-peptide/insulin ratio **(****Figure 8F****)** in fed and fasted WT and R138X mice. **Figure 8G** shows oral glucose tolerance test in WT and R138X mice. Data are displayed as glucose levels over time. **Figure 8H** shows plasma insulin levels in WT or R138X mice after an overnight fast (time 0) and at the indicated times after an intraperitoneal injection of glucose. Data are displayed as glucose levels over time. **Figure 8I** shows histology for insulin in pancreas isolated from WT and R138X mice. **Figure 8J** shows quantification of pancreatic insulin staining. **Figure 8K** shows quantification of islet number. Values represent the means ± SEM (n=6-9 mice per genotype).

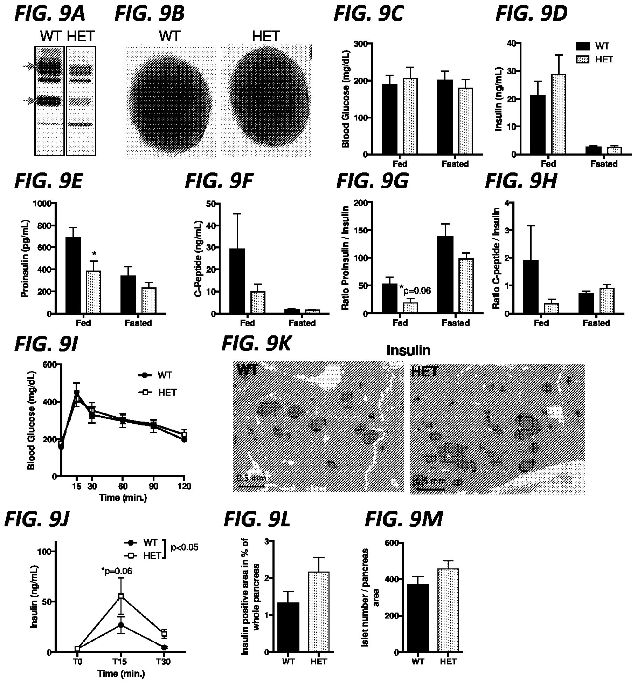

**Figures 9A-9M** show heterozygous R138X (HET) mice on HFD have higher glucose-stimulated insulin levels. **Figure 9A** shows Western blot of islets isolated from chow-fed WT, heterozygous R138X mice. Arrow: SLC30A8 protein; *denotes unspecific bands. **Figure 9B** shows dithizone staining of pancreatic islets isolated from WT and heterozygous R138X mice. **Figures 9C-9F** show blood glucose **(****Figure 9C****),** plasma insulin **(****Figure 9D****),** proinsulin **(****Figure 9E****),** and C-peptide **(****Figure 9F****)** levels in fed and fasted WT and R138X HET mice. **Figures 9G-9H** show proinsulin/insulin ratio **(****Figure 9G****)** and C-peptide/insulin ratio **(****Figure 9H****)** in fed and fasted WT and HET mice. **Figure 9I** shows oral glucose tolerance test in WT and R138X HET mice. Data are displayed as blood glucose levels over time. **Figure 9J** shows plasma insulin levels in WT or R138X HET mice after an overnight fast (time 0) and at the indicated times after an intraperitoneal injection of glucose. Data are displayed as glucose levels over time. **Figure 9K** shows histology for insulin in pancreas isolated from WT and R138X HET mice. **Figure 9L** shows quantification of pancreatic insulin staining. **Figure 9M** shows quantification of islet number. Values represent the means ± SEM (n=6-9 mice per genotype).

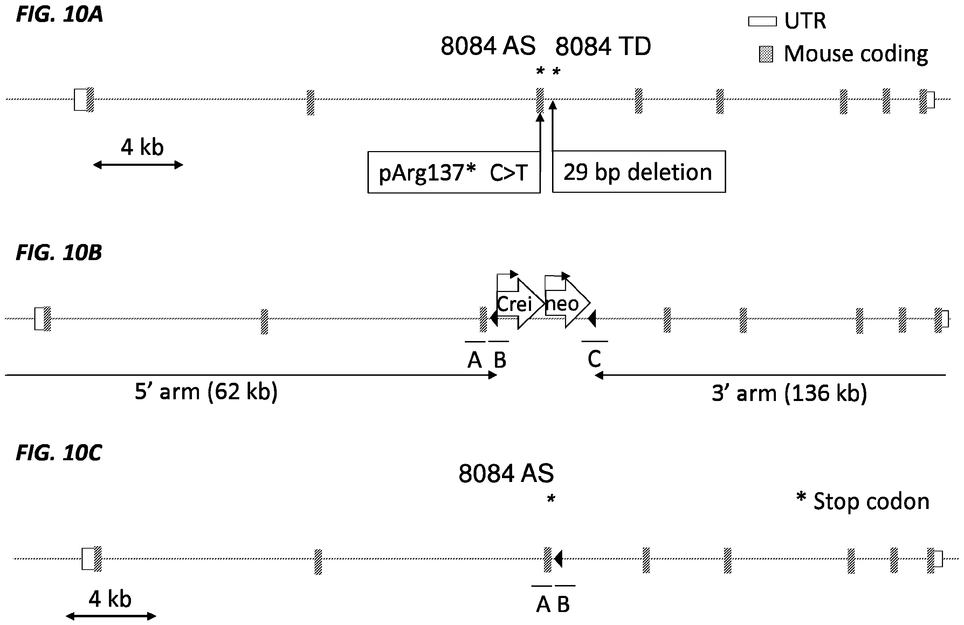

**Figures 10A-10C** show schematics for targeting the mouse *Slc30a8* locus to generate R138X mice. **Figure 10A** shows the wild type mouse locus and indicates the location of the pArg137^{∗} C>T point mutation and the 29 bp deletion that are generated through targeting. An allele-specific screening assay (8084 AS) and a TAQMAN^{®} downstream screening assay (8084 TD) are shown. **Figure 10B** shows a schematic of the targeted locus. **Figure 10C** shows a schematic of the targeted locus after self-excising of the self-deleting drug selection cassette.

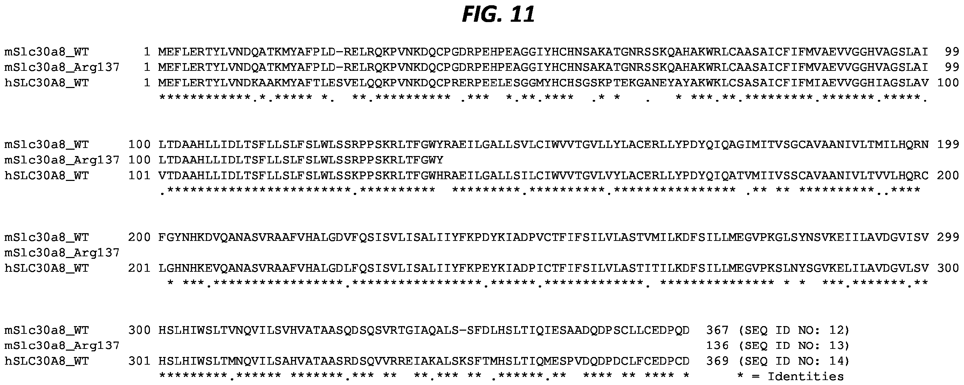

**Figure 11** shows a protein alignment of the wild type mouse SLC30A8 protein (SEQ ID NO: 12), the expected truncated SLC30A8 Arg137^{∗} protein in R138X mice (SEQ ID NO: 13), and the wild type human SLC30A8 protein (SEQ ID NO: 14).

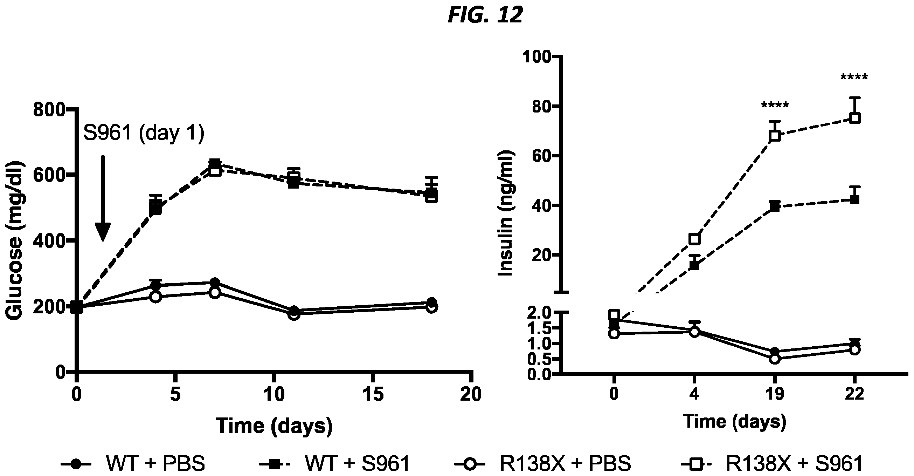

**Figure 12** shows fed plasma glucose levels and fed plasma insulin levels in WT (closed symbols) and homozygous R138X mice (open symbols) treated with either S961 (dashed lines) or PBS (solid lines) using a constant minipump infusion. Fed plasma glucose levels and insulin levels were measured at the indicated times throughout the experiment (21 days).

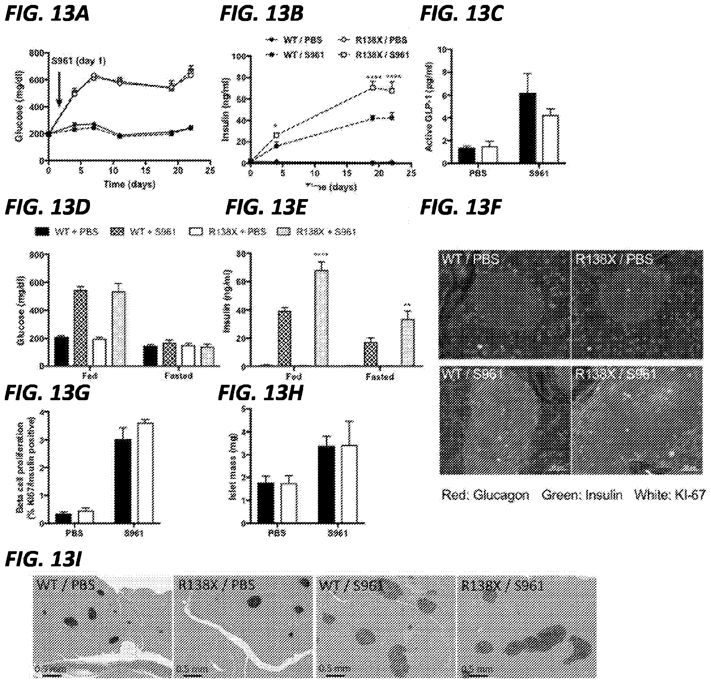

**Figures 13A-13I** show that R138X mice secrete more insulin under chronic hyperglycemia caused by the insulin receptor antagonist S961. **Figure 13A** shows nonfasted plasma glucose levels in R138X and WT mice continuously treated with the insulin receptor antagonist S961 (20 nMol/week) or PBS for 22 days. **Figure 13B** shows nonfasted plasma insulin on days 0, 4, 19 and 22 R138X and WT mice treated with S961 (20 nMol/week) or PBS. **Figure 13C** shows plasma active GLP-1 levels in WT and R138X mice after 22 days of treatment. **Figures 13D** and **13E** show fed and fasted glucose **(****Figure 13D****)** and insulin **(****Figure 13E****)** levels measured day 19 and 20 after initiation of treatment. **Figure 13F** shows immunohistochemistry for KI-67 (white), insulin (green), and glucagon (red). **Figure 13G** shows quantification of KI-67 and insulin double-positive cells. **Figure 13H** shows quantification of pancreatic insulin staining shown in **(****Figure 13I). Figure 13I** shows histology for insulin in pancreas isolated from WT and R138X mice after 22 days of treatment. Values represent the means ± SEM (n=5-7 mice per treatment and genotype).

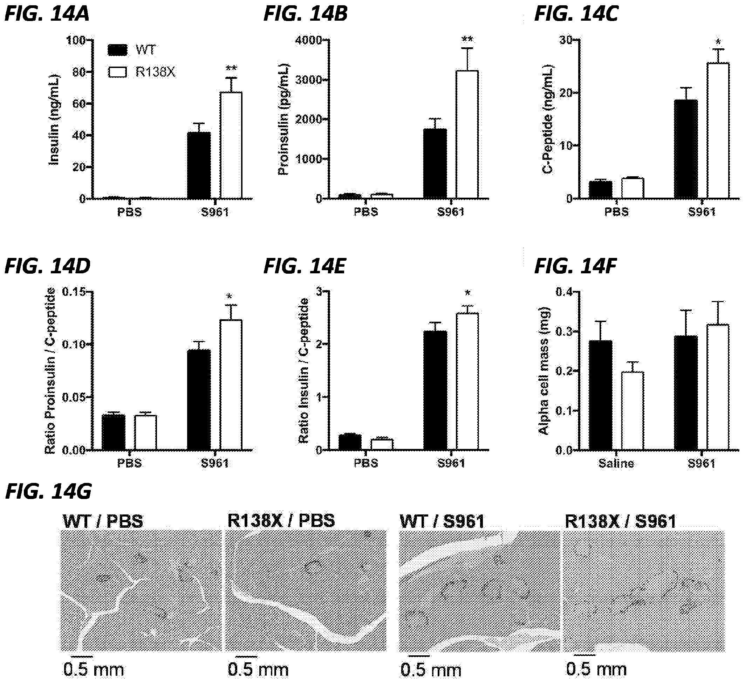

**Figures 14A-14G** show hormone levels and glucagon histology after 22 days of insulin receptor antagonist S961 (20 nMol/week) or PBS S961 treatment. Insulin **(****Figure 14A****),** Proinsulin **(****Figure 14B****),** C-peptide **(****Figure 14C****),** Proinsulin / C-peptide ratio **(****Figure 14D****),** Insulin / C-peptide ratio **(****Figure 14E****)** in fed and fasted WT and R138X mice after 22 days of treatment. **Figure 14F** shows quantification of pancreatic glucagon staining shown in **Figure 14G. Figure 14G** shows histology for glucagon in pancreas isolated from WT and R138X mice after 22 days of treatment. Values represent the means ± SEM (n=5-7 mice per genotype).

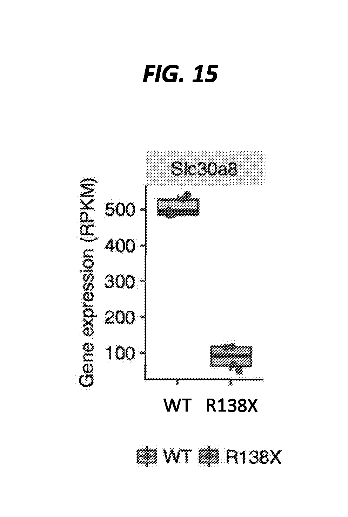

**Figure 15** shows gene expression of *Slc30a8,* expressed in RPKM (reads per kilobase million).

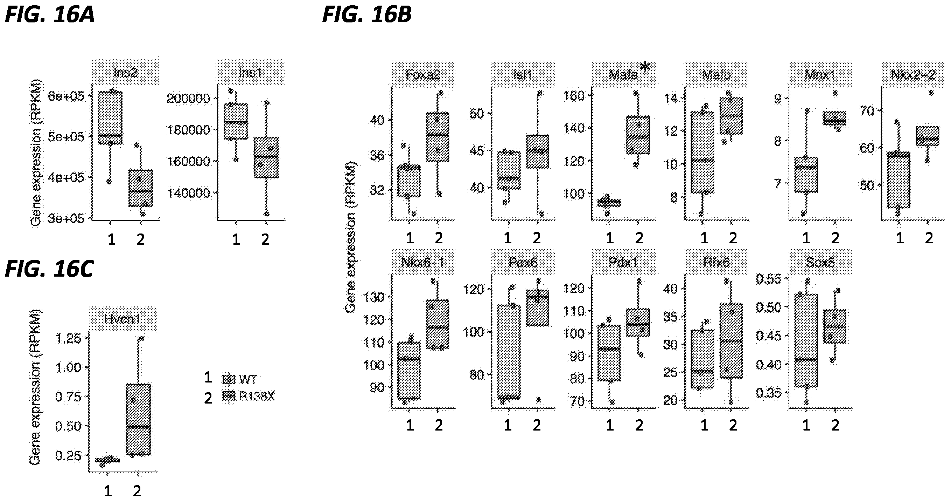

**Figures 16A-16C** show gene expression changes in islets from WT versus R138X mice. **Figure 16A** shows RPKM values for insulin 2 *(Ins2)* and insulin 1 *(Ins1).* **Figure 16B** shows RPKM values for beta-cell regulators. **Figure 16C** shows RPKM values for *Hvcn1.*

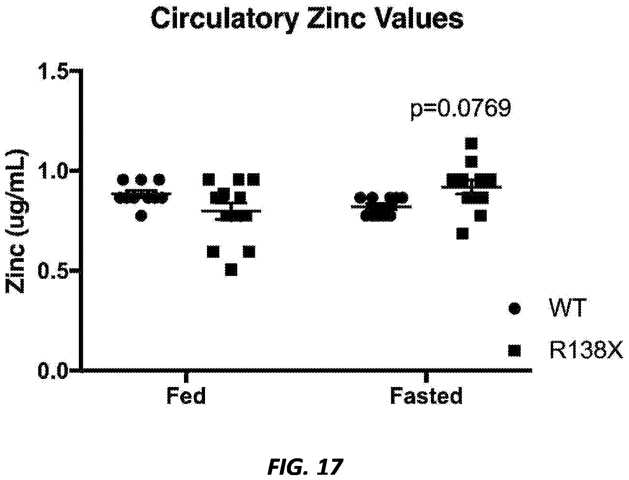

**Figure 17** shows circulatory zinc levels in fed and fasted male WT and homozygous R138X mice after 20 weeks on high-fat diet (HFD). The mice were approximately 29 weeks of age. 12 WT mice were assessed, and 13 R138X mice were assessed.

### DEFINITIONS

The terms "protein," "polypeptide," and "peptide," used interchangeably herein, include polymeric forms of amino acids of any length, including coded and non-coded amino acids and chemically or biochemically modified or derivatized amino acids. The terms also include polymers that have been modified, such as polypeptides having modified peptide backbones. The term "domain" refers to any part of a protein or polypeptide having a particular function or structure.

Proteins are said to have an "N-terminus" and a "C-terminus." The term "N-terminus" relates to the start of a protein or polypeptide, terminated by an amino acid with a free amine group (-NH2). The term "C-terminus" relates to the end of an amino acid chain (protein or polypeptide), terminated by a free carboxyl group (-COOH).

The terms "nucleic acid" and "polynucleotide," used interchangeably herein, include polymeric forms of nucleotides of any length, including ribonucleotides, deoxyribonucleotides, or analogs or modified versions thereof. They include single-, double-, and multi-stranded DNA or RNA, genomic DNA, cDNA, DNA-RNA hybrids, and polymers comprising purine bases, pyrimidine bases, or other natural, chemically modified, biochemically modified, non-natural, or derivatized nucleotide bases.

Nucleic acids are said to have "5' ends" and "3' ends" because mononucleotides are reacted to make oligonucleotides in a manner such that the 5' phosphate of one mononucleotide pentose ring is attached to the 3' oxygen of its neighbor in one direction via a phosphodiester linkage. An end of an oligonucleotide is referred to as the "5' end" if its 5' phosphate is not linked to the 3' oxygen of a mononucleotide pentose ring. An end of an oligonucleotide is referred to as the "3' end" if its 3' oxygen is not linked to a 5' phosphate of another mononucleotide pentose ring. A nucleic acid sequence, even if internal to a larger oligonucleotide, also may be said to have 5' and 3' ends. In either a linear or circular DNA molecule, discrete elements are referred to as being "upstream" or 5' of the "downstream" or 3' elements.

The term "genomically integrated" refers to a nucleic acid that has been introduced into a cell such that the nucleotide sequence integrates into the genome of the cell. Any protocol may be used for the stable incorporation of a nucleic acid into the genome of a cell.

The term "targeting vector" refers to a recombinant nucleic acid that can be introduced by homologous recombination, non-homologous-end-joining-mediated ligation, or any other means of recombination to a target position in the genome of a cell.

The term "wild type" includes entities having a structure and/or activity as found in a normal (as contrasted with mutant, diseased, altered, or so forth) state or context. Wild type genes and polypeptides often exist in multiple different forms (e.g., alleles).

The term "endogenous sequence" refers to a nucleic acid sequence that occurs naturally within a cell or non-human animal. For example, an endogenous *Slc30a8* sequence of a non-human animal refers to a native *Slc30a8* sequence that naturally occurs at the *Slc30a8* locus in the non-human animal. The term "endogenous locus" refers to a location on a chromosome at which a particular genetic element is naturally found. For example, an endogenous *Slc30a8* locus of a non-human animal refers to the naturally occurring *Slc30a8* locus in the non-human animal (i.e., the region of the non-human animal genome at which the *Slc30a8* gene is naturally found). As an example, the endogenous mouse *Slc30a8* locus maps to chromosome 15 (NCBI RefSeq GeneID 239436; Assembly GRCm38.p4; location NC_000081.6 (52295553-52335733)). The term "endogenous *Slc30a8* locus" of a non-human animal does not refer to a *Slc30a8* gene randomly inserted into the genome of the non-human animal or inserted at a region other than the region of the non-human animal genome at which the *Slc30a8* gene is naturally found. Likewise, the term "endogenous *Slc30a8* locus" of a non-human animal does not refer to a *Slc30a8* gene located, for example, in cells (e.g., human beta cells) grafted into the non-human animal.

"Exogenous" molecules or sequences include molecules or sequences that are not normally present in a cell in that form. Normal presence includes presence with respect to the particular developmental stage and environmental conditions of the cell. An exogenous molecule or sequence, for example, can include a mutated version of a corresponding endogenous sequence within the cell or can include a sequence corresponding to an endogenous sequence within the cell but in a different form (i.e., not within a chromosome). In contrast, endogenous molecules or sequences include molecules or sequences that are normally present in that form in a particular cell at a particular developmental stage under particular environmental conditions.

The term "heterologous" when used in the context of a nucleic acid or a protein indicates that the nucleic acid or protein comprises at least two segments that do not naturally occur together in the same molecule. For example, the term "heterologous," when used with reference to segments of a nucleic acid or segments of a protein, indicates that the nucleic acid or protein comprises two or more sub-sequences that are not found in the same relationship to each other (e.g., joined together) in nature. As one example, a "heterologous" region of a nucleic acid vector is a segment of nucleic acid within or attached to another nucleic acid molecule that is not found in association with the other molecule in nature. For example, a heterologous region of a nucleic acid vector could include a coding sequence flanked by sequences not found in association with the coding sequence in nature. Likewise, a "heterologous" region of a protein is a segment of amino acids within or attached to another peptide molecule that is not found in association with the other peptide molecule in nature (e.g., a fusion protein, or a protein with a tag). Similarly, a nucleic acid or protein can comprise a heterologous label or a heterologous secretion or localization sequence.

"Codon optimization" takes advantage of the degeneracy of codons, as exhibited by the multiplicity of three-base pair codon combinations that specify an amino acid, and generally includes a process of modifying a nucleic acid sequence for enhanced expression in particular host cells by replacing at least one codon of the native sequence with a codon that is more frequently or most frequently used in the genes of the host cell while maintaining the native amino acid sequence. For example, a nucleic acid encoding a Cas9 protein can be modified to substitute codons having a higher frequency of usage in a given prokaryotic or eukaryotic cell, including a bacterial cell, a yeast cell, a human cell, a non-human cell, a mammalian cell, a rodent cell, a mouse cell, a rat cell, a hamster cell, or any other host cell, as compared to the naturally occurring nucleic acid sequence. Codon usage tables are readily available, for example, at the "Codon Usage Database." These tables can be adapted in a number of ways. *See* Nakamura et al. (2000) Nucleic Acids Research 28:292. Computer algorithms for codon optimization of a particular sequence for expression in a particular host are also available (*see, e.g*., Gene Forge).

The term "locus" refers to a specific location of a gene (or significant sequence), DNA sequence, polypeptide-encoding sequence, or position on a chromosome of the genome of an organism. For example, a *"Slc30a8* locus" may refer to the specific location of a *Slc30a8* gene, *Slc30a8* DNA sequence, SLC30a8-encoding sequence, or *Slc30a8* position on a chromosome of the genome of an organism that has been identified as to where such a sequence resides. A *"Slc30a8* locus" may comprise a regulatory element of a *Slc30a8* gene, including, for example, an enhancer, a promoter, 5' and/or 3' untranslated region (UTR), or a combination thereof.

The term "gene" refers to a DNA sequence in a chromosome that codes for a product (e.g., an RNA product and/or a polypeptide product) and includes the coding region interrupted with non-coding introns and sequence located adjacent to the coding region on both the 5' and 3' ends such that the gene corresponds to the full-length mRNA (including the 5' and 3' untranslated sequences). The term "gene" also includes other non-coding sequences including regulatory sequences (e.g., promoters, enhancers, and transcription factor binding sites), polyadenylation signals, internal ribosome entry sites, silencers, insulating sequence, and matrix attachment regions. These sequences may be close to the coding region of the gene (e.g., within 10 kb) or at distant sites, and they influence the level or rate of transcription and translation of the gene.

The term "allele" refers to a variant form of a gene. Some genes have a variety of different forms, which are located at the same position, or genetic locus, on a chromosome. A diploid organism has two alleles at each genetic locus. Each pair of alleles represents the genotype of a specific genetic locus. Genotypes are described as homozygous if there are two identical alleles at a particular locus and as heterozygous if the two alleles differ.

The "coding region" or "coding sequence" of a gene consists of the portion of a gene's DNA or RNA, composed of exons, that codes for a protein. The region begins at the start codon on the 5' end and ends at the stop codon on the 3' end.

A "promoter" is a regulatory region of DNA usually comprising a TATA box capable of directing RNA polymerase II to initiate RNA synthesis at the appropriate transcription initiation site for a particular polynucleotide sequence. A promoter may additionally comprise other regions which influence the transcription initiation rate. The promoter sequences disclosed herein modulate transcription of an operably linked polynucleotide. A promoter can be active in one or more of the cell types disclosed herein (e.g., a eukaryotic cell, a non-human mammalian cell, a human cell, a rodent cell, a pluripotent cell, a one-cell stage embryo, a differentiated cell, or a combination thereof). A promoter can be, for example, a constitutively active promoter, a conditional promoter, an inducible promoter, a temporally restricted promoter (e.g., a developmentally regulated promoter), or a spatially restricted promoter (e.g., a cell-specific or tissue-specific promoter). Examples of promoters can be found, for example, in WO 2013/176772.

"Operable linkage" or being "operably linked" includes juxtaposition of two or more components (e.g., a promoter and another sequence element) such that both components function normally and allow the possibility that at least one of the components can mediate a function that is exerted upon at least one of the other components. For example, a promoter can be operably linked to a coding sequence if the promoter controls the level of transcription of the coding sequence in response to the presence or absence of one or more transcriptional regulatory factors. Operable linkage can include such sequences being contiguous with each other or acting in trans (e.g., a regulatory sequence can act at a distance to control transcription of the coding sequence).

The term "variant" refers to a nucleotide sequence differing from the sequence most prevalent in a population (e.g., by one nucleotide) or a protein sequence different from the sequence most prevalent in a population (e.g., by one amino acid).

"Sequence identity" or "identity" in the context of two polynucleotides or polypeptide sequences makes reference to the residues in the two sequences that are the same when aligned for maximum correspondence over a specified comparison window. When percentage of sequence identity is used in reference to proteins, residue positions which are not identical often differ by conservative amino acid substitutions, where amino acid residues are substituted for other amino acid residues with similar chemical properties (e.g., charge or hydrophobicity) and therefore do not change the functional properties of the molecule. When sequences differ in conservative substitutions, the percent sequence identity may be adjusted upwards to correct for the conservative nature of the substitution. Sequences that differ by such conservative substitutions are said to have "sequence similarity" or "similarity." Means for making this adjustment are well known. Typically, this involves scoring a conservative substitution as a partial rather than a full mismatch, thereby increasing the percentage sequence identity. Thus, for example, where an identical amino acid is given a score of 1 and a non-conservative substitution is given a score of zero, a conservative substitution is given a score between zero and 1. The scoring of conservative substitutions is calculated, e.g., as implemented in the program PC/GENE (Intelligenetics, Mountain View, California).

"Percentage of sequence identity" includes the value determined by comparing two optimally aligned sequences (greatest number of perfectly matched residues) over a comparison window, wherein the portion of the polynucleotide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison, and multiplying the result by 100 to yield the percentage of sequence identity. Unless otherwise specified (e.g., the shorter sequence includes a linked heterologous sequence), the comparison window is the full length of the shorter of the two sequences being compared.

Unless otherwise stated, sequence identity/similarity values include the value obtained using GAP Version 10 using the following parameters: % identity and % similarity for a nucleotide sequence using GAP Weight of 50 and Length Weight of 3, and the nwsgapdna.cmp scoring matrix; % identity and % similarity for an amino acid sequence using GAP Weight of 8 and Length Weight of 2, and the BLOSUM62 scoring matrix; or any equivalent program thereof. "Equivalent program" includes any sequence comparison program that, for any two sequences in question, generates an alignment having identical nucleotide or amino acid residue matches and an identical percent sequence identity when compared to the corresponding alignment generated by GAP Version 10.

The term "conservative amino acid substitution" refers to the substitution of an amino acid that is normally present in the sequence with a different amino acid of similar size, charge, or polarity. Examples of conservative substitutions include the substitution of a non-polar (hydrophobic) residue such as isoleucine, valine, or leucine for another non-polar residue. Likewise, examples of conservative substitutions include the substitution of one polar (hydrophilic) residue for another such as between arginine and lysine, between glutamine and asparagine, or between glycine and serine. Additionally, the substitution of a basic residue such as lysine, arginine, or histidine for another, or the substitution of one acidic residue such as aspartic acid or glutamic acid for another acidic residue are additional examples of conservative substitutions. Examples of non-conservative substitutions include the substitution of a non-polar (hydrophobic) amino acid residue such as isoleucine, valine, leucine, alanine, or methionine for a polar (hydrophilic) residue such as cysteine, glutamine, glutamic acid or lysine and/or a polar residue for a non-polar residue. Typical amino acid categorizations are summarized below.\

**Table 1. Amino Acid Categorizations.**

| | | | | | |
|---|---|---|---|---|---|
| Alanine | Ala | A | Nonpolar | Neutral | 1.8 |
| Arginine | Arg | R | Polar | Positive | -4.5 |
| Asparagine | Asn | N | Polar | Neutral | -3.5 |
| Aspartic acid | Asp | D | Polar | Negative | -3.5 |
| Cysteine | Cys | C | Nonpolar | Neutral | 2.5 |
| Glutamic acid | Glu | E | Polar | Negative | -3.5 |
| Glutamine | Gln | Q | Polar | Neutral | -3.5 |
| Glycine | Gly | G | Nonpolar | Neutral | -0.4 |
| Histidine | His | H | Polar | Positive | -3.2 |
| Isoleucine | Ile | I | Nonpolar | Neutral | 4.5 |
| Leucine | Leu | L | Nonpolar | Neutral | 3.8 |
| Lysine | Lys | K | Polar | Positive | -3.9 |
| Methionine | Met | M | Nonpolar | Neutral | 1.9 |
| Phenylalanine | Phe | F | Nonpolar | Neutral | 2.8 |
| Proline | Pro | P | Nonpolar | Neutral | -1.6 |
| Serine | Ser | S | Polar | Neutral | -0.8 |
| Threonine | Thr | T | Polar | Neutral | -0.7 |
| Tryptophan | Trp | W | Nonpolar | Neutral | -0.9 |
| Tyrosine | Tyr | Y | Polar | Neutral | -1.3 |
| Valine | Val | V | Nonpolar | Neutral | 4.2 |

A "homologous" sequence (e.g., nucleic acid sequence) includes a sequence that is either identical or substantially similar to a known reference sequence, such that it is, for example, at least 50%, at least 55%, at least 60%, at least 65%, at least 70%, at least 75%, at least 80%, at least 85%, at least 90%, at least 95%, at least 96%, at least 97%, at least 98%, at least 99%, or 100% identical to the known reference sequence. Homologous sequences can include, for example, orthologous sequence and paralogous sequences. Homologous genes, for example, typically descend from a common ancestral DNA sequence, either through a speciation event (orthologous genes) or a genetic duplication event (paralogous genes). "Orthologous" genes include genes in different species that evolved from a common ancestral gene by speciation. Orthologs typically retain the same function in the course of evolution. "Paralogous" genes include genes related by duplication within a genome. Paralogs can evolve new functions in the course of evolution.

The term *"in vitro"* includes artificial environments and to processes or reactions that occur within an artificial environment (e.g., a test tube). The term *"in vivo"* includes natural environments (e.g., a cell or organism or body) and to processes or reactions that occur within a natural environment. The term *"ex vivo"* includes cells that have been removed from the body of an individual and to processes or reactions that occur within such cells.

The term "reporter gene" refers to a nucleic acid having a sequence encoding a gene product (typically an enzyme) that is easily and quantifiably assayed when a construct comprising the reporter gene sequence operably linked to an endogenous or heterologous promoter and/or enhancer element is introduced into cells containing (or which can be made to contain) the factors necessary for the activation of the promoter and/or enhancer elements. Examples of reporter genes include, but are not limited, to genes encoding beta-galactosidase (lacZ), the bacterial chloramphenicol acetyltransferase (cat) genes, firefly luciferase genes, genes encoding beta-glucuronidase (GUS), and genes encoding fluorescent proteins. A "reporter protein" refers to a protein encoded by a reporter gene.

The term "fluorescent reporter protein" as used herein means a reporter protein that is detectable based on fluorescence wherein the fluorescence may be either from the reporter protein directly, activity of the reporter protein on a fluorogenic substrate, or a protein with affinity for binding to a fluorescent tagged compound. Examples of fluorescent proteins include green fluorescent proteins (e.g., GFP, GFP-2, tagGFP, turboGFP, eGFP, Emerald, Azami Green, Monomeric Azami Green, CopGFP, AceGFP, and ZsGreenl), yellow fluorescent proteins (e.g., YFP, eYFP, Citrine, Venus, YPet, PhiYFP, and ZsYellowl), blue fluorescent proteins (e.g., BFP, eBFP, eBFP2, Azurite, mKalamal, GFPuv, Sapphire, and T-sapphire), cyan fluorescent proteins (e.g., CFP, eCFP, Cerulean, CyPet, AmCyanl, and Midoriishi-Cyan), red fluorescent proteins (e.g., RFP, mKate, mKate2, mPlum, DsRed monomer, mCherry, mRFP1, DsRed-Express, DsRed2, DsRed-Monomer, HcRed-Tandem, HcRedl, AsRed2, eqFP611, mRaspberry, mStrawberry, and Jred), orange fluorescent proteins (e.g., mOrange, mKO, Kusabira-Orange, Monomeric Kusabira-Orange, mTangerine, and tdTomato), and any other suitable fluorescent protein whose presence in cells can be detected by flow cytometry methods.

Repair in response to double-strand breaks (DSBs) occurs principally through two conserved DNA repair pathways: homologous recombination (HR) and non-homologous end joining (NHEJ). *See* Kasparek & Humphrey (2011) Seminars in Cell & Dev. Biol. 22:886-897. Likewise, repair of a target nucleic acid mediated by an exogenous donor nucleic acid can include any process of exchange of genetic information between the two polynucleotides.

The term "recombination" includes any process of exchange of genetic information between two polynucleotides and can occur by any mechanism. Recombination can occur via homology directed repair (HDR) or homologous recombination (HR). HDR or HR includes a form of nucleic acid repair that can require nucleotide sequence homology, uses a "donor" molecule as a template for repair of a "target" molecule (i.e., the one that experienced the double-strand break), and leads to transfer of genetic information from the donor to target. Without wishing to be bound by any particular theory, such transfer can involve mismatch correction of heteroduplex DNA that forms between the broken target and the donor, and/or synthesis-dependent strand annealing, in which the donor is used to resynthesize genetic information that will become part of the target, and/or related processes. In some cases, the donor polynucleotide, a portion of the donor polynucleotide, a copy of the donor polynucleotide, or a portion of a copy of the donor polynucleotide integrates into the target DNA. *See* Wang et al. (2013) Cell 153:910-918; Mandalos et al. (2012) PLOS ONE 7:e45768:1-9; and Wang et al. (2013) Nat Biotechnol. 31:530-532.

NHEJ includes the repair of double-strand breaks in a nucleic acid by direct ligation of the break ends to one another or to an exogenous sequence without the need for a homologous template. Ligation of non-contiguous sequences by NHEJ can often result in deletions, insertions, or translocations near the site of the double-strand break. For example, NHEJ can also result in the targeted integration of an exogenous donor nucleic acid through direct ligation of the break ends with the ends of the exogenous donor nucleic acid (i.e., NHEJ-based capture). Such NHEJ-mediated targeted integration can be preferred for insertion of an exogenous donor nucleic acid when homology directed repair (HDR) pathways are not readily usable (e.g., in non-dividing cells, primary cells, and cells which perform homology-based DNA repair poorly). In addition, in contrast to homology-directed repair, knowledge concerning large regions of sequence identity flanking the cleavage site is not needed, which can be beneficial when attempting targeted insertion into organisms that have genomes for which there is limited knowledge of the genomic sequence. The integration can proceed via ligation of blunt ends between the exogenous donor nucleic acid and the cleaved genomic sequence, or via ligation of sticky ends (i.e., having 5' or 3' overhangs) using an exogenous donor nucleic acid that is flanked by overhangs that are compatible with those generated by a nuclease agent in the cleaved genomic sequence. *See, e.g.,* US 2011/020722, WO 2014/033644, WO 2014/089290, and Maresca et al. (2013) Genome Res. 23(3):539-546. If blunt ends are ligated, target and/or donor resection may be needed to generation regions of microhomology needed for fragment joining, which may create unwanted alterations in the target sequence.

The term "R138X mice" refers to mice carrying a mutation in the mouse *Slc30a8* locus corresponding to the human *SLC30A8* putative LOF mutation, R138X. The mutation in the mouse *Slc30a8* locus is c.409C>T (transcript accession number NM_172816.3), p.Arg137X, meaning a C to T substitution at nucleotide 409 of the coding sequence resulting in the codon encoding amino acid residue 137 being mutated from a codon encoding an arginine residue to a stop codon (R137^{∗stop}). The corresponding human *SLC30A8* mutation is c.412 C>T (transcript accession number NM_173851), p.Arg138X, meaning a C to T substitution at nucleotide 412 of the coding sequence resulting in the codon encoding amino acid residue 138 being mutated from a codon encoding an arginine residue to a stop codon (R138^{∗stop}). Amino acid residue 137 of the mouse SLC30A8 protein corresponds with amino acid residue 138 of the human SLC30A8 protein when the two are optimally aligned. *See* **Figure 11****.** Although the mutation in the mouse *Slc30a8* gene is in the codon encoding amino acid residue 137 of the mouse SLC30A8 protein, these mice are referred to herein as "R138X mice" in reference to the corresponding human *SLC30A8* mutation.

Compositions or methods "comprising" or "including" one or more recited elements may include other elements not specifically recited. For example, a composition that "comprises" or "includes" a protein may contain the protein alone or in combination with other ingredients. The transitional phrase "consisting essentially of' means that the scope of a claim is to be interpreted to encompass the specified elements recited in the claim and those that do not materially affect the basic and novel characteristic(s) of the claimed invention. Thus, the term "consisting essentially of' when used in a claim of this invention is not intended to be interpreted to be equivalent to "comprising."

"Optional" or "optionally" means that the subsequently described event or circumstance may or may not occur and that the description includes instances in which the event or circumstance occurs and instances in which it does not.

Designation of a range of values includes all integers within or defining the range, and all subranges defined by integers within the range.

Unless otherwise apparent from the context, the term "about" encompasses values within a standard margin of error of measurement (e.g., SEM) of a stated value.

The term "and/or" refers to and encompasses any and all possible combinations of one or more of the associated listed items, as well as the lack of combinations when interpreted in the alternative ("or").

The term "or" refers to any one member of a particular list and also includes any combination of members of that list.

The singular forms of the articles "a," "an," and "the" include plural references unless the context clearly dictates otherwise. For example, the term "a protein" or "at least one protein" can include a plurality of proteins, including mixtures thereof.

Statistically significant means p ≤0.05.

### DETAILED DESCRIPTION

### I.Overview

Disclosed herein are non-human animal genomes, non-human animal cells, and non-human animals comprising in their genome a mutated *Slc30a8* locus and methods of using such non-human animal cells and non-human animals. The present invention relates to the embodiments as characterized in the appended claims, in particular to a non-human animal which in accordance with the invention is a rodent whose genome comprises an endogenous *Slc30a8* locus comprising a mutated *Slc30a8* gene, wherein the mutated *Slc30a8* gene comprises a nonsense mutation at the 3' end of the third exon of the *Slc30a8* gene and results in the rodent having an enhanced capacity for insulin secretion relative to a rodent without the mutations. The zinc transporter SLC30A8 is primarily expressed in the islets of the endocrine pancreas. *SLC30A8* is only one of three genes to date for which putative loss-of-function mutations have been shown to protect from development of type 2 diabetes. This is an interesting but also puzzling observation since mouse models deficient in *Slc30a8* have mildly impaired glucose homeostasis. We have generated a mouse model comprising a mutated *Slc30a8* locus mimicking a protective human *SLC30A8* allele. This mouse model can for the first time explain why humans are protected from type 2 diabetes. The mice have increased insulin secretory capacity. In particular, the mice have higher glucose-induced insulin secretion, which may be mediated in part by an increased number of pancreatic beta cells. These *SLC30A8* mutation data support the notion that beta-cell expansion is a valid therapeutic strategy to manage type 2 diabetes.

The cells and non-human animal models disclosed herein can be used for drug screening as well as to provide insights into the mechanism of T2D (e.g., the mechanism by which SLC30A8 contributes to insulin processing and diabetes) and potentially new therapeutic and diagnostic targets.

### II. Non-Human Animals Comprising a Mutated Slc30a8 Locus

The rodent cells and non-human animals, *i.e.* rodents disclosed herein comprise (e.g., in their genome) a mutation (e.g., a mutation that does not naturally occur in non-human animals) in the *Slc30a8* locus. The mutated *Slc30a8* locus can have a premature termination codon and/or can encode a truncated SLC30A8 protein. The non-human animals comprising a mutated *Slc30a8* locus have an enhanced capacity for insulin secretion when fed a high-fat diet relative to a non-human animal without the mutation when fed the same diet.

### A. Slc30a8

The rodent cells and non-human animals, *i.e.* rodents described herein comprise a mutated *Slc30a8* (solute carrier family 30 member 8) locus. Other names for SLC30A8 include zinc transporter 8, ZnT-8, and Znt8. The protein encoded by *Slc30a8* is a zinc transporter involved in the accumulation of zinc in intracellular vesicles. *Slc30a8* is expressed at a high level in the pancreas, particularly in islets of Langerhans. The encoded protein co-localizes with insulin in the secretory pathway granules of the insulin-secreting INS-1 cells.

Human *SLC30A8* maps to human 8q24.11 on chromosome 8 (NCBI RefSeq GeneID 169026; Assembly GRCh38.p7; location 116950273-117176714). The gene has been reported to have eight exons and seven introns. However, isoforms with more than eight exons (including non-coding exons) are also possible. The wild type human SLC30A8 protein has been assigned UniProt accession number Q8IWU4. Two isoforms are known, Q8IWU4 isoform 1 (SEQ ID NO: 14) and Q8IWU4 isoform 2. The mRNA encoding isoform 1 is assigned NCBI RefSeq NM_173851.2 (SEQ ID NO: 17). The full-length human protein has 369 amino acids including six transmembrane regions, four intracellular topological domains, and three extracellular topological domains. Delineations between these domains are as designated in UniProt. Reference to human SCL30A8 includes the canonical (wild type) forms as well as all allelic forms and isoforms. Any other forms of human SLC30A8 have amino acids numbered for maximal alignment with the wild type form, aligned amino acids being designated the same number.

Mouse *Slc30a8* maps to chromosome 15 (NCBI RefSeq GeneID 239436; Assembly GRCm38.p4; location NC_000081.6 (52295553-52335733)). The gene has been reported to have eight exons and seven introns. The wild type mouse SLC30A8 protein has been assigned UniProt accession number Q8BGG0 (SEQ ID NO: 12). The mRNA encoding mouse SLC30A8 is assigned NCBI RefSeq NM_172816.3 (SEQ ID NO: 16). The full-length mouse protein has 367 amino acids including six transmembrane regions, four intracellular topological domains, and three extracellular topological domains. Delineations between these domains are as designated in UniProt. Reference to mouse SCL30A8 includes the canonical (wild type) forms, as well as all allelic forms and isoforms. Any other forms of mouse SLC30A8 have amino acids numbered for maximal alignment with the wild type form, aligned amino acids being designated the same number.

An exemplary rat SLC30A8 protein is designated by UniProt Accession Number P0CE46.

Polymorphisms in the human *SLC30A8* gene are associated with altered risk of type 2 diabetes (T2D). *See, e.g.,* Sladek et al. (2007) Nature 445(7130):881-885 and Davidson et al. (2014) Trends Endocrinol. Metab. 25(8):415-424. Some such polymorphisms or mutations in humans can reduce risk of T2D or are protective against T2D in humans. A polymorphism or mutation in the *SLC30A8* gene that reduces risk of T2D includes a polymorphism or mutation that confers protection against T2D, that confers resistance to T2D, or that is associated with protection against or resistance to T2D. For example, various stop codon mutations, frameshift mutations, splice site mutations, or initiator codon variations that cause protein truncation in human SLC30A8 are protective against T2D when heterozygous. One of these is designated c.412 C>T (transcript accession number NM_173851), p.Arg138X meaning a C to T substitution at nucleotide 412 of the coding sequence resulting in the codon encoding amino acid residue 138 being mutated from a codon encoding an arginine residue to a stop codon (R138^{∗stop}). *See, e.g*., Flannick et al. (2014) Nat. Genet. 46(4):357-363. These phenotypes have not been replicated in previous animal models but are replicated in the non-human animals disclosed herein.

### B. Mutated Slc30a8 Loci

The mutated *Slc30a8* loci disclosed herein result in non-human animals having an enhanced capacity for insulin secretion when fed a high-fat diet relative to a non-human animal without the mutation when fed the same diet. The mutated *Slc30a8* loci may reduce T2D risk. Non-human animals comprising a mutated *Slc30a8* locus can have one or more or all of the following characteristics relative to the non-human animal without the mutation (e.g., a wild type non-human animal): (1) increased glucose-induced insulin secretion when fed a high-fat diet; (2) increased pancreatic beta-cell proliferation when fed a high-fat diet; (3) increased number of pancreatic beta cells when fed a high-fat diet; and (4) increased fed plasma insulin levels after blockade of the insulin receptor (e.g., with S961). Additionally or alternatively, non-human animals comprising a mutated *Slc30a8* locus can have one or more or all of the following characteristics relative to the non-human animal without the mutation (e.g., a wild type non-human animal): (1) increased circulating insulin levels after 20, 25, 30, 35, 40, 45, or 50 weeks (e.g., after 20 or 30 weeks) being fed on a high-fat diet; (2) increased number of pancreatic beta cells after 20, 25, 30, 35, 40, 45, or 50 weeks (e.g., after 20 or 30 weeks) being fed on a high-fat diet; (3) decrease in the proinsulin-to-insulin ratio when fed a high-fat diet; (4) increased fed plasma insulin levels after blockade of the insulin receptor (e.g., after 1, 2, 3, 4, 5, 6, 7, 8, 9, 10, 11, 12, 13, 14, 15, 16, 17, 18, 19, 20, 21, 22, 23, 24, or 25 days); and (5) pancreatic beta cells with improved insulin processing and better health (e.g., as evidenced by decrease in ratio of proinsulin-to-insulin when fed a high-fat diet). Additionally or alternatively, non-human animals comprising a mutated *Slc30a8* locus can have significant *Slc30a8* mRNA expression in islets (e.g., at least 5%, 10%, 15%, 20%, 25%, 30%, 35%, 40%, 45%, 50%, 55%, 60%, 65%, 70%, 75%, 80%, 85%, 90%, 95%, or 100% of wild type levels, such as at least 25% or 30% of wild type levels).

Compared to non-human animals without the mutation (e.g., wild type non-human animals), non-human animals with a mutated *Slc30a8* locus can have a normal metabolic phenotype when fed a control chow diet. Compared to non-human animals without the mutation (e.g., wild type non-human animals), non-human animals with a mutated *Slc30a8* locus can have normal glucose homeostasis and/or normal glucose-induced insulin secretion when fed a control chow diet. Compared to non-human animals without the mutation (e.g., wild type non-human animals), non-human animals with a mutated *Slc30a8* locus can also have one or more or all of the following characteristics when fed a control chow diet: do not differ significantly in body weight, do not differ significantly in circulating blood glucose levels, do not differ significantly in circulating insulin levels, do not differ significantly in circulating proinsulin levels, do not differ significantly in circulating C-peptide levels, do not differ significantly in the ratio of proinsulin/C-peptide, do not differ significantly in glucose tolerance, do not differ significantly in insulin sensitivity, do not differ significantly in glucose-induced insulin secretion, do not differ significantly in beta-cell mass, and do not differ significantly in alpha-cell mass. Compared to non-human animals without the mutation (e.g., wild type non-human animals), non-human animals with a mutated *Slc30a8* locus can have a decrease in the ratio of insulin/C-peptide when fed a control chow diet. This may indicate higher insulin clearance.

Compared to non-human animals without the mutation (e.g., wild type non-human animals), non-human animals with a mutated *Slc30a8* locus have an increased capacity to secrete insulin. As one example, the non-human animals can have an enhanced capacity for insulin secretion when fed a high-fat diet. Such non-human animals can have increased insulin secretion relative to the non-human animal without the mutation when fed a high-fat diet, wherein the increased insulin secretion is associated with increased beta-cell proliferation or beta-cell mass relative to the non-human animal without the mutation. The increased beta-cell proliferation can be insulin-receptor-dependent (e.g., dependent on an insulin receptor in the beta cells). As another example, the non-human animals can have an enhanced capacity for insulin secretion in response to hyperglycemia, such as severe hyperglycemia. Such non-human animals can have increased insulin secretion in response to hyperglycemia induced by insulin receptor inhibition relative to the non-human animal without the mutation. For example, such non-human animals can have increased insulin secretion relative to the non-human animal without the mutation in response to hyperglycemia induced by insulin receptor inhibition, wherein the increased insulin secretion is not associated with increased beta-cell proliferation or beta-cell mass relative to the non-human animal without the mutation. The increased insulin secretion compared to non-human animals without the mutation can be at least about 5%, 10%, 15%, 20%, 25%, 30%, 35%, 40%, 45%, or 50%. For example, the non-human animals can have increased insulin secretion in response to hyperglycemia induced by insulin receptor inhibition, such as hyperglycemia caused by the insulin receptor antagonist S961. In some non-human animals, this effect is not associated with enhanced beta-cell proliferation or increased beta-cell mass. In some non-human animals, the increase in plasma insulin is not a result of one or more or all of the following: improved proinsulin processing, decreased insulin clearance, increased beta-cell proliferation, increased islet mass, increased beta-cell mass, increased alpha-cell mass, or insulin depletion in beta cells.

Compared to non-human animals without the mutation (e.g., wild type non-human animals), non-human animals with a mutated *Slc30a8* locus can have decreased mitochondrial gene expression (e.g., decrease in expression of genes in the mitochondrial oxidative phosphorylation (OXPHOS) system). Examples of such genes are described in more detail in Example 2 and in Tables 7-9. Compared to non-human animals without the mutation (e.g., wild type non-human animals), non-human animals with a mutated *Slc30a8* locus can have increased *Hvcn1* (hydrogen voltage-gated channel 1, or voltage-gated hydrogen channel 1) expression.

Compared to non-human animals without the mutation (e.g., wild type non-human animals), non-human animals with a mutated *Slc30a8* locus can have loss of islet zinc accumulation.

Compared to non-human animals without the mutation (e.g., wild type non-human animals), non-human animals with a mutated *Slc30a8* locus can also have one or more or all of the following characteristics when fed a control chow diet: do not differ in body weight, blood glucose, insulin, or C-peptide levels (fed or fasted); have no change in fed proinsulin levels; might have decreased fasted proinsulin levels; have no change in the ratio of proinsulin to insulin (fed or fasted); have an increased fed ratio of C-peptide to insulin; have no change in the fasted ratio of C-peptide to insulin; and have no change in glucose tolerance or glucose-induced insulin secretion.

Compared to non-human animals without the mutation (e.g., wild type non-human animals), non-human animals with a mutated *Slc30a8* locus can also have one or more or all of the following characteristics when fed a high-fat diet: have no change in fed or fasted blood glucose; have increased fed circulating insulin levels; have no change in fasted circulating insulin levels; have no change in fed proinsulin levels; have decreased fasted proinsulin levels; have increased fed circulating C-peptide levels; have no change in fasted circulating C-peptide levels; have a decreased ratio of proinsulin to insulin (fed and fasted); have no change in the ratio of C-peptide to insulin; have an increased insulin-positive area in the pancreas; have an increased islet number per pancreas area; have increased beta-cell proliferation; and have an increased number of insulin-producing beta cells. In addition, compared to non-human animals without the mutation, non-human animals with a mutated *Slc30a8* locus can have increased fed plasma insulin levels after blockade of the insulin receptor (e.g., using S961).

A mutated *Slc30a8* locus disclosed herein can be a *Slc30a8* locus encoding a truncated SLC30A8 protein (i.e., protein-truncating variants). The mutation causing the truncation can be, for example, a frameshift mutation, a nonsense mutation, a splice site mutation, or an initiator codon SNV. *See, e.g.,* Flannick et al. (2014) Nat. Genet. 46(4):357-363. A nonsense mutation is a mutation in which a sense codon that corresponds to one of the twenty amino acids specified by the genetic code is changed to a chain-terminating codon (i.e., termination codon or stop codon). A frameshift mutation arises when the normal sequence of codons is disrupted by the insertion or deletion of one or more nucleotides, provided that the number of nucleotides added or removed is not a multiple of three. A frameshift mutation is a sequence change between the translation initiation codon (start codon) and termination codon (stop codon) in which, compared to a reference sequence, translation shifts to another frame. For example, the reading frame can be shifted one nucleotide in the 5' direction (-1 frameshift) or one nucleotide in the 3' direction (+1 frameshift). A protein encoded by a gene with a frameshift mutation will be identical to the protein encoded by the wild type gene from the N-terminus to the frameshift mutation, but different beyond that point. Such frameshifts can result in a premature termination codon. A splice site mutation can result, for example, in skipping of an exon and potentially a subsequent frameshift that results in a premature termination codon. In one example, a mutated *Slc30a8* locus disclosed herein can be a *Slc30a8* locus in which the *Slc30a8* gene has a premature termination codon (i.e., stop codon). In a specific example, the mutation comprises, consists essentially of, or consists of a nonsense mutation.

The mutation can be anywhere within the *Slc30a8* locus. For example, the mutation can be in the first exon, the second exon, the third exon, the fourth exon, the fifth exon, the sixth exon, the seventh exon, or the eighth exon of the *Slc30a8* gene. Similarly, the mutation can be in a region of the *Slc30a8* gene corresponding to the first, second, third, fourth, fifth, sixth, seventh, or eighth exon of the human *SLC30A8* gene when optimally aligned with the human *SLC30A8* gene. In a specific example, the mutation is in the third exon of the *Slc30a8* gene or in a region of the *Slc30a8* gene corresponding to the third exon of the human *SLC30A8* gene when optimally aligned with the human *SLC30A8* gene. In accordance with the present invention, the mutation is at the 3' end of the third exon of the *Slc30a8* gene or in a region of the *Slc30a8* gene corresponding to the 3' end of third exon of the human *SLC30A8* gene when optimally aligned with the human *SLC30A8* gene.

In one example, the mutation is at a residue corresponding to residue 412 of the human *SLC30A8* coding sequence (CDS) set forth in SEQ ID NO: 21 (i.e., residue c.412, transcript accession number NM_173851) when the coding sequence of the mutated *Slc30a8* gene is optimally aligned with SEQ ID NO: 21. Alternatively, the mutation is at a residue within 1, 2, 3, 4, 5, 10, 15, 20, 25, 30, 40, 50, 100, 200, 300, 400, or 500 nucleotides of residue 412 of the human *SLC30A8* coding sequence set forth in SEQ ID NO: 21 (i.e., residue c.412, transcript accession number NM_173851) when the coding sequence of the mutated *Slc30a8* gene is optimally aligned with SEQ ID NO: 21.

In another example, the mutation is at a residue corresponding to residue 409 of the mouse *Slc30a8* coding sequence (CDS) set forth in SEQ ID NO: 20 (i.e., residue c.409, transcript accession number NM_173851.2) when the coding sequence of the mutated *Slc30a8* gene is optimally aligned with SEQ ID NO: 20. Alternatively, the mutation is at a residue within 1, 2, 3, 4, 5, 10, 15, 20, 25, 30, 40, 50, 100, 200, 300, 400, or 500 nucleotides of residue 409 of the mouse *Slc30a8* coding sequence set forth in SEQ ID NO: 20 (i.e., residue c.409, transcript accession number NM_173851.2) when the coding sequence of the mutated *Slc30a8* gene is optimally aligned with SEQ ID NO: 20.

In another example, the mutation results in a stop codon in a codon corresponding to residue 138 in the human SLC30A8 protein set forth in SEQ ID NO: 14 (i.e., amino acid 138 in UniProt accession number Q8IWU4.2) when optimally aligned with SEQ ID NO: 14. Alternatively, the stop codon corresponds to a codon within 1, 2, 3, 4, 5, 10, 15, 20, 25, 30, 40, 50, 60, 70, 80, 90, 100, 110, 120, 130, 140, 150, 160, 170, 180, 190, 200, 210, 220, 230, 240, or 250 amino acids of residue 138 in the human SLC30A8 protein set forth in SEQ ID NO: 14 (i.e., amino acid 138 in UniProt accession number Q8IWU4.2) when optimally aligned with SEQ ID NO: 14.

In another example, the mutation results in a stop codon in a codon corresponding to residue 137 in the mouse SLC30A8 protein set forth in SEQ ID NO: 12 (i.e., amino acid 137 in UniProt accession number Q8BGG0.1) when optimally aligned with SEQ ID NO: 12. Alternatively, the stop codon corresponds to a codon within 1, 2, 3, 4, 5, 10, 15, 20, 25, 30, 40, 50, 60, 70, 80, 90, 100, 110, 120, 130, 140, 150, 160, 170, 180, 190, 200, 210, 220, 230, 240, or 250 amino acids of residue 137 in the mouse SLC30A8 protein set forth in SEQ ID NO: 12 (i.e., amino acid 137 in UniProt accession number Q8BGG0.1) when optimally aligned with SEQ ID NO: 12.

In accordance with the present inventon the mutated *Slc30a8* locus is endogenous to the non-human animal. In a specific example, the mutated *Slc30a8* locus encodes an mRNA (cDNA) that comprises, consists essentially of, or consists of a sequence that is at least 85%, 90%, 95%, 96%, 97%, 98%, 99%, or 100% identical to SEQ ID NO: 18. The coding sequence of the mutated *SLC30A8* locus can comprise, consist essentially of, or consist of a sequence that is at least 85%, 90%, 95%, 96%, 97%, 98%, 99%, or 100% identical to SEQ ID NO: 22 or degenerates thereof encoding the same amino acid sequence. The resulting SLC30A8 protein encoded by the mutated *SLC30A8* locus can comprise, consist essentially of, or consist of a sequence that is at least 85%, 90%, 95%, 96%, 97%, 98%, 99%, or 100% identical to SEQ ID NO: 13.

Optionally, a mutated *SLC30A8* locus can comprise other exogenous or heterologous elements. Examples of such elements can include selection cassettes, reporter genes, recombinase recognition sites, or other elements. As one example, a mutated *SLC30A8* locus can comprise a removable selection cassette (e.g., a self-deleting selection cassette) flanked by recombinase recognition sequences (e.g., loxP sites). Alternatively, the mutated *SLC30A8* locus can lack other elements (e.g., can lack a selection cassette and/or can lack a reporter gene). Examples of suitable reporter genes and reporter proteins are disclosed elsewhere herein. Examples of suitable selection markers include neomycin phosphotransferase (neoᵣ), hygromycin B phosphotransferase (hygᵣ), puromycin-N-acetyltransferase (puroᵣ), blasticidin S deaminase (bsrᵣ), xanthine/guanine phosphoribosyl transferase (gpt), and herpes simplex virus thymidine kinase (HSV-k). Examples of recombinases include Cre, Flp, and Dre recombinases. One example of a Cre recombinase gene is Crei, in which two exons encoding the Cre recombinase are separated by an intron to prevent its expression in a prokaryotic cell. Such recombinases can further comprise a nuclear localization signal to facilitate localization to the nucleus (e.g., NLS-Crei). Recombinase recognition sites include nucleotide sequences that are recognized by a site-specific recombinase and can serve as a substrate for a recombination event. Examples of recombinase recognition sites include FRT, FRT11, FRT71, attp, att, rox, and lox sites such as loxP, lox511, lox2272, lox66, lox71, loxM2, and lox5171.

Other elements such as reporter genes or selection cassettes can be self-deleting cassettes flanked by recombinase recognition sites. *See, e.g.,* US 8,697,851 and US 2013/0312129. As an example, the self-deleting cassette can comprise a Crei gene (comprises two exons encoding a Cre recombinase, which are separated by an intron) operably linked to a mouse *Prml* promoter and a neomycin resistance gene operably linked to a human ubiquitin promoter. By employing the *Prml* promoter, the self-deleting cassette can be deleted specifically in male germ cells of F0 animals. The polynucleotide encoding the selection marker can be operably linked to a promoter active in a cell being targeted. Examples of promoters are described elsewhere herein. As another specific example, a self-deleting selection cassette can comprise a hygromycin resistance gene coding sequence operably linked to one or more promoters (e.g., both human ubiquitin and EM7 promoters) followed by a polyadenylation signal, followed by a Crei coding sequence operably linked to one or more promoters (e.g., an mPrm1 promoter), followed by another polyadenylation signal, wherein the entire cassette is flanked by loxP sites.

The mutated *SLC30A8* locus can also be a conditional allele. For example, the conditional allele can be a multifunctional allele, as described in US 2011/0104799. For example, the conditional allele can comprise: (a) an actuating sequence in sense orientation with respect to transcription of a target gene; (b) a drug selection cassette (DSC) in sense or antisense orientation; (c) a nucleotide sequence of interest (NSI) in antisense orientation; and (d) a conditional by inversion module (COIN, which utilizes an exon-splitting intron and an invertible gene-trap-like module) in reverse orientation. *See, e.g.,* US 2011/0104799. The conditional allele can further comprise recombinable units that recombine upon exposure to a first recombinase to form a conditional allele that (i) lacks the actuating sequence and the DSC; and (ii) contains the NSI in sense orientation and the COIN in antisense orientation. *See, e.g.,* US 2011/0104799.

### C. Non-Human Animal Genomes, Non-Human Animal Cells, and Non-Human Animals Comprising a Mutated Slc30a8 Locus

Non-human animal genomes, non-human animal cells, and non-human animals comprising a mutated *Slc30a8* locus as described elsewhere herein are provided. The genomes, cells, or non-human animals can be male or female. The genomes, cells, or non-human animals can be heterozygous or homozygous for the mutated *Slc30a8* locus. A diploid organism has two alleles at each genetic locus. Each pair of alleles represents the genotype of a specific genetic locus. Genotypes are described as homozygous if there are two identical alleles at a particular locus and as heterozygous if the two alleles differ.

The non-human animal genomes or cells provided herein can be any non-human animal, *i.e.* rodent genome or cell comprising a *Slc30a8* locus or a genomic locus homologous or orthologous to the human *SLC30A8* locus. The genomes can be from or the cells can be a rodent cell, a rat cell, a mouse cell, or a hamster cell.. The term "non-human" excludes humans.

The cells can also be any type of undifferentiated or differentiated state. For example, a cell can be a totipotent cell, a pluripotent cell (e.g., a human pluripotent cell or a non-human pluripotent cell such as a mouse embryonic stem (ES) cell or a rat ES cell), or a non-pluripotent cell. Totipotent cells include undifferentiated cells that can give rise to any cell type, and pluripotent cells include undifferentiated cells that possess the ability to develop into more than one differentiated cell types. Such pluripotent and/or totipotent cells can be, for example, ES cells or ES-like cells, such as an induced pluripotent stem (iPS) cells. ES cells include embryo-derived totipotent or pluripotent cells that are capable of contributing to any tissue of the developing embryo upon introduction into an embryo. ES cells can be derived from the inner cell mass of a blastocyst and are capable of differentiating into cells of any of the three vertebrate germ layers (endoderm, ectoderm, and mesoderm).

The cells provided herein can also be germ cells (e.g., sperm or oocytes). The cells can be mitotically competent cells or mitotically-inactive cells, meiotically competent cells or meiotically-inactive cells. Similarly, the cells can also be primary somatic cells or cells that are not a primary somatic cell. Somatic cells include any cell that is not a gamete, germ cell, gametocyte, or undifferentiated stem cell. For example, the cells can be beta cells, pancreatic islet cells, or pancreatic cells.

Suitable cells provided herein also include primary cells. Primary cells include cells or cultures of cells that have been isolated directly from an organism, organ, or tissue. Primary cells include cells that are neither transformed nor immortal. They include any cell obtained from an organism, organ, or tissue which was not previously passed in tissue culture or has been previously passed in tissue culture but is incapable of being indefinitely passed in tissue culture. Such cells can be isolated by conventional techniques and include, for example, beta cells.

Other suitable cells provided herein include immortalized cells. Immortalized cells include cells from a multicellular organism that would normally not proliferate indefinitely but, due to mutation or alteration, have evaded normal cellular senescence and instead can keep undergoing division. Such mutations or alterations can occur naturally or be intentionally induced. Specific examples of immortalized cell lines are the EndoC-βH2, 1.1B4, 1.4E7, 1.1E7, RIN, HIT, MIN, INS-1, and βTC cell lines. *See, e.g.,* Scharfmann et al. (2014) J. Clin. Invest. 124(5):2087-2098 and McCluskey et al. (2011) J. Biol. Chem. 286(25):21982-21992. Numerous types of immortalized cells are well known. Immortalized or primary cells include cells that are typically used for culturing or for expressing recombinant genes or proteins.

The cells provided herein also include one-cell stage embryos (i.e., fertilized oocytes or zygotes). Such one-cell stage embryos can be from any genetic background (e.g., BALB/c, C57BL/6, 129, or a combination thereof for mice), can be fresh or frozen, and can be derived from natural breeding or *in vitro* fertilization.

The cells provided herein can be normal, healthy cells, or can be diseased or mutant-bearing cells.

Non-human animals comprising a mutated *Slc30a8* locus as described herein can be made by the methods described elsewhere herein. The term "animal" includes any member of the animal kingdom, including, for example, mammals, fishes, reptiles, amphibians, birds, and worms. In a specific example, the non-human animal is a non-human mammal. Non-human mammals include, for example, non-human primates, monkeys, apes, orangutans, cats, dogs, horses, rabbits, rodents (e.g., mice, rats, hamsters, and guinea pigs), and livestock (e.g., bovine species such as cows and steer; ovine species such as sheep and goats; and porcine species such as pigs and boars). Domesticated animals and agricultural animals are also included. The term "non-human animal" excludes humans. In accordance with the present invention the non-human animals are rodents, such as mice and rats.

The non-human animals can be from any genetic background. For example, suitable mice can be from a 129 strain, a C57BL/6 strain, a mix of 129 and C57BL/6, a BALB/c strain, or a Swiss Webster strain. Examples of 129 strains include 129P1, 129P2, 129P3, 129X1, 129S1 (e.g., 129S1/SV, 129S1/Svlm), 129S2, 129S4, 129S5, 129S9/SvEvH, 129S6 (129/SvEvTac), 129S7, 129S8, 129T1, and 129T2. *See, e.g.,* Festing et al. (1999) Mammalian Genome 10:836. Examples of C57BL strains include C57BL/A, C57BL/An, C57BL/GrFa, C57BL/Kal_wN, C57BL/6, C57BL/6J, C57BL/6ByJ, C57BL/6NJ, C57BL/10, C57BL/10ScSn, C57BL/10Cr, and C57BL/Ola. Suitable mice can also be from a mix of an aforementioned 129 strain and an aforementioned C57BL/6 strain (e.g., 50% 129 and 50% C57BL/6). Likewise, suitable mice can be from a mix of aforementioned 129 strains or a mix of aforementioned BL/6 strains (e.g., the 129S6 (129/SvEvTac) strain).

Similarly, rats can be from any rat strain, including, for example, an ACI rat strain, a Dark Agouti (DA) rat strain, a Wistar rat strain, a LEA rat strain, a Sprague Dawley (SD) rat strain, or a Fischer rat strain such as Fisher F344 or Fisher F6. Rats can also be obtained from a strain derived from a mix of two or more strains recited above. For example, a suitable rat can be from a DA strain or an ACI strain. The ACI rat strain is characterized as having black agouti, with white belly and feet and an *RT1^{av1}* haplotype. Such strains are available from a variety of sources including Harlan Laboratories. The Dark Agouti (DA) rat strain is characterized as having an agouti coat and an *RT1^{av1}* haplotype. Such rats are available from a variety of sources including Charles River and Harlan Laboratories. Some suitable rats can be from an inbred rat strain. *See, e.g.,* US 2014/0235933.

### III. Methods of Making Non-Human Animals Comprising a Mutated Slc30a8 Locus

Various methods are provided for making a non-human animal genome, non-human animal cell, or non-human animal comprising a mutated *Slc30a8* locus as disclosed elsewhere herein. Any convenient method or protocol for producing a genetically modified organism is suitable for producing such a genetically modified non-human animal. *See, e.g.,* Cho et al. (2009) Current Protocols in Cell Biology 42:19.11:19.11.1-19.11.22 and Gama Sosa et al. (2010) Brain Struct. Funct. 214(2-3):91-109. Such genetically modified non-human animals can be generated, for example, through gene knock-in at a targeted *Slc30a8* locus.

For example, the method of producing a non-human animal comprising a mutated *Slc30a8* locus, *i.e.* rodent of the invention can comprise: (1) modifying the genome of a pluripotent cell to comprise the mutated *Slc30a8* locus; (2) identifying or selecting the genetically modified pluripotent cell comprising the mutated *Slc30a8* locus; (3) introducing the genetically modified pluripotent cell into a non-human animal host embryo; and (4) gestating the host embryo implanted in a surrogate mother. For example, the method of producing a non-human animal comprising a mutated *Slc30a8* locus can comprise: (1) modifying the genome of a pluripotent cell to comprise the mutated *Slc30a8* locus; (2) identifying or selecting the genetically modified pluripotent cell comprising the mutated *Slc30a8* locus; (3) introducing the genetically modified pluripotent cell into a non-human animal host embryo; and (4) gestating the host embryo in a surrogate mother. Optionally, the host embryo comprising modified pluripotent cell (e.g., a non-human ES cell) can be incubated until the blastocyst stage before being implanted into and gestated in the surrogate mother to produce an F0 non-human animal. The surrogate mother can then produce an F0 generation non-human animal comprising the mutated *Slc30a8* locus.

The methods can further comprise identifying a cell or animal having a modified target genomic locus (i.e., a mutated *Slc30a8* locus). Various methods can be used to identify cells and animals having a targeted genetic modification.

The step of modifying the genome can, for example, utilize exogenous repair templates (e.g., targeting vectors) to modify a *Slc30a8* locus to comprise an *Slc30a8* mutation disclosed herein. As one example, the targeting vector can be for generating a mutated *Slc30a8* gene at an endogenous *Slc30a8* locus (e.g., endogenous non-human animal *Slc30a8* locus), wherein the targeting vector comprises a 5' homology arm targeting a 5' target sequence at the endogenous *Slc30a8* locus and a 3' homology arm targeting a 3' target sequence at the endogenous *Slc30a8* locus, wherein the targeting vector comprises a mutation in the *Slc30a8* gene, wherein the mutated *Slc30a8* gene encodes a truncated SLC30A8 protein. As a specific example, the mutation can result in a stop codon in a codon corresponding to residue 138 in the human SLC30A8 protein.

The exogenous repair templates can be for non-homologous-end-joining-mediated insertion or homologous recombination. Exogenous repair templates can comprise deoxyribonucleic acid (DNA) or ribonucleic acid (RNA), they can be single-stranded or doublestranded, and they can be in linear or circular form. For example, a repair template can be a single-stranded oligodeoxynucleotide (ssODN).

Exogenous repair templates can also comprise nucleic acid inserts including segments of DNA to be integrated in the *Slc30a8* locus. Integration of a nucleic acid insert in the *Slc30a8* locus can result in addition of a nucleic acid sequence of interest in the *Slc30a8* locus, deletion of a nucleic acid sequence of interest in the *Slc30a8* locus, or replacement of a nucleic acid sequence of interest in the *Slc30a8* locus (i.e., deletion and insertion).

Exogenous repair templates can also comprise a heterologous sequence that is not present at an untargeted endogenous *Slc30a8* locus. For example, an exogenous repair template can comprise a selection cassette, such as a selection cassette flanked by recombinase recognition sites.

Some exogenous repair templates comprise homology arms. If the exogenous repair template acid also comprises a nucleic acid insert, the homology arms can flank the nucleic acid insert. For ease of reference, the homology arms are referred to herein as 5' and 3' (i.e., upstream and downstream) homology arms. This terminology relates to the relative position of the homology arms to the nucleic acid insert within the exogenous repair template. The 5' and 3' homology arms correspond to regions within the *Slc30a8* locus, which are referred to herein as "5' target sequence" and "3' target sequence," respectively.

A homology arm and a target sequence "correspond" or are "corresponding" to one another when the two regions share a sufficient level of sequence identity to one another to act as substrates for a homologous recombination reaction. The term "homology" includes DNA sequences that are either identical or share sequence identity to a corresponding sequence. The sequence identity between a given target sequence and the corresponding homology arm found in the exogenous repair template can be any degree of sequence identity that allows for homologous recombination to occur. For example, the amount of sequence identity shared by the homology arm of the exogenous repair template (or a fragment thereof) and the target sequence (or a fragment thereof) can be at least 50%, 55%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98%, 99% or 100% sequence identity, such that the sequences undergo homologous recombination. Moreover, a corresponding region of homology between the homology arm and the corresponding target sequence can be of any length that is sufficient to promote homologous recombination. In some targeting vectors, the intended mutation in the *Slc30a8* locus is included in one of the homology arms. In other targeting vectors, the intended mutation in the *Slc30a8* locus is included in an insert nucleic acid flanked by the homology arms.

In cells other than one-cell stage embryos, the exogenous repair template can be a "large targeting vector" or "LTVEC," which includes targeting vectors that comprise homology arms that correspond to and are derived from nucleic acid sequences larger than those typically used by other approaches intended to perform homologous recombination in cells. LTVECs also include targeting vectors comprising nucleic acid inserts having nucleic acid sequences larger than those typically used by other approaches intended to perform homologous recombination in cells. For example, LTVECs make possible the modification of large loci that cannot be accommodated by traditional plasmid-based targeting vectors because of their size limitations. For example, the targeted locus can be (i.e., the 5' and 3' homology arms can correspond to) a locus of the cell that is not targetable using a conventional method or that can be targeted only incorrectly or only with significantly low efficiency in the absence of a nick or double-strand break induced by a nuclease agent (e.g., a Cas protein). LTVECs can be of any length and are typically at least 10 kb in length. The sum total of the 5' homology arm and the 3' homology arm in an LTVEC is typically at least 10 kb.

The screening step can comprise, for example, a quantitative assay for assessing modification of allele (MOA) of a parental chromosome. For example, the quantitative assay can be carried out via a quantitative PCR, such as a real-time PCR (qPCR). The real-time PCR can utilize a first primer set that recognizes the target locus and a second primer set that recognizes a non-targeted reference locus. The primer set can comprise a fluorescent probe that recognizes the amplified sequence. Modification-of-allele (MOA) assays including loss-of-allele (LOA) and gain-of-allele (GOA) assays are described, e.g., in US 2014/0178879 and Frendewey et al. (2010) Methods Enzymol. 476:295-307. Retention assays as described in US 2016/0145646 and WO 2016/081923, can also be used.

Other examples of suitable quantitative assays include fluorescence-mediated in situ hybridization (FISH), comparative genomic hybridization, isothermic DNA amplification, quantitative hybridization to an immobilized probe(s), INVADER^{®} Probes, TAQMAN^{®} Molecular Beacon probes, or ECLIPSE^{™} probe technology (*see, e.g.*, US 2005/0144655).

An example of a suitable pluripotent cell is an embryonic stem (ES) cell (e.g., a mouse ES cell or a rat ES cell). The modified pluripotent cell can be generated, for example, through recombination by (a) introducing into the cell one or more exogenous donor nucleic acids (e.g., targeting vectors) comprising an optional insert nucleic acid flanked, for example, by 5' and 3' homology arms corresponding to 5' and 3' target sites, wherein the insert nucleic acid or one of the homology arms comprises a mutated *Slc30a8* locus or the mutation to be made at the *Slc30a8* locus; and (b) identifying at least one cell comprising in its genome the insert nucleic acid integrated at the endogenous *Slc30a8* locus. The modified pluripotent cell can be generated, for example, through recombination by (a) introducing into the cell one or more targeting vectors comprising an insert nucleic acid flanked by 5' and 3' homology arms corresponding to 5' and 3' target sites, wherein the insert nucleic acid comprises a mutated *Slc30a8* locus or the mutation to be made at the *Slc30a8* locus; and (b) identifying at least one cell comprising in its genome the insert nucleic acid integrated at the endogenous *Slc30a8* locus. Any targeting vector can be used. In one example, a large targeting vector (LTVEC) is used. The LTVEC can be, for example, at least 10 kb in length or can have 5' and 3' homology arms, the sum total of which is at least 10 kb in length. As another example, the targeting vector can be a single-stranded oligodeoxynucleotide (ssODN). The ssODN can be, for example, between about 80 to about 200 nucleotides in length.

Alternatively, the modified pluripotent cell can be generated by (a) introducing into the cell: (i) a nuclease agent, wherein the nuclease agent induces a nick or double-strand break at a recognition site within the endogenous *Slc30a8* locus; and (ii) one or more exogenous donor nucleic acids (e.g., targeting vectors) optionally comprising an insert nucleic acid flanked by, for example, 5' and 3' homology arms corresponding to 5' and 3' target sites located in sufficient proximity to the recognition site, wherein the insert nucleic acid or one of the homology arms comprises the mutated *Slc30a8* locus or the mutation to be made at the *Slc30a8* locus; and (c) identifying at least one cell comprising a modification (e.g., integration of the insert nucleic acid) at the endogenous *Slc30a8* locus. Alternatively, the modified pluripotent cell can be generated by (a) introducing into the cell: (i) a nuclease agent, wherein the nuclease agent induces a nick or double-strand break at a recognition site within the endogenous *Slc30a8* locus; and (ii) one or more targeting vectors comprising an insert nucleic acid flanked by 5' and 3' homology arms corresponding to 5' and 3' target sites located in sufficient proximity to the recognition site, wherein the insert nucleic acid comprises the mutated *Slc30a8* locus or the mutation to be made at the *Slc30a8* locus; and (c) identifying at least one cell comprising a modification (e.g., integration of the insert nucleic acid) at the endogenous *Slc30a8* locus. Any nuclease agent that induces a nick or double-strand break into a desired recognition site can be used. Examples of suitable nucleases include a Transcription Activator-Like Effector Nuclease (TALEN), a zinc-finger nuclease (ZFN), a meganuclease, and Clustered Regularly Interspersed Short Palindromic Repeats (CRISPR)/CRISPR-associated (Cas) systems (e.g., CRISPR/Cas9 systems) or components of such systems (e.g., CRISPR/Cas9). *See, e.g.,* US 2013/0309670 and US 2015/0159175. Examples of suitable methods for making targeted modifications to cells are described, e.g., in US 2016/0145646 and WO 2016/081923.

The donor cell can be introduced into a host embryo at any stage, such as the blastocyst stage or the pre-morula stage (i.e., the 4-cell stage or the 8-cell stage). Progeny that are capable of transmitting the genetic modification though the germline are generated. *See, e.g.,* US Patent No. 7,294,754.

Alternatively, the method of producing the non-human animals described elsewhere herein can comprise: (1) modifying the genome of a one-cell stage embryo to comprise the mutated *Slc30a8* locus using the methods described above for modifying pluripotent cells; (2) selecting the genetically modified embryo; and (3) gestating the genetically modified embryo implanted into a surrogate mother. Alternatively, the method of producing the non-human animals described elsewhere herein can comprise: (1) modifying the genome of a one-cell stage embryo to comprise the mutated *Slc30a8* locus using the methods described above for modifying pluripotent cells; (2) selecting the genetically modified embryo; and (3) gestating the genetically modified embryo in a surrogate mother. Progeny that are capable of transmitting the genetic modification though the germline are generated.

Nuclear transfer techniques are also described that can also be used to generate the non-human animals. Briefly, methods for nuclear transfer can include the steps of: (1) enucleating an oocyte or providing an enucleated oocyte; (2) isolating or providing a donor cell or nucleus to be combined with the enucleated oocyte; (3) inserting the cell or nucleus into the enucleated oocyte to form a reconstituted cell; (4) implanting the reconstituted cell into the womb of an animal to form an embryo; and (5) allowing the embryo to develop. In such methods, oocytes are generally retrieved from deceased animals, although they may be isolated also from either oviducts and/or ovaries of live animals. Oocytes can be matured in a variety of well-known media prior to enucleation. Enucleation of the oocyte can be performed in a number of well-known manners. Insertion of the donor cell or nucleus into the enucleated oocyte to form a reconstituted cell can be by microinjection of a donor cell under the zona pellucida prior to fusion. Fusion may be induced by application of a DC electrical pulse across the contact/fusion plane (electrofusion), by exposure of the cells to fusion-promoting chemicals, such as polyethylene glycol, or by way of an inactivated virus, such as the Sendai virus. A reconstituted cell can be activated by electrical and/or non-electrical means before, during, and/or after fusion of the nuclear donor and recipient oocyte. Activation methods include electric pulses, chemically induced shock, penetration by sperm, increasing levels of divalent cations in the oocyte, and reducing phosphorylation of cellular proteins (as by way of kinase inhibitors) in the oocyte. The activated reconstituted cells, or embryos, can be cultured in well-known media and then transferred to the womb of an animal. *See, e.g.,* US 2008/0092249, WO 1999/005266, US 2004/0177390, WO 2008/017234, and US Patent No. 7,612,250.

The various methods provided herein allow for the generation of a genetically modified non-human F0 animal wherein the cells of the genetically modified F0 animal comprise the mutated *Slc30a8* locus. Depending on the method used to generate the F0 animal, the number of cells within the F0 animal that have the mutated *Slc30a8* locus will vary. The introduction of the donor ES cells into a pre-morula stage embryo from a corresponding organism (e.g., an 8-cell stage mouse embryo) via for example, the VELOCIMOUSE^{®} method allows for a greater percentage of the cell population of the F0 animal to comprise cells having the nucleotide sequence of interest comprising the targeted genetic modification. For example, at least 50%, 60%, 65%, 70%, 75%, 85%, 86%, 87%, 87%, 88%, 89%, 90%, 91%, 92%, 93%, 94%, 95%, 96%, 97%, 98%, 99% or 100% of the cellular contribution of the non-human F0 animal can comprise a cell population having the targeted modification.

The cells of the genetically modified F0 animal can be heterozygous for the mutated *Slc30a8* locus or can be homozygous for the mutated *Slc30a8* locus.

### IV. Methods of Screening Compounds

The non-human animals, *i.e.* rodents having the mutated *Slc30a8* loci described herein can be used for screening compounds for activity potentially useful in inhibiting, ameliorating, or reducing type 2 diabetes or ameliorating type-2-diabetes-like symptoms or screening compounds for activity potentially harmful in promoting or exacerbating type 2 diabetes or type-2-diabetes-like symptoms. Wild type non-human animals or non-human animals that do not have the mutated *Slc30a8* loci described herein can also be used for screening compounds for activity potentially useful in inhibiting, ameliorating, or reducing type 2 diabetes or ameliorating type-2-diabetes-like symptoms or screening compounds for activity potentially harmful in promoting or exacerbating type 2 diabetes or type-2-diabetes-like symptoms by monitoring phenotypic aspects associated with the protective mutated *Slc30a8* loci described herein. Compounds having activity inhibiting, ameliorating, or reducing type 2 diabetes or ameliorating type-2-diabetes-like symptoms are potentially useful as therapeutics or prophylactics against type 2 diabetes. Compounds having activity promoting or exacerbating type 2 diabetes or type-2-diabetes-like symptoms are identified as toxic and should be avoided as therapeutics or in other circumstances in which they may come into contact with humans (e.g., in foods, agriculture, construction, or water supply).

Examples of compounds that can be screened include antibodies, antigen-binding proteins, site-specific DNA binding proteins (e.g., CRISPR-Cas complexes), polypeptides, beta-turn mimetics, polysaccharides, phospholipids, hormones, prostaglandins, steroids, aromatic compounds, heterocyclic compounds, benzodiazepines, oligomeric N-substituted glycines, and oligocarbamates. Large combinatorial libraries of the compounds can be constructed by the encoded synthetic libraries (ESL) method described in WO 1995/012608, WO 1993/006121, WO 1994/008051, WO 1995/035503, and WO 1995/030642. Peptide libraries can also be generated by phage display methods. *See, e.g.,* US 5,432,018. Use of libraries of guide RNAs for targeting CRISPR-Cas systems to different genes are disclosed, e.g., in WO 2014/204727, WO 2014/093701, WO 2015/065964, and WO 2016/011080.

Animal-based assays generally involve administering a compound to the non-human animal comprising the mutated *Slc30a8* locus and assessing signs or symptoms closely resembling those of type 2 diabetes, signs or symptoms related to type 2 diabetes, or phenotypic aspects related to glucose, insulin, glucose metabolism, or type 2 diabetes in humans for change in response. The change can be assessed before and after contacting the non-human animal with the compound or by performing a control experiment performed with a control animal having the same *Slc30a8* mutation (e.g., wild type cohort sibling) without the compound.

Suitable phenotypic aspects that can be monitored include, for example, capacity to secrete insulin. For example, the capacity to secrete insulin when fed a high-fat diet can be monitored. Alternatively, the capacity to secrete insulin in response to hyperglycemia, such as severe hyperglycemia, can be monitored. For example, the insulin secretion can be in response to hyperglycemia induced by insulin receptor inhibition, such as hyperglycemia caused by the insulin receptor antagonist S961.

Other suitable phenotypic aspects that can be monitored include mitochondrial gene expression (e.g., expression of genes in the mitochondrial oxidative phosphorylation (OXPHOS) system) or expression of *Hvcn1.* Examples of such mitochondrial genes are described in more detail in Example 2 and in Tables 6-8. Other suitable phenotypic aspects that can be monitored include ratio of insulin/C-peptide when fed a control chow diet or insulin clearance. Other suitable phenotypic aspects that can be monitored include islet zinc accumulation.

Other suitable phenotypic aspects that can be monitored include, for example, (1) glucose-induced insulin secretion when fed a high-fat diet (e.g., circulating insulin levels after 20 weeks being fed on a high-fat diet); (2) pancreatic beta-cell proliferation levels when fed a high-fat diet (e.g., after 20 weeks being fed on a high-fat diet); (3) number of pancreatic beta cells when fed a high-fat diet (e.g., after 20 weeks being fed on a high-fat diet); and (4) fed plasma insulin levels after blockade of the insulin receptor (e.g., with using S961). Alternatively or additionally, suitable phenotypic aspects that can be monitored include, for example, proinsulin-to-insulin ratio when fed a high-fat diet or insulin processing and health of pancreatic beta cells.

For example, activity for exacerbating type 2 diabetes can be identified by one or both of: (1) decreased capacity to secrete insulin (e.g., when fed a high-fat diet or in response to hyperglycemia) or (2) reduced insulin clearance (e.g., increased ratio of insulin/C-peptide when fed a control chow diet). Activity for exacerbating type 2 diabetes can also be identified by one or both of (1) increased mitochondrial gene expression (e.g., expression of genes in the mitochondrial oxidative phosphorylation (OXPHOS) system) or (2) decreased expression of *Hvcn1.*

Activity for exacerbating type 2 diabetes can also be identified by one or more or all of: (1) decreased glucose-induced insulin secretion when fed a high-fat diet (e.g., decreased circulating insulin levels after 20 weeks being fed on a high-fat diet); (2) decreased pancreatic beta-cell proliferation when fed a high-fat diet (e.g., after 20 weeks being fed on a high-fat diet); (3) decreased number of pancreatic beta cells when fed a high-fat diet (e.g., after 20 weeks being fed on a high-fat diet); and (4) decreased fed plasma insulin levels after blockade of the insulin receptor. Alternatively or additionally, activity for exacerbating type 2 diabetes can be identified by one or more or all of increased ratio of proinsulin-to-insulin and worsened insulin processing and health of pancreatic beta cells (e.g., as evidenced by increased ratio of proinsulin-to-insulin when fed a high-fat diet).

Alternatively, activity for ameliorating type 2 diabetes can be identified by one or both of: (1) increased capacity to secrete insulin (e.g., when fed a high-fat diet or in response to hyperglycemia) or (2) increased insulin clearance (e.g., increased ratio of insulin/C-peptide when fed a control chow diet). Activity for exacerbating type 2 diabetes can also be identified by one or both of: (1) decreased mitochondrial gene expression (e.g., expression of genes in the mitochondrial oxidative phosphorylation (OXPHOS) system) or (2) increased expression of *Hvcn1.*

Activity for ameliorating type 2 diabetes can also be identified by one or more or all of (1) increased glucose-induced insulin secretion when fed a high-fat diet (e.g., increased circulating insulin levels after 20 weeks being fed on a high-fat diet); (2) increased pancreatic beta-cell proliferation when fed a high-fat diet (e.g., after 20 weeks being fed on a high-fat diet); (3) increased number of pancreatic beta cells when fed a high-fat diet (e.g., after 20 weeks being fed on a high-fat diet); and (4) increased fed plasma insulin levels after blockade of the insulin receptor. Alternatively or additionally, activity for ameliorating type 2 diabetes can be identified by one or more or all of decreased ratio of proinsulin-to-insulin and improved insulin processing and health of pancreatic beta cells (e.g., as evidenced by decreased ratio of proinsulin-to-insulin when fed a high-fat diet).

Such phenotypic aspects can be assayed as described in the examples provided herein. The decrease or increase can be statistically significant. For example, the decrease or increase can be by at least about 1%, at least about 2%, at least about 3%, at least about 4%, at least about 5%, at least about 10%, at least about 15%, at least about 20%, at least about 30%, at least about 40%, at least about 50%, at least about 60%, at least about 70%, at least about 80%, at least about 90%, or 100%.

. If different versions of a sequence are associated with an accession number at different times, the version associated with the accession number at the effective filing date of this application is meant. The effective filing date means the earlier of the actual filing date or filing date of a priority application referring to the accession number if applicable. Likewise, if different versions of a publication, website or the like are published at different times, the version most recently published at the effective filing date of the application is meant unless otherwise indicated. Any feature, step, element, embodiment, or aspect of the invention can be used in combination with any other unless specifically indicated otherwise. The present invention has been described in some detail by way of illustration and example for purposes of clarity and understanding.

### BRIEF DESCRIPTION OF THE SEQUENCES

The nucleotide and amino acid sequences listed in the accompanying sequence listing are shown using standard letter abbreviations for nucleotide bases, and three-letter code for amino acids. The nucleotide sequences follow the standard convention of beginning at the 5' end of the sequence and proceeding forward (i.e., from left to right in each line) to the 3' end. Only one strand of each nucleotide sequence is shown, but the complementary strand is understood to be included by any reference to the displayed strand. When a nucleotide sequence encoding an amino acid sequence is provided, it is understood that codon degenerate variants thereof that encode the same amino acid sequence are also provided. The amino acid sequences follow the standard convention of beginning at the amino terminus of the sequence and proceeding forward (i.e., from left to right in each line) to the carboxy terminus.

**Table 2. Description of Sequences.**

| **SEQ ID NO** | **Type** | **Description** |
|---|---|---|
| 1 | DNA | 8084 AS Fwd Primer |
| 2 | DNA | 8084 AS WT Probe |
| 3 | DNA | 8084 AS Rev Primer |
| 4 | DNA | 8084 AS Mut Probe |
| 5 | DNA | 8084 TD Fwd Primer |
| 6 | DNA | 8084 TD Probe |
| 7 | DNA | 8084 TD Rev Primer |
| 8 | DNA | *Slc30a8* intron 2 / *Slc30a8* exon 3 pArg137^{∗} / *Slc30a8* intron 3 |
| 9 | DNA | *Slc30a8* intron 3 / Xhol / (loxP) self-deleting neomycin selection cassette |
| 10 | DNA | Self-deleting neomycin selection cassette (loxP) / ICEUI / Nhel / *Slc30a8* intron 3 |
| 11 | DNA | *Slc30a8* intron 3 / XhoI / loxP / ICEUI / Nhel / *Slc30a8* intron 3 |
| 12 | Protein | Mouse SLC30A8 WT (UniProt Q8BGG0.1) |
| 13 | Protein | Mouse SLC30A8 p.Arg137X |
| 14 | Protein | Human SLC30A8 WT (UniProt Q8IWU4.2) |
| 15 | Protein | Human SLC30A8 p.Arg138X |
| 16 | DNA | Mouse *Slc30a8* cDNA WT (NM_172816.3) |
| 17 | DNA | Human *SLC30A8* cDNA WT (NM_173851.2) |
| 18 | DNA | Mouse *Slc30a8* cDNA c.409C>T |
| 19 | DNA | Human *SLC30A8* cDNA c.412C>T |
| 20 | DNA | Mouse *Slc30a8* CDS WT |
| 21 | DNA | Human *SLC30A8* CDS WT |
| 22 | DNA | Mouse *Slc30a8* CDS c.409C>T |
| 23 | DNA | Human *SLC30A8* CDS c.412C>T |
| 24 | DNA | Deleted Sequence in Mouse *Slc30a8* in p.Arg137X Allele (MAID 8084) |
| 25 | DNA | Probe |
| 26 | DNA | F Primer |
| 27 | DNA | R Primer |

### EXAMPLES

### Example 1. Generation of Mice Comprising a Mutated Slc30a8 Locus

To generate a mouse model carrying an allele corresponding to putative human *SLC30A8* LOF allele (c.412C>T (transcript accession number NM_173851.2), p.Arg138X), a mutated mouse *Slc30a8* allele was generated (c.409C>T (transcript accession number NM_172816.3), p.Arg137X). Information on the mouse *Slc30a8* and human *SLC30A8* genes is provided in **Table 3.** An alignment of the human SLC30A8 and the mouse SLC30A8 proteins is shown in **Figure 11****.** The mutated allele has a replacement of dC for dT (c.409C>T) at the mouse *Slc30a8* codon encoding R137 in exon 3, changing the codon from CGA to TGA (p.Arg137X), making a stop codon expected to produce a truncated protein. The mutated allele also has a self-deleting neomycin selection cassette flanked by loxP sites (loxP- mPrm1-Crei-hUb1-em7-Neo-pA-loxP cassette (4,810 bp)) inserted at intron 3, deleting 29 bp of endogenous intron 3 sequence. The endogenous *Slc30a8* locus is shown in **Figure 10A****,** and the targeted *Slc30a8* locus is shown in **Figure 10B****.** The expected sequence for the region including the boundaries of *Slc30a8* intron 2 / *Slc30a8* exon 3 pArg137^{∗} / *Slc30a8* intron 3 (denoted by horizontal line "A" in **Figure 10B****)** is set forth in SEQ ID NO: 8. The expected sequence for the region including the boundaries of *Slc30a8* intron 3 / Xhol / (loxP) self-deleting cassette (denoted by horizontal line "B" in **Figure 10B****)** is set forth in SEQ ID NO: 9. The expected sequence for the region including the boundaries of self-deleting cassette (loxP) / ICEUI / NheI / *Slc30a8* intron 3 (denoted by horizontal line "C" in **Figure 10B****)** is set forth in SEQ ID NO: 10.

**Table 3. Mouse Slc30a8 and Human SLC30A8.**

| | **Mouse** | **Human** |
|---|---|---|
| **Official Symbol** | Slc30a8 | SLC30A8 |
| **NCBI GenelD** | 239436 | 169026 |
| **Primary Source** | MGI:2442682 | HGNC:20303 |
| **RefSeq mRNA ID** | NM_172816.3 | NM_173851.2 |
| **UniProt ID** | Q8BGG0 | Q8IWU4 |
| **Genomic Assembly** | GRCm38.p4 | GRCh38.p7 |
| **Location** | Chr 15: 52295553-52335733 (+) | Chr 8: 116950273-117176714 (+) |

The targeted *Slc30a8* locus after deletion of the self-deleting loxP-mPrm1-Crei-hUb1-em7-Neo-pA -loxP cassette is shown in **Figure 10C****.** After cassette deletion, loxP and cloning sites (77 bp) remain in *Slc30a8* intron 3. The expected sequence for the region including the boundaries of *Slc30a8* intron 2 / *Slc30a8* exon 3 pArg137^{∗} / *Slc30a8* intron 3 (denoted by horizontal line "A" in **Figure 10C****)** is set forth in SEQ ID NO: 8. The expected sequence for the region including the boundaries of *Slc30a8* intron 3 / Xhol / loxP / ICEUI / Nhel / *Slc30a8* intron 3 (denoted by horizontal line "B" in **Figure 10C****)** is set forth in SEQ ID NO: 11.

A large targeting vector was generated comprising a 5' homology arm including 62 kb of sequence (but with the point mutation in the mouse *Slc30a8* codon encoding R137) upstream from the region in *Slc30a8* targeted for deletion and 136 kb of the sequence downstream of the region in *Slc30a8* targeted for deletion. The large targeting vector was designed to introduce a point mutation in the mouse *Slc30a8* codon in exon 3 encoding R137 and to replace the 29 bp region in intron 3 with the 4,810 bp self-deleting neomycin selection cassette. *See* **Figure 10B****.** The self-deleting neomycin selection cassette included the following components from 5' to 3': loxP site, mouse protamine (*Prm1*) promoter, Crei (Cre coding sequence optimized to include intron), human ubiquitin promoter, EM7 promoter, neomycin phosphotransferase (neoᵣ) coding sequence, polyA, and loxP site. To generate the targeted allele, CRISPR/Cas9 components were introduced into F1H4 mouse embryonic stem cells together with the large targeting vector. Loss-of-allele assays and allele-specific TAQMAN^{®}assays using the primers and probes set forth in **Table 4** were performed to confirm correct modification of the *Slc30a8* allele. The loss-of-allele assays are denoted as "8084 TD" (TAQMAN^{®} Downstream Assay) and the allele-specific assays are denoted as "8084 AS" (Allele-Specific Assay) in **Figure 10A****.** Such assays are described, for example, in US 2014/0178879; US 2016/0145646; WO 2016/081923; and Frendewey et al. (2010) Methods Enzymol. 476:295-307.

**Table 4. Assays for Detection of Targeted Slc30a8 Locus.**

| **Assay** | **Primer or Probe** | **Sequence** |
|---|---|---|
| 8084 AS WT | Fwd | CGAGGCCCCCTTCCAA (SEQ ID NO: 1) |
| | Probe (FAM-MGB) | CATTTGGGTGGTATCGA (SEQ ID NO: 2) |
| | Rev | TGCGCAATCAGAGAATGTTACC (SEQ ID NO: 3) |
| 8084 AS Mut | Fwd | CGAGGCCCCCTTCCAA (SEQ ID NO: 1) |
| | Probe (VIC-MGB) | CATTTGGGTGGTATTGA (SEQ ID NO: 4) |
| | Rev | TGCGCAATCAGAGAATGTTACC (SEQ ID NO: 3) |
| 8084 TD | Fwd | TGTGGGAATGTGAGCTCTCAAC (SEQ ID NO: 5) |
| | Probe (Cal O-BHQ1) | CAGCTGACAGCAAGATTAATGGAAGTACC (SEQ ID NO: 6) |
| | Rev | GACCCTGGAGACAGAAATGTC (SEQ ID NO: 7) |

F0 mice were then generated from targeted ES cell clones using the VELOCIMOUSE^{®} method. *See, e.g.,* US 7,576,259; US 7,659,442; US 7,294,754; US 2008/007800; and Poueymirou et al. (2007) Nature Biotech. 25(1):91-99.

The sequence of the expected SLC30A8 protein expressed from the wild type mouse locus is set forth in SEQ ID NO: 12. The sequence of the expected truncated SLC30A8 protein expressed from the targeted mouse locus (SLC30A8 Arg137^{∗}) is set forth in SEQ ID NO: 13. An alignment of the two sequences is shown in **Figure 11****.**

### Example 2. Characterization of SLC30A8 R138X Mice.

### Summary

*SLC30A8* encodes a zinc transporter that is primarily expressed in the pancreatic islets of Langerhans. In beta cells, it transports zinc into insulin-containing secretory granules. Loss-of-function (LOF) mutations in *SLC30A8* protect against type 2 diabetes in humans. Several *Slc30a8* knockout mouse lines have been characterized but do not fully recapitulate the human phenotype. Indeed, *Slc30a8* deficient mice have reduced plasma insulin levels and impaired glucose tolerance, a phenotype that we replicate here in control *Slc30a8-deficient* mice. We generated a knock-in mouse model carrying a mutation in the mouse *Slc30a8* locus (c.409C>T (transcript accession number NM_172816.3), p.Arg137X) corresponding to the human *SLC30A8* putative LOF mutation, R138X. Although the mutation in the mouse *Slc30a8* gene is in the codon encoding amino acid residue 137 of the mouse SLC30A8 protein, these mice are referred to herein as "R138X mice" in reference to the corresponding human mutation. Interestingly, the R138X mice had increased glucose-induced insulin secretion when fed a high-fat diet. This was associated with increased beta-cell proliferation and expansion of the beta-cell area. Importantly, heterozygous R138X mice showed a similar increase in glucose-induced insulin secretion. In contrast, chow-fed R138X mice had normal body weight, glucose tolerance, and pancreatic beta-cell mass. Interestingly, in hyperglycemic conditions induced by the insulin receptor antagonist S961, the R138X mice showed about 50% increase in insulin secretion. This effect was not associated with enhanced beta-cell proliferation or beta-cell mass. These data suggest that the *SLC30A8* R138X mutation in humans protects against type 2 diabetes in part by increasing the capacity of beta cells to secrete insulin. This may in part be mediated by an increased number of pancreatic beta cells.

### Introduction

SLC30A8 is a zinc transporter (ZnT8) located in the islet cells of the endocrine pancreas. In the beta cells, SLC30A8 transports zinc into the insulin-containing granules. Two zinc ions bind with and stabilize a hexameric form of insulin triggering insulin crystallization. *See, e.g.,* Dodson and Steiner (1998) Curr. Opin. Struct. Biol. 8(2):189-194. While loss of islet SLC30A8 substantially decreases islet zinc content and insulin crystallization, its effects on proinsulin processing, secretion and regulation of glucose metabolism are still unclear. *See, e.g.,* Lemaire et al. (2009) Proc. Natl. Acad. Sci. U.S.A. 106(35): 14872-14877 and Nicolson et al. (2009) Diabetes 58(9):2070-2083. Several groups have generated *Slc30a8* knockout (KO) mouse models either globally or restricted to the pancreatic beta cells. The observed metabolic phenotype of these models is weak, and, although there are differences between studies, most of the models show either no change or a mild impairment of glucose tolerance associated with reduced or unchanged plasma insulin levels. *See, e.g.,* Rutter et al. (2016) Proc. Nutr. Soc. 75(1):61-72. Most importantly, none of the studies have reported beneficial effects associated with *Slc30a8* deficiency. Therefore, it came as a surprise when Flannick and colleagues reported that haploinsufficiency of *SLC30A8* is protective against the development of type 2 diabetes (T2D) in humans. *See, e.g.,* Flannick et al. (2014) Nat. Genet. 46(4):357-363. Their study identified 12 putative loss-of-function (LOF) variants that collectively decreased the risk for T2D by 65%. One of the most abundant variants, p.Arg138^{∗} (referred to as R138X herein), causing a premature stop codon, was individually associated with protection from T2D and was reported to encode an unstable ZnT8 protein. *See, e.g.,* Flannick et al. (2014) Nat. Genet. 46(4):357-363. Thus, mouse and human data seemingly contradict each other, and it is currently not clear how the rare putative LOF variants reduce the risk of T2D.

To investigate this discrepancy between the effect of human *SLC30A8* putative LOF variants and the *Slc30a8* KO mouse models as well as gain insight as to why *SLC30A8* putative LOF variants are protective in humans, we generated and phenotyped a new *Slc30a8* mouse model carrying the R138X mutation as a representative human putative LOF variant. Interestingly, we found that the beta cells in the R138X mice have an extraordinary capacity to secrete insulin in response to severe hyperglycemia.

### Results

**R138X Islets Appear to Show Loss of SLC30A8 Function Despite Partial Protection** of **the R138X Transcript from Nonsense-Mediated Decay.** To investigate how human *SLC30A8* putative LOF variants influence the risk of T2D, we altered the codon of *Slc30a8* in the mouse genome encoding amino acid residue 137 (codon 137, equivalent to codon 138 in human) from CGA to TGA, changing the arginine into a stop codon *(R138X)* as detailed in Example 1. The R138X mice were born with the expected Mendelian ratio and no obvious growth abnormalities. RNA *in situ* hybridization showed strong *Slc30a8* mRNA staining in pancreatic islets from wild type (WT) mice but no staining in islets from homozygous *Slc30a8* KO mice, validating the specificity of the probe **(****Figure 1A****).** In contrast to islets from *Slc30a8* KO mice, islets from R138X mice showed a substantial amount of *Slc30a8* RNA staining **(****Figure 1A****).** Quantification of *Slc30a8* mRNA using real-time PCR revealed a 70% reduction in R138X islets, although the absolute *Slc30a8* transcript level in the R138X islets (CT value: 22.2) was still high and comparable with the expression level of the housekeeping gene *Gapdh* (CT value: 22.1) (**Figure 1B****).** The presence of this high level of *Slc30a8* RNA in the R138X mice suggests that the R138X transcript cannot effectively be degraded by nonsense-mediated mRNA decay, a process that degrades mRNAs carrying premature translation termination codons. *See, e.g.,* Holbrook et al. (2004) Nat. Genet. 36(8):801-808.

To determine whether the detected RNA in the R138X mice is translated into a truncated protein, we performed Western (immuno-) blotting for SLC30A8 protein using an antibody raised against an N-terminal peptide of the mouse protein. We were not able to identify a truncated protein at the expected molecular weight (-16 kDa for the monomer) in lysates from isolated islets under the current conditions **(****Figure 1C****),** despite being able to detect the R138X protein and stabilize it by proteasomal inhibition when overexpressed in HEK293 cells **(****Figure 5****).** Similarly, mass spectrometry analysis of whole cell lysates or immunoprecipitates from WT and R138X islets did not identify SLC30A8 peptides in R138X islets (data not shown). In addition, zinc staining using dithizone revealed that islets from KO and R138X mice were zinc depleted demonstrating the absence of a functional SLC30A8 protein in both mouse lines (**Figure 1D****)**. However, no significant change was observed in circulating zinc levels **(****Figure** 17). In summary, introduction of the human R138X putative LOF variant into the mouse *Slc30a8* gene appears to result in loss of SLC30A8 function.

**R138X Mice on Chow Diet Have a Normal Metabolic Phenotype.** We first investigated whether the introduction of the R138X stop codon altered metabolic parameters in chow-fed mice. WT and R138X mice did not differ in their body weight and circulating blood glucose and insulin levels **(****Figures 2A-2C****,** **2L****; Table 5).** Because zinc has been implicated in insulin processing and clearance, we also measured circulating proinsulin and C-peptide levels and calculated the ratio of these hormones to insulin **(****Figures 2C-2F****).** While there was no difference in circulating C-peptide, fasted proinsulin levels were slightly reduced in the R138X mice **(****Figure 2C****).** The ratio of proinsulin to insulin did not differ between WT and R138X mice, but the ratio of C-peptide to insulin in the fed state was slightly elevated suggesting reduced insulin clearance **(****Figures 2E-2F****).** Circulating proinsulin and C-peptide levels were similar between WT and R138X mice **(****Figures 2C-2D****).** While the ratio of proinsulin / C-peptide did not change, we did observe a mild decrease in the ratio of insulin / C-peptide, suggesting higher insulin clearance in R138X mice similar to what has been observed in *Slc30a8* KO mice **(****Figure 2P****).** *See, e.g.,* Tamaki et al. (2013) J. Clin. Invest. 123(10):4513-4524. Reduced glucose tolerance was not apparent in the R138X mice, and we did not observe changes in glucose-induced insulin secretion **(****Figures 2G-2H****).** Glucose tolerance and insulin sensitivity were similar in the R138X and control mice, and we did not observe changes in glucose-induced insulin secretion **(****Figures 2G, 2H****,** and **2M****).** In addition, the absence of islet zinc did not affect islet morphology as insulin and glucagon staining was not altered **(****Figures 2I-2K****;** **Figures 6A-****6B).** In addition, the absence of islet zinc did not affect islet morphology as beta-cell and alpha-cell masses were not altered **(****Figures 2I****,** **2N****,** **6A,** and **6E****).** Taken together, these data show that the R138X variant does not affect glucose homeostasis or glucose-induced insulin secretion in chow-fed mice.

**Table 5. Body Weight of SLC30A8 KO and R138X Mice.**

| **Genotype** | **Sex** | **Diet** | **BW (g)** |
|---|---|---|---|
| WT | Male | Chow | 26 ± 0.4 |
| R138K | Male | Chow | 27 ± 0.4 |
| WT | Male | Chow | 29 ± 1.1 |
| KO | Male | Chow | 27 ± 0.6 |
| WT | Male | HFD | 55 ± 1.9 |
| R138X | Male | HFD | 57 ± 0.9 |
| WT | Male | HFD | 53+1.4 |
| KO | Male | HFD | 54 ± 1.2 |
| WT | Male | HFD | 51+ 0.9 |
| R138 HET | Male | HFD | 48 ± 0.8 |
| WT | Female | HFD | 54 ± 4.2 |
| R138X | Female | HFD | 54± 2.1 |

**Table 6. Body Composition of Chow-Fed SLC30A8 KO and R138X Mice.**

| **Genotype** | **Sex** | **Lean Volume (cm³)** | **Fat Volume (cm³)** | **Bone Volume (cm³)** | **Bone Density (mgHA/cm³)** | **Bone Mineral Content (mgHA)** |
|---|---|---|---|---|---|---|
| WT | Male | 13.5 ± 0.4 | 2.6 ± 0.2 | 1.1 ± 0.03 | 283 ± 3.3 | 320 ± 12.4 |
| R138X | Male | 13.4±0.3 | 3.1 ± 0.2 | 1.1+0.11 | 281 ± 2.8 | 311 ±6.6 |
| WT | Female | 13.7 ± 0.3 | 4.4 ± 0.5 | 1.2 ± 0.02 | 443 ± 3.4 | 525 ± 5.4 |
| R138X | Female | 13.8 ± 0.4 | 5.5 ± 0.5 | 1.2 ± 0.02 | 439 ± 5.2 | 519 ± 15.8 |
| WT | Male | 14.1 ± 0.3 | 4.4 ± 0.3 | 1.2 ± 0.01 | 294 ± 2.9 | 339 ± 6.3 |
| KO | Male | 13.9 ± 0.2 | 4.2 ± 0.4 | 1.2 ± 0.01 | 297 ± 2.9 | 343 ± 5.9 |

We also generated *Slc30a8* KO mice in which the coding sequence from the *Slc30a8* start codon to the *Slc30a8* stop codon (i.e., the entire coding sequence) was deleted. We then phenotyped *Slc30a8* KO mice on chow diet **(****Figures 7A-7K****).** In agreement with published results, the *Slc30a8* KO mice showed mild impaired glucose tolerance and reduced glucose-induced plasma insulin levels **(****Figures 7B, 7G, 7H****).** *See, e.g.,* Lemaire et al. (2009) Proc. Natl. Acad. Sci. U.S.A. 106(35):14872-14877; Nicolson et al. (2009) Diabetes 58(9):2070-2083; and Rutter et al. (2016) Proc. Nutr. Soc. 75(1):61-72. In addition, *Slc30a8* KO mice had a higher ratio of C-peptide to insulin, suggesting increased insulin clearance similar to a recent report and presumably reflecting impaired zinc secretion from *Slc30a8* KO islets. *See, e.g.,* Tamaki et al. (2013) J. Clin. Invest. 123(10):4513-4524 and Mitchell et al. (2016) Mol. Endocrinol. 30(1):77-91.

Taken together, these data show that introduction of the R138X putative LOF variant has minor effects on proinsulin processing and does not alter glucose homeostasis or glucose-induced insulin secretion in mice maintained on regular chow diet. This R138X phenotype thus differs from that observed in *Slc30a8* KO mice, which have impaired glucose tolerance and reduced glucose-induced plasma insulin levels when maintained on regular chow diet.

**R138X Mice on High-Fat Diet Have Increased Insulin Secretion and Beta-Cell Number.** Since carriers of the human R138X are protected from development of T2D, we challenged the mice with a high-fat diet (HFD). Body weight and fed glucose levels did not differ between WT and R138X mice (**Table 5,** **Figure 3A**). However, after 20 weeks on HFD we observed higher circulating insulin levels in fed R138X mice compared to WT mice (**Figure 3B**). While circulating C-peptide levels mimicked the insulin levels, fasted proinsulin levels were lower in the R138X mice, resulting in a decreased proinsulin to insulin ratio (**Figures 3C-****3F**). Despite the increase in fed insulin, glucose tolerance was not significantly improved in R138X mice (**Figure 3G**). To examine insulin secretion in more detail, we measured circulating insulin after an oral glucose tolerance test (oGTT). In R138X mice, an oral glucose bolus doubled the amount of circulating insulin at 15 min when compared to WT mice (**Figure 3H**). Strikingly, this was associated with an increased number of insulin-producing beta cells (**Figure 3I**). Quantification of the insulin staining showed a doubling of the insulin-positive area, as well as the islet number (**Figures 3J-3K**). In contrast, we detected no change in the number of alpha cells using glucagon staining (**Figures 6C-6D**). Ki67 staining suggested that the increase in beta-cell area was secondary to enhanced beta-cell proliferation. The number of insulin- and Ki67-double positive cells increased two-fold in the pancreas of R138X mice compared to WT mice. A similar, although less pronounced, metabolic phenotype was observed in female R138X mice (**Figures 8A-8K**). After more than 20 weeks on HFD, female R138X mice showed a reduced proinsulin to insulin ratio when fasted, and an increase in circulating insulin levels associated with a higher number of islets (**Figures 8B-8K**).

Because the protective phenotype of the R138X variant was observed in heterozygous carriers, we also analyzed heterozygous R138X mice on HFD (**Figures 9A-9M**). Western blot analysis showed a strong reduction of SLC30A8 protein in islets from heterozygous R138X mice (**Figure 9A**), while islet zinc levels were comparable to WT mice when examined by dithizone staining (**Figure 9B**). Although the phenotype was milder compared to the homozygous R138X mice, heterozygous R138X mice showed an increase in glucose-induced insulin secretion (**Figure 9J**) associated with an expansion of beta-cell area after more than 20 weeks on HFD (**Figure 9K-9M**). Taken together, these data suggest that HFD mice carrying the human R138X mutation have greater capacity to secrete insulin, which in part results from an increased number of beta cells.

As an alternative way to induce insulin resistance and increase beta-cell insulin secretion, we used the insulin receptor antagonist S961 to block the insulin receptor in R138X mice and WT mice. Blockade of the insulin receptor increased circulating glucose and insulin levels as expected in both R138X mice and WT mice. However, we observed a doubling of the circulating insulin in R138X mice compared to WT mice. *See* **Figure 12****.** SLC30A8 KO mice do not show this increase in insulin secretion over WT mice (data not shown). These data are consistent with the data suggesting that HFD mice carrying the human R138X mutation have greater capacity to secrete insulin.

***Slc30a8* KO Mice on High-Fat Diet Do Not Have Increased Circulating Insulin Levels or Beta-Cell Number.** We also examined our *Slc30a8* KO mice after 20 weeks on HFD (**Figures 4A-4K****).** These mice did not show an increase in fed circulating insulin levels, but had reduced fasted insulin, proinsulin and C-peptide levels (**Figures 4B-4D****).** Similar to the R138X mice, the proinsulin to insulin ratio was lower in the *Slc30a8* KO mice (**Figure 4E**). However, the C-peptide to insulin ratio was elevated in the *Slc30a8* KO mice similar to what was observed on chow diet. While glucose tolerance was unchanged, *Slc30a8* KO mice showed lower glucose-induced circulating insulin levels. In contrast to what we observed in the R138X mice, beta-cell number was unchanged in the *Slc30a8* KO mice (**Figures 4I-4K**).

**R138X Mice Have Increased Insulin Secretion in Response to Insulin Receptor Inhibition-Induced Hyperglycemia.** Next, we wanted to investigate a potential effect of the R138X mutation in metabolically challenged mice. We used the insulin receptor antagonist S961 to induce severe insulin resistance and hyperglycemia. *See, e.g.,* Schaffer et al. (2008) Biochem. Biophys. Res. Commun. 376(2):380-383. As expected, S961 caused hyperglycemia (approximately 600 mg/dl) in all treated animals, while the control animals kept normal fed glucose levels (approximately200 mg/dl) (**Figure 13A**). Circulating insulin levels rose in response to S961 in all treated mice. Strikingly, R138X mice had more than 50% (WT: 42.33 ± 5.03 ng/ml; R138X: 67.75 ± 19.16 ng/ml) higher circulating insulin levels upon S961-induced hyperglycemia compared to S961-treated WT mice (**Figure 13B**). The increase in plasma insulin was not a result of improved proinsulin processing or decreased insulin clearance (**Figures 14A-14E**), or changes in circulating GLP-1 levels between the R138X and WT mice (**Figure 13C**). Circulating insulin was higher in fed and fasted condition in R138X mice treated with S961, but blood glucose levels did not differ between genotypes because of the inhibition of insulin action on the insulin receptor by S961 (**Figures 13D-13E**). Of note, fasted insulin levels were substantially elevated with S961 treatment in WT and R138X mice when compared to PBS-treated mice despite normal fasting blood glucose levels.

To investigate whether the higher insulin levels in the mutant mice were a consequence of increased beta-cell proliferation, we measured Ki-67 / insulin positive beta cells. While beta-cell proliferation clearly increased with S961 treatment, there was no difference in beta-cell proliferation between R138X and WT mice (**Figures 13F-13G**). Consistent with the proliferation data, there was no difference in islet or alpha-cell mass in S961-treated WT and R138X mice (**Figures 13F** and **13H****,** **Figures 14F-14G**). Despite the large increase in blood insulin, beta cells from S961-treated R138X mice did not appear to be more insulin-depleted than beta cells from the S961-treated WT mice (**Figure 13F**). In summary, these data suggest that islets from the R138X mice have higher capacity to secrete insulin when challenged with hyperglycemia.

**Islets from R138X Mice Have Decreased Mitochondrial Gene Expression and Increased *Hvcn1* Expression.** To understand the differences between WT and R138X islets in more detail, we performed RNA sequencing on islets isolated from chow-fed mice. In confirmation with our RNAscope and TAQMAN^{®} data (**Figures 1A-1B**), the amount of *Slc30a8* mRNA was strongly reduced (from approximately 500 RPKM in WT to approximately100 RKPM in R138X), but not absent (**Figure 15**). We found that 61 genes (19 genes increased, 43 genes decreased) were differentially regulated in R138X islets (**Table 7**). Interestingly, this gene list does not contain genes involved in zinc metabolism or insulin biosynthesis. However, the expression of insulin 2 (*Ins2*) was significantly down-regulated and the expression of insulin 1 (*Ins1*) trended lower too (**Figure 16A**). This prompted us to look more carefully into the expression levels of beta-cell regulators, including transcription factors for insulin. As shown in **Figure 16B**, *Mafa* was significantly upregulated in islets of R138X mice but had missed our cutoff of 1.5-fold. Since *Mafa* positively regulates insulin transcription and its expression is usually positively associated with the expression of the hormone, it does not explain the reduction the *Ins2* gene expression. Out of the 43 down-regulated genes, 12 genes were mitochondrial proteins and 7 belonged to the group of Oxphos genes (**Table 7**). We therefore looked into the expression level of all Oxphos genes and noted that the expression of most of these genes was reduced (**Table 8, Table 9**). Complex I, III, and V were mainly affected with a reduction in gene expression of about 40%, suggesting a reduced capacity to generate ATP.

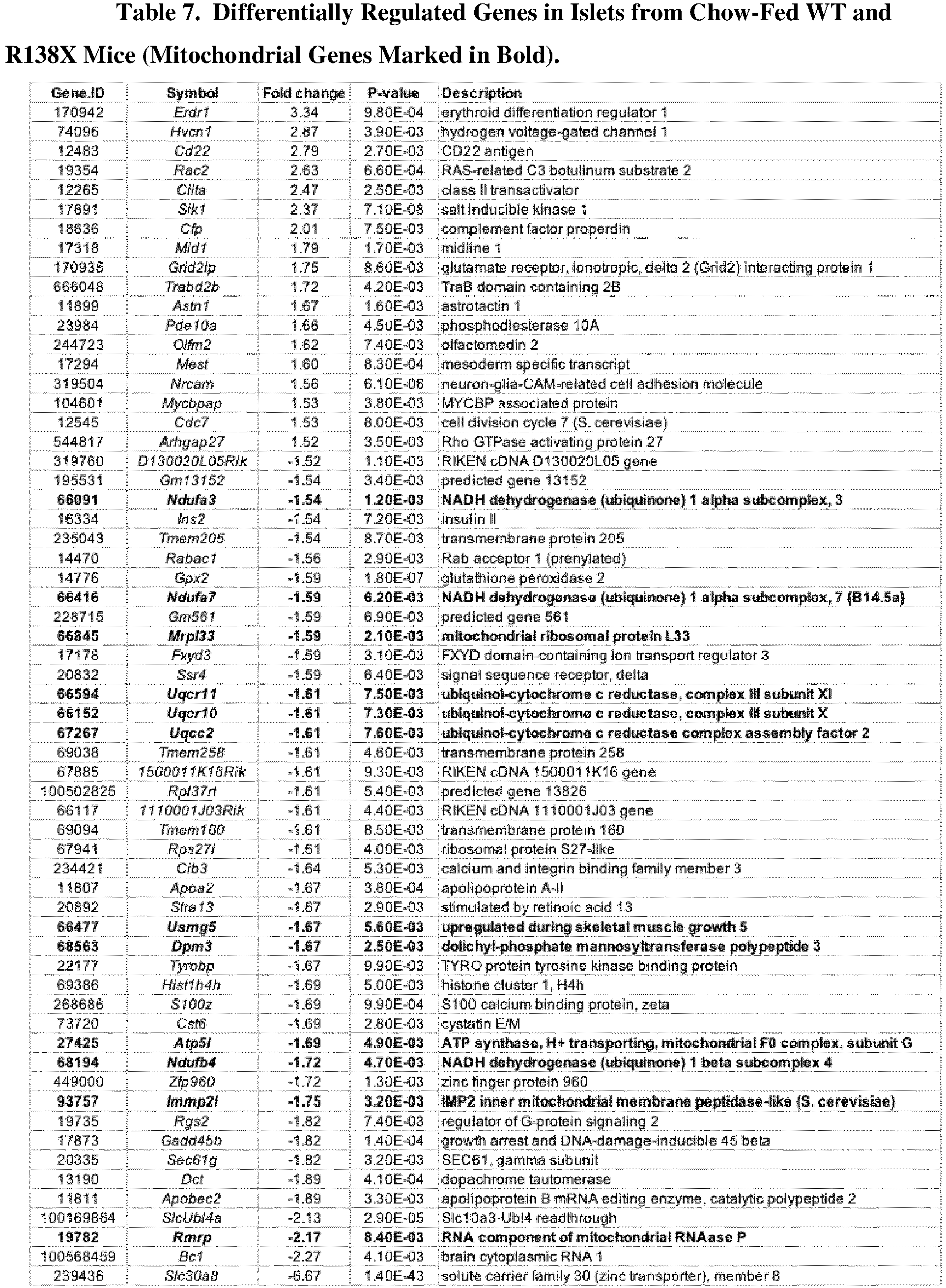

**Table 8. Summary of Oxphos Genes Expression Changes in Islets of WT and R138X Mice.**

| **Complex** | **Total** | **Significantly Regulated (p<0.05)** | **% Regulated** |
|---|---|---|---|
| Complex I | 43 | 19 | 44.19 |
| Complex II | 4 | 1 | 25.00 |
| Complex III | 10 | 4 | 40.00 |
| Complex IV | 19 | 5 | 26.32 |
| Complex V | 20 | 9 | 45.00 |

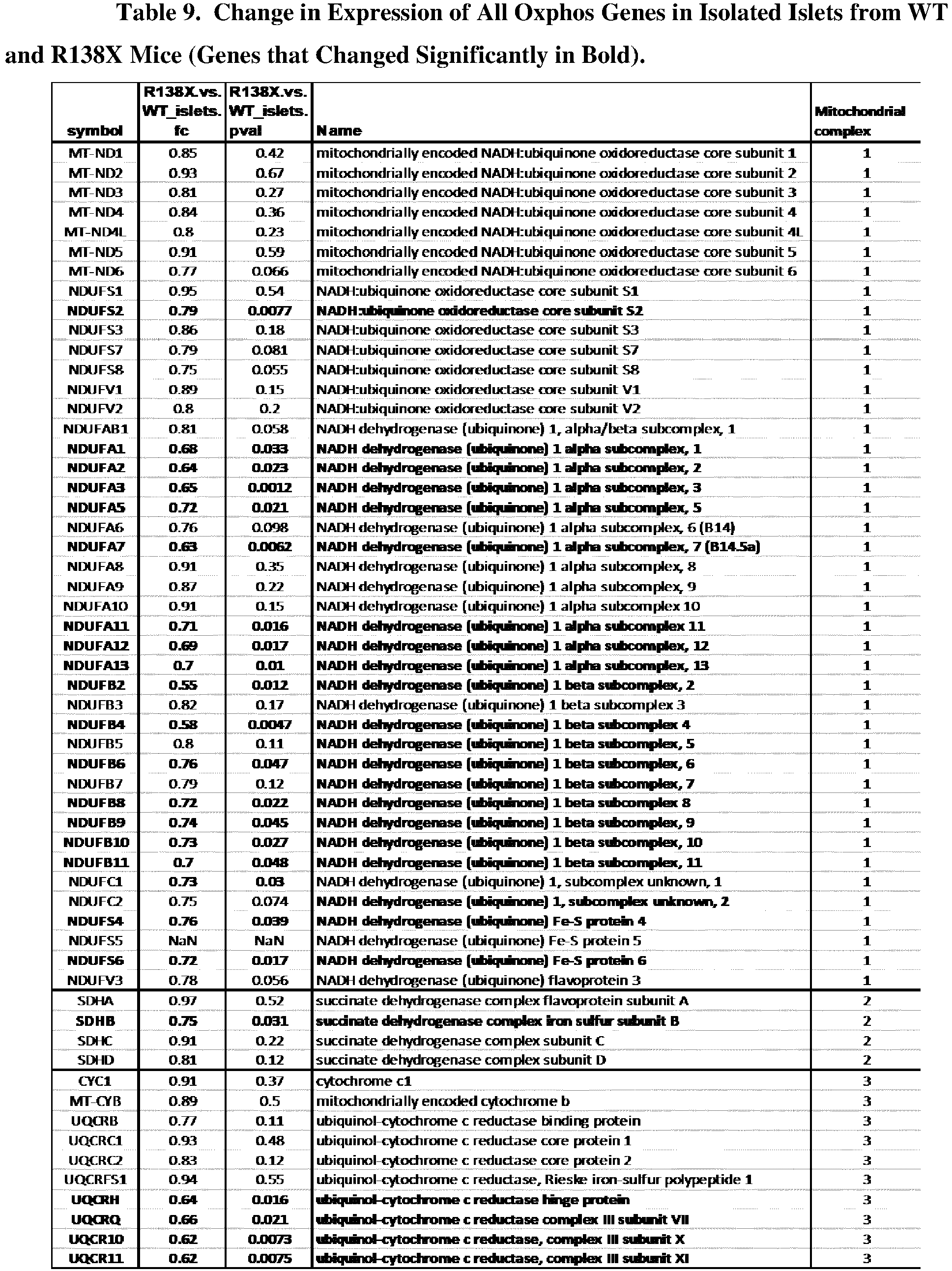

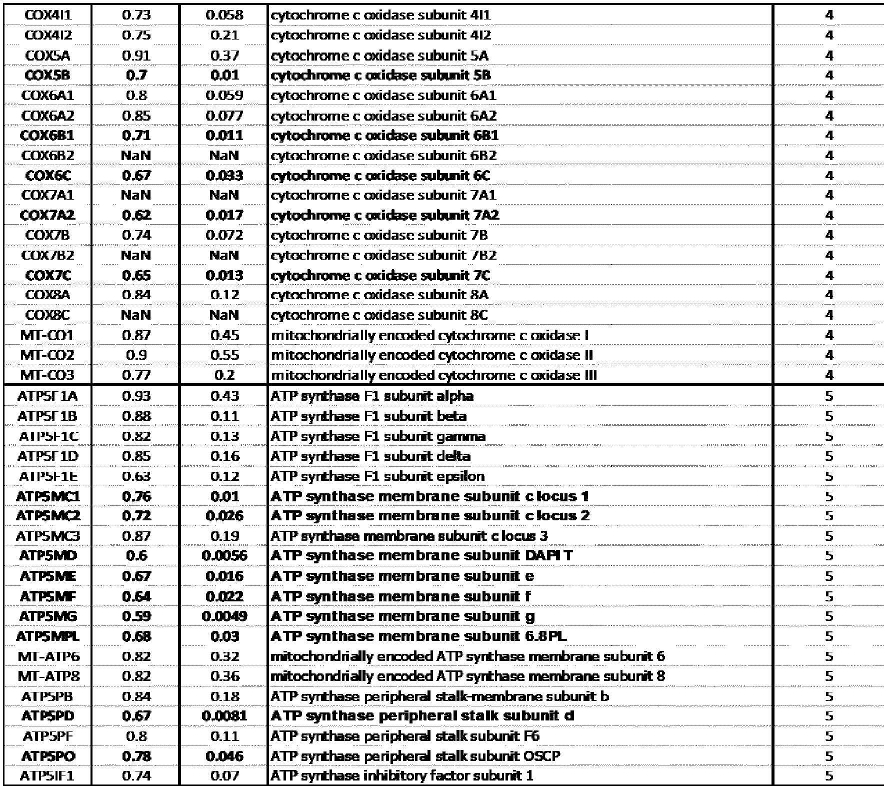

Of note, the expression of the voltage-gated proton channel Hv1 (Hvcn1, alternate names VSOP, HV1) was upregulated in islets from R138X mice. Since this channel is Zn²⁺-regulated and present in the insulin secretory granules, and loss of *Hvcn1* expression impairs insulin secretion *in vivo* and *in vitro,* this might be important for the observed phenotype in the R138X mice. *See, e.g.,* Zhao et al. (2015) Biochem. Biophys. Res. Commun. 468(4):746-751; Wang et al. (2017) "The Voltage-gated Proton Channel Hv1 Is Required for Insulin Secretion in Pancreatic β Cells," bioRxiv 097816, doi.org/10.1101/097816; and Qiu et al. (2016) Proc. Natl. Acad. Sci. U.S.A. 113(40):E5962-E5971.

### Discussion

In this study, we generated a new *Slc30a8* putative LOF mouse model (R138X mice) mimicking one of the two most common human *SLC30A8* putative LOFs (p.Arg138^{∗}). *See, e.g.,* Flannick et al. (2014) Nat. Genet. 46(4):357-363. Data obtained from these mice can for the first time explain why *SLC30A8* putative LOF mutations in humans are protective against development of T2D. Introduction of the R138X mutation resulted in an increased number of beta cells allowing for higher insulin secretion in an insulin resistant state. These data indicate that R138X beta cells can therefore better compensate for the increased insulin demand preventing and/or delaying the failure of beta cells and onset of T2D. Using this model, we show that the introduction of the R138X mutation results in the secretion of 50% more insulin in response to hyperglycemia. These data suggest that the protection from T2D in humans is mediated, at least in part, by an increase in the insulin secretory capacity allowing the beta cells to compensate for enhanced insulin demand thereby preventing or delaying the failure of these cells and onset of T2D. The present approach thus demonstrates the feasibility of using mouse genetics to explore the mechanisms of how identified human T2D risk or protective genes act. The present approach also emphasizes the utility of mouse genetics to explore the mechanisms of genetic variants which affect the risk of T2D in humans.

More than eight different *Slc30a8* knockout mouse models have been reported. *See, e.g.,* Lemaire et al. (2009) Proc. Natl. Acad. Sci. U.S.A. 106(35):14872-14877; Nicolson et al. (2009) Diabetes 58(9):2070-2083; Tamaki et al. (2013) J. Clin. Invest. 123(10):4513-4524; Pound et al. (2009) Biochem. J. 421(3):371-376; Pound et al. (2012) PLoS One 7(7):e40972; Wijesekara et al. (2010) Diabetologia 53(8):1656-1668; and Hardy et al. (2012) Am. J. Physiol. Endocrinol. Metab. 302(9):E1084-E1096. These models vary in genetic background, *Slc30a8* targeting strategy, and the specific Cre-line that was used when mice with beta-cell-specific deletions were generated. The metabolic phenotypes of these mice vary too. Differences in body weight gain, glucose tolerance and insulin secretion have been reported on chow and high-fat diet (HFD). *See, e.g.,* Rutter et al. (2016) Proc. Nutr. Soc. 75(1):61-72. However, all published *Slc30a8* knockout phenotypes point towards a worsening of glucose control with impaired glucose tolerance observed in at least six publications. For comparative reasons, we also generated a *Slc30a8* KO mouse line on 100% C57BL/6 background, same as the R138X. This knockout mouse phenocopied to a large extend the published data showing impaired glucose tolerance (on chow diet), lower glucose-induced insulin secretion (on chow and HFD), and impaired insulin clearance (on chow and HFD). On the contrary, we did not observe a major metabolic phenotype, including worsening of glucose control in the R138X mice on chow diet. The difference between the knockout and the R138X mouse lines became even more apparent after more than 20 weeks on HFD. Insulin secretion and the amount of beta cells was increased in R138X mice, but not in *Slc30a8* KO mice. The late appearance of the observed phenotype suggests that the compensatory increase in beta-cell proliferation and number happened rather late in HFD feeding. However, we cannot exclude the possibility that the number of beta cells differed already after 10 weeks on HFD when no change in insulin secretion was observed. Hardy *et al.* observed an increase in the number of beta cells and circulating insulin in their global *Slc30a8* KO mouse after a HFD treatment of 16 weeks. *See, e.g.,* Hardy et al. (2012) Am. J. Physiol. Endocrinol. Metab. 302(9):E1084-E1096. However, this was most likely a consequence of the deteriorated glucose control with marked obesity, hyperglycemia, and enhanced insulin resistance in their KO mice compared to wild type mice, an effect not observed in the present study. Importantly, while Hardy *et al.* observed an increase in proinsulin to insulin ratio in the *Slc30a8* KO mice, we observed a decrease in this ratio in the R138X mice on HFD, suggesting that the beta cells had improved insulin processing and better health than the WT beta cells. *See, e.g.,* Hardy et al. (2012) Am. J. Physiol. Endocrinol. Metab. 302(9):E1084-E1096. Interestingly, the improvement in proinsulin to insulin ratio was also observed in our *Slc30a8* KO mice, showing that knockout and R138X mice do not differ in all metabolic aspects. Since a high proinsulin to insulin ratio is associated with progression to T2D in humans, this could also contribute to *SLC30A8* LOF-mediated protection from T2D. *See, e.g.,* Kirchhoff et al. (2008) Diabetologia 51(4):597-601; Loopstra-Masters et al. (2011) Diabetologia 54(12):3047-3054; and Saad et al. (1990) J. Clin. Endocrinol. Metab. 70(5):1247-1253.

All *Slc30a8* KO phenotypes show either no change or a decrease in circulating insulin, which was in some instances associated with worsening of glucose tolerance. *See, e.g.,* Nicolson et al., (2009) Diabetes 58(9):2070-2083; Pound et al. (2009) Biochem. J. 421(3):371-376; and Mitchell et al. (2016) Mol. Endocrinol. 30(1):77-91. On the contrary, an increase in *in vitro* insulin secretion has been observed in islets from three KO models. *See, e.g.,* Nicolson et al., (2009) Diabetes 58(9):2070-2083; Tamaki et al. (2013) J. Clin. Invest. 123(10):4513-4524; and Hardy et al. (2012) Am. J. Physiol. Endocrinol. Metab. 302(9):E1084-E1096. We did not observe a major metabolic phenotype, including a change in glucose-induced insulin secretion, in chow-fed R138X mice. When challenged with S961, however, these mice were able to secrete much more insulin than their WT counterparts. This increase in insulin secretion was not secondary to an increase in beta-cell mass and has not been reported in any *Slc30a8* KO model.

It is unclear why the metabolic phenotype of the R138X mice differs so much from the *Slc30a8* KO mice, but the data suggest a potential gain of function for the R138X allele. Currently, it is unclear whether the R138X mice are like *Slc30a8* KO mice, since we cannot rule out that the remaining mRNA is translated into a truncated protein. We were not able to detect a truncated version of the SLC30A8 protein that could readily explain phenotype differences in the islets of the R138X mice. However, the RNA is clearly present, and overexpressed R138X protein was detected in HEK293 cells and accumulated by proteasomal inhibition (**Figure 5**), which is contrary to what was previously observed in HeLa cells. *See, e.g.,* Flannick et al. (2014) Nat. Genet. 46(4):357-363. Thus, we cannot exclude the existence of low levels of protein, perhaps in a subset of the islet cells from the R138X mice. Independent of the potential expression of SLC30A8 protein in R138X mice, dithizone staining clearly indicated that the introduction of the R138X putative LOF mutation resulted in a loss of islet zinc accumulation consistent with what is described in the *Slc30a8* KO mice. Another possibility is that the truncated *Slc30a8* mRNA, while no longer coding for a peptide, now serves a role as a long noncoding RNA, affecting the transcription of related genes (possibly including other *Slc30a* family members) *in trans. See, e.g.,* Batista and Chang (2013) Cell 152(6):1298-1307.

Changes in subcellular free Zn²⁺ may lead to increased proliferation and/or insulin secretion for a number of reasons. A plethora of intracellular signaling systems are affected by free Zn²⁺ in mammalian cells with almost 10% of the cellular proteome able to bind these ions. *See, e.g.,* Rutter et al. (2016) Proc. Nutr. Soc. 75(1):61-72. Although free Zn²⁺ concentrations are likely to be lowered globally in the cytosol by *Slc30a8* inactivation in the beta cell, highly localized *increases* in free Zn²⁺ close to the plasma membrane in R138X beta cells might enhance signaling by receptor tyrosine kinases (e.g. insulin or IGF1 receptors) by inhibiting protein tyrosine phosphatases, thus promoting cell growth. *See, e.g.,* Gerber et al. (2014) Diabetologia 57(8):1635-1644 and Bellomo et al. (2014) Metallomics 6(7):1229-1239. On the other hand, globally lowered cytosolic Zn²⁺ levels are expected to reduce apoptotic rates, for example by interaction with caspases. *See, e.g.,* Fukamachi et al. (1998) Biochem. Biophys. Res. Commun. 246(2):364-369.

Insulin secretion may be upregulated in R138X mice through upregulation of the voltage-gated proton channel Hvcn1. Hvcn1 is present in secretory granules and inhibited by zinc ions. *See, e.g.,* Qiu et al. (2016) Proc. Nat. Acad. Sci. U.S.A. 113(40):E5962-E5971. In addition, *Hvcn1* KO mice have decreased insulin secretion and impaired glucose control. Wang et al. (2017) "The Voltage-gated Proton Channel Hv1 Is Required for Insulin Secretion in Pancreatic β Cells," bioRxiv 097816, doi.org/10.1101/097816. Since islets from the R138X mice are zinc-depleted, one would expect an increase in the activity of this channel. On top of that, we see a 3-fold upregulation of the *Hvcn1* transcript in islets from R138X mice. While these data suggest that the increase in insulin secretion is at least in part mediated by Hvcn1, further experiments have to validate this hypothesis. Another interesting finding is the reduction of mitochondrial gene expression in R138X mice. This seems rather counterintuitive, since ATP synthesis is required for proper insulin secretion. *See, e.g.,* Wiederkehr and Wollheim (2012) Mol. Cell. Endocrinol. 353(1-2):128-137. However, a recent study described an alternative pathway to activate insulin secretion in beta cells. *See, e.g.,* De Marchi et al. (2017) J. Cell. Sci. 130(11):1929-1939. This pathway is independent of ATP synthesis (oligomycin-resistant), depends on permissive cytosolic Ca²⁺, is accompanied by smaller mitochondrial glutathione reduction, and is achieved by activating mitochondrial complex II. This is interesting taking into consideration that *Gpx2* expression (glutathione peroxidase 2) (**Table 7**) and only one gene from Oxphos Complex II was reduced in the R138X mice. Correspondingly, deletion of the protein kinase Liver Kinase B1 (LKB1) selectively in beta cells leads to marked mitochondrial defects and impaired Ca²⁺ dynamics, while glucose-stimulated insulin secretion is enhanced most likely due to the up-regulation of the latter pathway. *See, e.g.,* Swisa et al. (2015) J. Biol. Chem. 290(34):20934-20946. Further experiments examining respiration and insulin secretion can be undertaken to investigate whether this alternative pathway is predominantly used in beta cells from R138X mice.

Restoration of beta-cell number is a key strategy for the development of novel T2D drugs. One of the biggest challenges to this approach is that factors that promote beta-cell replication in rodents have often failed to do so in human beta cells. However, our data in mice modeling the human *SLC30A8* R138X mutation suggest that an increase in beta-cell number is one of the mechanisms underlying the T2D protective effects of some *SLC30A8* mutations in humans. Therefore, understanding the mechanisms underlying the observed expansion of beta-cell area in R138X mice could lead to the identification of novel therapeutic targets with higher chance of translating into humans.

In conclusion, our data from the R138X mice offer for the first time an explanation as to why humans carrying the R138X mutation are protected against T2D. *See, e.g.,* Flannick et al. (2014) Nat. Genet. 46(4):357-363. It therefore is a relevant model to study the mechanism underlying this protection. This is especially important given that increasing insulin secretion is a prominent therapeutic strategy to combat T2D. Therefore, understanding the mechanism underlying the increased insulin secretion in R138X mice could lead to the identification of novel therapeutic targets.

### Mechanism of Action

We have generated a new mouse model (R138X) mimicking the human SLC30A8 LoF mutation p.Arg138^{∗} that protects humans from the development of type 2 diabetes. R138X mice in high-fed-diet conditions have increased insulin secretion associated with increased beta-cell mass and proliferation. Alternatively, when we induce hyperglycemia using S961 (insulin receptor inhibitor), R138X mice have 50% increased insulin secretion that is independent from beta-cell mass and proliferation changes compared to WT mice. This suggests that the insulin receptor is required for the increase in proliferation in R138X mice. In addition, the insulin receptor in beta cells has been shown to be required for an increase in beta-cell proliferation in mice on high-fat diet. Since R138X mice have increased capacity to secrete insulin, the higher insulin can act on the insulin receptor in beta cells and induce proliferation to a larger extent than in WT mice. To investigate this, activation of the insulin pathway is examined by doing histology for p-Akt, p-S6, and p-Erk in pancreata from WT and R138X mice treated with high-fat diet or S961. In addition, isolated islets are examined to determine whether they have higher p-AKT and p-Erk levels and whether they proliferate more *in vitro.*

### Methods

**Animals.** All procedures were conducted in compliance with protocols approved by the Regeneron Pharmaceuticals Institutional Animal Care and Use Committee. The *Slc30a8* R138X and knockout mouse lines were made in pure C57BL/6NTac background using VELOCIGENE technology. *See, e.g.,* Valenzuela et al. (2003) Nat. Biotechnol. 21(6):652-659. For the generation of the knockout mouse line, the whole *Slc30a8* coding region was deleted and replaced with a LacZ gene. The neomycin gene was removed through the use of a self-deleting neomycin selection cassette. For the generation of the R138X mice, nucleotide 409 was changed from T into C in exon 3, which changes the arginine into a stop codon. The mutated allele has a self-deleting neomycin selection cassette flanked by loxP sites inserted at intron 3, deleting 29 bp of endogenous intron 3 sequence (CAGCTGACAGCAAGATTAATGGAAGTACC (SEQ ID NO: 24)). Mice were housed (up to five mice per cage) in a controlled environment (12-h light/dark cycle, 22±1°C, 60-70% humidity) and fed ad libitum with either chow (Purina Laboratory 23 Rodent Diet 5001, LabDiet) or high-fat diet (Research Diets, D12492; 60% fat by calories) starting at age 12-18 weeks. All data shown are compared to WT littermates.

**Glucose Tolerance Test.** Mice were fasted overnight (16 hr) followed by oral gavage of glucose (Sigma) at 2 g/kg body weight. Blood samples were obtained from the tail vein at the indicated times and glucose levels were measured using the AlphaTrak2 glucometer (Abbott). Submandibular bleeds for insulin were done at 0, 15, and 30 min post-injection in separate experiments to not interfere with glucose measurements.

**Insulin Tolerance Test.** Mice were fasted for 4 hr followed by intraperitoneal injection of either 0.75 U/kg (chow-fed) or 1.5 U/kg (high-fat-fed) of human insulin (Eli Lilly). Blood samples were obtained from the tail vein at the indicated times and glucose was measured using the AlphaTrak2 glucometer (Abbott).

**Plasma Insulin, Proinsulin and C-peptide Measurements.** Submandibular bleeds of either overnight fasted or fed animals were done in the morning or following an oGTT. Insulin was analyzed with the mouse insulin ELISA (Mercodia). Proinsulin was analyzed with the mouse proinsulin ELISA (Mercodia). C-peptide was analyzed with the mouse C-peptide ELISA (ALPCO). All ELISAs were performed according to the manufacturer's instructions.

**Histology.** Pancreata were fixed in 10% neutral buffered formalin solution for 48 h and then embedded in paraffin. Two sections of the pancreas from each animal were stained with an α-glucagon (REGN745, an α-glucagon monoclonal antibody generated in-house) or an α-insulin (Dako) antibody, and areas of glucagon and insulin positive cells were measured using Halo digital imaging analysis software (Indica Labs). The percent of glucagon and insulin positive areas in proportion to the whole pancreas area were calculated. The islet number per slide was counted and normalized to the whole pancreas area. The analysis was set to capture every cluster of insulin-positive cells down to a single cell resolution. We excluded artifacts (e.g., debris) smaller than one single beta cell (<150 µm²; area calculated using a diameter of about 14 µm). For fluorescence staining, pancreas sections were stained with a combination of α-insulin (Dako, A0564) antibody and α-KI67 antibody (R&D) followed by appropriate secondary antibodies. Fluorescent signal was detected using a microscope slide scanner (Zeiss Axio Scan.Z1). Islet cell types were quantified using the HALO image analysis using the Cytonuclear Fluorescence module (Indica Labs). The percent of glucagon- and insulin-positive areas relative to the whole pancreas area were calculated. Taking the area as basis, α-cell mass was calculated by multiplying the α-cell area for each animal by the weight of the animal's pancreas. To quantify islet mass, islet number and size was measured by counting the number of insulin-positive islets on a section. Every stained area larger than 150 µm was counted as an islet. The islet number was normalized to the whole pancreas area multiplied by the individual pancreas weight to get the islet mass.

**RNA *in situ* Hybridization.** For RNA analysis, pancreas tissue was permeabilized and hybridized with combinations of mRNA probes for mouse *Gcg, Ins2,* and *Slc30A8* according to the manufacturer's instructions (Advanced Cell Diagnostics). A fluorescent kit was used to amplify the mRNA signal. Fluorescent signal was detected using a microscope slide scanner (Zeiss Axio Scan.Z1).

**Islet Isolation and Dithizone Staining.** Mouse islets were isolated by density gradient separation after perfusing pancreas with Liberase TL (Roche, #05401020001) through the common bile duct. Following 13 min digestion at 37°C, the pancreas solution was washed and filtered through a 400-µm wire mesh strainer and islets were separated by Histopaque gradient centrifugation (Sigma-Aldrich, #H 1077) or by hand picking under a dissection microscope. Isolated islets were cultured overnight in RPMI-1640 medium (Gibco), supplemented with 10% (v/v) FBS, 10 mM HEPES, 50 µM β-mercaptoethanol, 1.0 mM sodium-pyruvate, 100 U/mL penicillin and 100 µg/mL streptomycin at 37°C with a 5% CO₂ in air atmosphere. Up to 50 islets were hand-picked and transferred to a new dish containing 100 µg/ml dithizone solution. Islets were stained for up to 10 min and transferred back into a PBS solution for microscopy (Zeiss Confocal microscope).

**Western (Immuno-) Blot Analysis.** Islets were lysed in RIPA lysis buffer and the protein concentration was determined. 20 µg of total cell lysate was resolved by SDS/PAGE using Criterion TGX 4-20% precast gel (Bio-Rad) under reducing conditions and transferred to nitrocellulose membranes. The membranes were probed with a polyclonal α-mouse-SLC30A8 Rabbit Ab (custom made from Thermo Fisher) and detected using an enhanced chemiluminescent detection system. The SLC30A8 polyclonal Ab was raised against a peptide spanning amino acids 29-43 of the mouse SLC30A8 protein.

**R138X Overexpression and Proteasomal Inhibition. Human** SLC30A8 WT and the first 137 amino acids followed by a stop codon were cloned with a C-terminal myc tag. HEK293 cells (0.4 × 10⁶ cells/well) were seeded into a 6-well plate. The next day, cells were transfected with 0.1 µg DNA/well using Mirus TransIT-TL1 following the manufacturer's instructions. Two days after transfection either DMSO, proteasomal (10 µM MG-132 or 1 µM Epoxomicin) or lysosomal inhibitors (10 µg/ml Cloroquine Salt) were spiked in. Cells were harvested after 6 h treatment in RIPA buffer. 30 µg of the cell lysates were analyzed by Western blotting.

**qPCR Analysis.** Between 37.5 nanograms and 375 nanograms of RNA, per RT-qPCR assay to be run, was mixed with SuperScript^{®} VILO^{™} Master Mix (ThermoFisher, Cat# 11755500) and cycled according to the manufacturer's instructions. The cDNA was diluted with nuclease free water to between 0.5 nanograms per microliter and 5 nanograms per microliter. RT-qPCR assays were made by combining water, mastermix (ThermoFisher, Cat #4370074 or Bioline, Cat #CSA-01113), and 20X assay mix. The assay mix was a commercially available mixture of forward and reverse primers combined with a fluorescently labeled and quenched probe sequence (ThermoFisher, Cat# 351372 or LGC BioSearch, Cat# DLO-RFBL-MIX). Samples were run in triplicate on a 384-well plate (ThermoFisher, Cat# 4343370) by pipetting 5 microliters diluted cDNA and 10 microliters of appropriate RT-PCR assay into each well. The 384-well plate was covered with an optically clear seal (Agilent, Cat# 16985-001), spun down, and read on an ABI 7900HT Fast Real-Time PCR System with 384-Well Block Module and Automation Accessory (ThermoFisher, Cat# 4329002) for 40 cycles according to the specifics of the mastermix used. The sequences of the *Slc30a8* probe and primers are as follows: Probe: TCCAAAACTGGGCAGTGAGTTCAACA (SEQ ID NO: 25), Forward primer: AATTGCAGTGCTGCTTTGC (SEQ ID NO: 26), Reverse primer: AGCTGCGGCTGTTGTTGTC (SEQ ID NO: 27).

**RNA Preparation.** Islets were isolated as described above and incubated over night at 37°C. The next day, 500 islets per genotype were picked into Trizol (Invitrogen) as one sample and kept at -80°C until RNA extraction and sequencing. Five different WT samples and four different R138X samples were analyzed. Total RNA was purified from all samples using MagMAX^{™}-96 for Microarrays Total RNA Isolation Kit (Ambion by Life Technologies) according to the manufacturer's specifications. Genomic DNA was removed using MagMAX^{™}Turbo^{™}DNase Buffer and TURBO DNase from the MagMAX kit listed above (Ambion by Life Technologies). mRNA was purified from total RNA using Dynabeads^{®} mRNA Purification Kit (Invitrogen). Strand-specific RNA-seq libraries were prepared using KAPA mRNA-Seq Library Preparation Kit (Kapa Biosystems). Twelve-cycle PCR was performed to amplify libraries. Sequencing was performed on Illumina HiSeq^{®}2500 (Illumina) by multiplexed single-read run with 33 cycles.

**RNAseq Read Mapping and Statistical Analysis of Differentially Expressed RNA.** Raw sequence data (BCL files) were converted to FASTQ format via Illumina bcl2fastq v2.17. Reads were decoded based on their barcodes and read quality was evaluated with FastQC. Reads were mapped to the mouse genome (NCBI GRCm38) using ArrayStudio^{®} software (OmicSoft^{®}) allowing two mismatches. Reads mapped to the exons of a gene were summed at the gene level. Differential expressed genes were identified by DESeq2 package and significantly perturbed genes were defined with fold changes no less than 1.5 in either up or down direction and with p-values of at least 0.01.

**Data Analyses.** Data are reported as mean ± SEM. Statistical analyses were performed using Prism 6.0 (GraphPad Software). All parameters were analyzed by two-way ANOVA (^{∗}p) and Students TTest (^{#}p) as indicated always comparing genotypes not treatments; a threshold of *P*<0.05 was considered statistically significant. Significant difference between genotypes by two-way ANOVA is indicated on the top of the graph. Significant differences of specific data points are indicated on top of the point / graph. ^{∗}p / ^{#}p<0.5, ^{∗∗}p / ^{##}p<0.01, ^{∗∗∗}p / ^{###}p<0.001, ^{∗∗∗∗}p / ^{####}p<0.0001.

## Claims

1. A rodent whose genome comprises an endogenous *Slc30a8* locus comprising a mutated *Slc30a8* gene, wherein the mutated *Slc30a8* gene comprises a nonsense mutation at the 3' end of the third exon of the *Slc30a8* gene and results in the rodent having an enhanced capacity for insulin secretion relative to a rodent without the mutation.

2. The rodent of claim 1, wherein the enhanced capacity for insulin secretion is in response to hyperglycemia,
optionally wherein the rodent has increased insulin secretion in response to hyperglycemia induced by insulin receptor inhibition relative to the rodent without the mutation, and
optionally wherein the increased insulin secretion in response to hyperglycemia induced by insulin receptor inhibition is not associated with increased beta-cell proliferation or beta-cell mass relative to the rodent without the mutation.

3. The rodent of any preceding claim, wherein the enhanced capacity for insulin secretion is when fed a high-fat diet,
optionally wherein the rodent has increased insulin secretion relative to the rodent without the mutation when fed a high-fat diet, wherein the increased insulin secretion is associated with increased beta-cell proliferation or beta-cell mass relative to the rodent without the mutation, and
optionally wherein the increased beta-cell proliferation is insulin-receptor-dependent.

4. The rodent of any preceding claim, wherein:
(I) the nonsense mutation is in a codon corresponding to the codon encoding R138 in SEQ ID NO: 14 when the SLC30A8 protein encoded by the mutated *Slc30a8* gene is optimally aligned with SEQ ID NO: 14; and/or
(II) the nonsense mutation is at a position corresponding to residue 412 in SEQ ID NO: 21 when the coding sequence of the mutated *Slc30a8* gene is optimally aligned with SEQ ID NO: 21.

5. The rodent of any preceding claim, wherein the mutated *Slc30a8* gene is endogenous to the rodent.

6. The rodent of any preceding claim, wherein the rodent is a rat or a mouse.

7. The rodent of claim 6, wherein the rodent is the mouse.

8. The rodent of claim 7, wherein the mutated *Slc30a8* gene encodes a SLC30A8 protein comprising the sequence set forth in SEQ ID NO: 13 and/or wherein the mutated *Slc30a8* gene comprises the coding sequence set forth in SEQ ID NO: 22.

9. The rodent of any preceding claim, wherein:
(I) the rodent has decreased mitochondrial gene expression relative to the rodent without the mutation; and/or
(II) the rodent has increased *Hvcn1* expression relative to the rodent without the mutation; and/or
(III) the rodent has normal glucose homeostasis and glucose-induced insulin secretion on a control chow diet relative to the rodent without the mutation; and/or
(IV) the rodent has a normal metabolic phenotype on a control chow diet relative to the rodent without the mutation; and/or
(V) *Slc30a8* mRNA expression levels in the islets of the rodent are at least 25% of *Slc30a8* mRNA expression levels in the islets of the rodent without the mutation.

10. The rodent of any preceding claim, wherein the rodent has one or more or all of the following characteristics relative to the rodent without the mutation:
(a) increased glucose-induced insulin secretion when fed the high-fat diet;
(b) increased pancreatic beta-cell proliferation when fed the high-fat diet;
(c) increased number of pancreatic beta cells when fed the high-fat diet; and
(d) increased fed plasma insulin levels after blockade of the insulin receptor.

11. The rodent of any preceding claim, wherein the rodent has one or more or all of the following characteristics relative to the rodent without the mutation:
(a) increased circulating insulin levels after fed the high-fat diet for 20 weeks;
(b) increased number of pancreatic beta cells after fed the high-fat diet for 20 weeks;
(c) decreased proinsulin-to-insulin ratio when fed the high-fat diet; and
(d) increased fed plasma insulin levels after blockade of the insulin receptor.

12. The rodent of any preceding claim, wherein the rodent is heterozygous for the mutation.

13. The rodent of any one of claims 1-11, wherein the rodent is homozygous for the mutation.

14. The rodent of any preceding claim, wherein the rodent is male.

15. The rodent of any one of claims 1-13, wherein the rodent is female.

16. A method of screening a compound for activity for ameliorating or exacerbating type-2-diabetes, comprising:
(a) contacting a subject rodent of any one of claims 1-15 with the compound; and
(b)
(I) measuring one or more of the following in the subject rodent relative to a control rodent not contacted with the compound, wherein the control rodent comprises the same *Slc30a8* mutation as the subject rodent: (1) glucose-induced insulin secretion when fed a high-fat diet; (2) pancreatic beta-cell proliferation levels when fed the high-fat diet; (3) number of pancreatic beta cells when fed the high-fat diet; and (4) fed plasma insulin levels after blockade of the insulin receptor,
whereby activity for ameliorating type 2 diabetes is identified by one or more of the following in the subject rodent compared with the control rodent: (1) increased glucose-induced insulin secretion when fed the high-fat diet; (2) increased pancreatic beta-cell proliferation when fed the high-fat diet; (3) increased number of pancreatic beta cells when fed the high-fat diet; and (4) increased fed plasma insulin levels after blockade of the insulin receptor, and
whereby activity for exacerbating type 2 diabetes is identified by one or more of the following in the subject rodent compared with the control rodent: (1) decreased glucose-induced insulin secretion when fed the high-fat diet; (2) decreased pancreatic beta-cell proliferation when fed the high-fat diet; (3) decreased number of pancreatic beta cells when fed the high-fat diet; and (4) decreased fed plasma insulin levels after blockade of the insulin receptor; or
(II) measuring one or more of the following in the subject rodent relative to a control rodent not contacted with the compound, wherein the control rodent comprises the same *Slc30a8* mutation as the subject rodent: (1) capacity to secrete insulin in response to hyperglycemia; (2) insulin clearance; (3) mitochondrial gene expression; and (4) *Hvcn1* expression,
whereby activity for ameliorating type 2 diabetes is identified by one or more of the following in the subject rodent compared with the control rodent: (1) increased capacity to secrete insulin in response to hyperglycemia; (2) increased insulin clearance; (3) decreased mitochondrial gene expression; and (4) increased *Hvcn1* expression, and
whereby activity for exacerbating type 2 diabetes is identified by one or more of the following in the subject rodent compared with the control rodent: (1) decreased capacity to secrete insulin in response to hyperglycemia; (2) decreased insulin clearance; (3) increased mitochondrial gene expression; and (4) decreased *Hvcn1* expression.

17. A rodent cell whose genome comprises an endogenous *Slc30a8* locus comprising a mutated *Slc30a8* gene, wherein the mutated *Slc30a8* gene comprises a nonsense mutation at the 3' end of the third exon of the *Slc30a8* gene, and wherein a rodent comprising the mutated *Slc30a8* gene has an enhanced capacity for insulin secretion relative to a rodent without the mutation.

18. A method for making the rodent of any one of claims 1-15, comprising:
(I)
(a) modifying the genome of a pluripotent rodent cell to comprise the endogenous *Slc30a8* locus comprising the mutated *Slc30a8* gene comprising the nonsense mutation at the 3' end of the third exon of the *Slc30a8* gene;
(b) identifying or selecting the genetically modified pluripotent rodent cell comprising the endogenous *Slc30a8* locus comprising the mutated *Slc30a8* gene;
(c) introducing the genetically modified pluripotent rodent cell into a rodent host embryo; and
(d) gestating the rodent host embryo in a rodent surrogate mother; or
(II)
(a) modifying the genome of a rodent one-cell stage embryo to comprise the endogenous *Slc30a8* locus comprising the mutated *Slc30a8* gene;
(b) selecting the genetically modified rodent one-cell stage embryo comprising the endogenous *Slc30a8* locus comprising the mutated *Slc30a8* gene; and
(c) gestating the genetically modified rodent one-cell stage embryo in a rodent surrogate mother.

## Patentansprüche

1. Nagetier, dessen Genom einen endogenen *Slc30a8-*Locus umfasst, der ein mutiertes *Slc30a8*-Gen umfasst, wobei das mutierte *Slc30a8*-Gen eine Nonsense-Mutation am 3'-Ende des dritten Exons des *Slc30a8*-Gens umfasst und dazu führt, dass das Nagetier im Vergleich zu einem Nagetier ohne die Mutation eine erhöhte Fähigkeit zur Insulinsekretion aufweist.

2. Nagetier nach Anspruch 1, wobei die erhöhte Fähigkeit zur Insulinsekretion als Reaktion auf eine Hyperglykämie auftritt,
wobei optional das Nagetier eine erhöhte Insulinsekretion als Reaktion auf eine durch Insulinrezeptorhemmung induzierte Hyperglykämie im Vergleich zu dem Nagetier ohne die Mutation aufweist, und
wobei optional die erhöhte Insulinsekretion als Reaktion auf die durch die Insulinrezeptorhemmung induzierte Hyperglykämie nicht mit einer erhöhten Betazellproliferation oder Betazellmasse im Vergleich zu dem Nagetier ohne die Mutation assoziiert ist.

3. Nagetier nach einem der vorhergehenden Ansprüche, wobei die erhöhte Fähigkeit zur Insulinsekretion auftritt, wenn es mit einer fettreichen Diät gefüttert wird,
wobei optional das Nagetier eine erhöhte Insulinsekretion im Vergleich zu dem Nagetier ohne die Mutation aufweist, wenn es mit einer fettreichen Diät gefüttert wird, wobei die erhöhte Insulinsekretion mit einer erhöhten Beta-Zellproliferation oder BetaZellmasse im Vergleich zu dem Nagetier ohne die Mutation assoziiert ist, und
wobei optional die gesteigerte Betazellproliferation insulinrezeptorabhängig ist.

4. Nagetier nach einem der vorhergehenden Ansprüche, wobei:
(I) die Nonsense-Mutation in einem Codon liegt, das dem Codon entspricht, das R138 in SEQ ID NO: 14 kodiert, wenn das SLC30A8-Protein, das von dem mutierten *Slc30a8*-Gen kodiert wird, optimal zu SEQ ID NO: 14 aligniert ist; und/oder
(II) die Nonsense-Mutation an einer Position liegt, die dem Rest 412 in SEQ ID NO: 21 entspricht, wenn die kodierende Sequenz des mutierten *Slc30a8*-Gens optimal zu SEQ ID NO: 21 aligniert ist.

5. Nagetier nach einem der vorhergehenden Ansprüche, wobei das mutierte *Slc30a8*-Gen endogen im Nagetier vorkommt.

6. Nagetier nach einem der vorhergehenden Ansprüche, wobei das Nagetier eine Ratte oder eine Maus ist.

7. Nagetier nach Anspruch 6, wobei das Nagetier die Maus ist.

8. Nagetier nach Anspruch 7, wobei das mutierte *Slc30a8*-Gen für ein SLC30A8-Protein kodiert, umfassend die in SEQ ID NO: 13 dargelegte Sequenz und/oder wobei das mutierte *Slc30a8*-Gen die in SEQ ID NO: 22 dargelegte kodierende Sequenz umfasst.

9. Nagetier nach einem der vorhergehenden Ansprüche, wobei:
(I) das Nagetier eine verringerte mitochondriale Genexpression im Vergleich zu dem Nagetier ohne die Mutation aufweist; und/oder
(II) das Nagetier eine erhöhte *Hvcn1*-Expression im Vergleich zu dem Nagetier ohne die Mutation aufweist; und/oder
(III) das Nagetier bei einer Kontrollfutterdiät im Vergleich zu dem Nagetier ohne die Mutation eine normale Glukose-Homöostase und glukoseinduzierte Insulinsekretion aufweist; und/oder
(IV) das Nagetier bei einer Kontrollfutterdiät im Vergleich zu dem Nagetier ohne die Mutation einen normalen metabolischen Phänotyp aufweist; und/oder
(V) die *Slc30a8*-mRNA-Expressionsniveaus in den Inseln des Nagetiers mindestens 25% der *Slc30a8*-mRNA-Expressionsniveaus in den Inseln des Nagetiers ohne die Mutation betragen.

10. Nagetier nach einem der vorhergehenden Ansprüche, wobei das Nagetier eines oder mehrere oder alle der folgenden Merkmale im Vergleich zu dem Nagetier ohne die Mutation aufweist:
(a) erhöhte Glukose-induzierte Insulinsekretion, wenn es mit der fettreichen Diät gefüttert wird;
(b) verstärkte Proliferation der Pankreas-Betazellen, wenn es mit der fettreichen Diät gefüttert wird;
(c) erhöhte Anzahl von Pankreas-Betazellen, wenn es mit der fettreichen Diät gefüttert wird; und
(d) erhöhte Insulinspiegel im gefütterten Plasma nach Inhibierung des Insulinrezeptors.

11. Nagetier nach einem der vorhergehenden Ansprüche, wobei das Nagetier eines oder mehrere oder alle der folgenden Merkmale im Vergleich zu dem Nagetier ohne die Mutation aufweist:
(a) erhöhte zirkulierende Insulinspiegel nach 20-wöchiger Fütterung mit fettreicher Diät;
(b) erhöhte Anzahl von Pankreas-Betazellen nach 20-wöchiger Fütterung mit fettreicher Diät;
(c) vermindertes Verhältnis von Proinsulin zu Insulin bei Fütterung mit der fettreichen Diät; und
(d) erhöhte Insulinspiegel im gefütterten Plasma nach Inhibierung des Insulinrezeptors.

12. Nagetier nach einem der vorhergehenden Ansprüche, wobei das Nagetier heterozygot für die Mutation ist.

13. Das Nagetier nach einem der Ansprüche 1-11, wobei das Nagetier homozygot für die Mutation ist.

14. Das Nagetier nach einem der vorhergehenden Ansprüche, wobei das Nagetier männlich ist.

15. Das Nagetier nach einem der Ansprüche 1-13, wobei das Nagetier weiblich ist.

16. Verfahren zum Screening einer Verbindung auf ihre Aktivität zur Verbesserung oder Verschlimmerung von Typ-2-Diabetes, umfassend:
(a) Inkontaktbringen eines betreffenden Nagetieres nach einem der Ansprüche 1-15 mit der Verbindung; und
(b)
(I) Messen eines oder mehrerer der folgenden Parameter in dem betreffenden Nagetier im Vergleich zu einem Kontrollnagetier, das nicht mit der Verbindung in Kontakt gebracht wurde, wobei das Kontrollnagetier die gleiche *Slc30a8*-Mutation wie das betreffende Nagetier aufweist: (1) Glukose-induzierte Insulinsekretion, wenn eine fettreiche Diät gefüttert wird; (2) Pankreas-Beta-Zell-Proliferationsniveaus, wenn die fettreiche Diät gefüttert wird; (3) Anzahl der Pankreas-Beta-Zellen, wenn die fettreiche Diät gefüttert wird; und (4) gefütterter Insulinspiegel im Plasma nach Inhibierung des Insulinrezeptors,
wobei die Aktivität zur Verbesserung von Typ-2-Diabetes durch eines oder mehrere der folgenden Parameter in dem betreffenden Nagetier im Vergleich zu dem Kontrollnagetier festgestellt wird: (1) erhöhte Glukose-induzierte Insulinsekretion, wenn die fettreiche Diät gefüttert wird; (2) erhöhte Pankreas-Beta-Zellen-Proliferation, wenn die fettreiche Diät gefüttert wird; (3) erhöhte Anzahl von Pankreas-Beta-Zellen, wenn die fettreiche Diät gefüttert wird; und (4) gefüttert erhöhter Insulinspiegel im Plasma nach Inhibierung des Insulinrezeptors,
wobei die Aktivität zur Verschlimmerung von Typ-2-Diabetes durch eines oder mehrere der folgenden Parameter in dem betreffenden Nagetier im Vergleich zu dem Kontrollnagetier festgestellt wird: (1) verringerte Glukose-induzierte Insulinsekretion, wenn die fettreiche Diät gefüttert wird; (2) verringerte von Pankreas-Beta-Zell-Proliferation, wenn die fettreiche Diät gefüttert wird; (3) verringerte Anzahl von Pankreas-Beta-Zellen, wenn die fettreiche Diät gefüttert wird; und (4) gefüttert verringerte Insulinspiegel im Plasma nach Blockade des Insulinrezeptors; oder
(II) Messen von einem oder mehreren der folgenden Parameter in dem betreffenden Nagetier im Vergleich zu einem Kontrollnagetier, das nicht mit der Verbindung in Kontakt gebracht wurde, wobei das Kontrollnagetier die gleiche *SIc30a8*-Mutation wie das betreffende Nagetier aufweist: (1) Fähigkeit zur Insulinsekretion als Reaktion auf Hyperglykämie; (2) Insulin-Clearance; (3) mitochondriale Genexpression; und (4) *Hvcn1*-Expression,
wobei Aktivität zur Verbesserung von Typ-2-Diabetes aus einem oder mehreren der folgenden Parameter in dem betreffenden Nagetier im Vergleich zu dem Kontrollnagetier festgestellt wird: (1) erhöhte Fähigkeit zur Insulinsekretion als Reaktion auf Hyperglykämie; (2) erhöhte Insulin-Clearance; (3) verringerte mitochondriale Genexpression; und (4) erhöhte *Hvcn1*-Expression, und
wobei Aktivität zur Verschlimmerung von Typ-2-Diabetes aus einem oder mehreren der folgenden Parameter in dem betreffenden Nagetier im Vergleich zu dem Kontrollnagetier festgestellt wird: (1) verringerte Fähigkeit zur Insulinsekretion als Reaktion auf Hyperglykämie; (2) verringerte Insulin-Clearance; (3) erhöhte mitochondriale Genexpression; und (4) verringerte *Hvcn1-*Expression.

17. Nagetierzelle, deren Genom einen endogenen *Slc30a8-*Locus umfasst, der ein mutiertes *Slc30a8*-Gen umfasst, wobei das mutierte *Slc30a8*-Gen eine Nonsense-Mutation am 3'-Ende des dritten Exons des *Slc30a8*-Gens umfasst, und wobei ein Nagetier umfassend das mutierte *Slc30a8*-Gen*,* eine erhöhte Fähigkeit zur Insulinsekretion im Vergleich zu einem Nagetier ohne die Mutation aufweist.

18. Verfahren zur Herstellung des Nagetiers nach einem der Ansprüche 1-15, umfassend:
(I)
(a) Verändern des Genoms einer pluripotenten Nagetierzelle, so dass es den endogenen *Slc30a8-*Locus umfasst, der das mutierte *Slc30a8*-Gen*,* umfassend die Nonsense-Mutation am 3'-Ende des dritten Exons des *Slc30a8*-Gens, umfasst;
(b) Identifizieren oder Selektieren der genetisch veränderten pluripotenten Nagetierzelle, umfassend den endogenen *Slc30a8*-Locus, der das mutierte *Slc30a8-*Gen umfasst;
(c) Einbringen der genetisch veränderten pluripotenten Nagetierzelle in einen Nagetier-Wirtsembryo; und
(d) Austragen des Nagetier-Wirtsembryos in einer Nagetier-Leihmutter; oder
(II)
(a) Verändern des Genoms eines Nagetier-Embryos im Ein-Zell-Stadium, so dass es den endogenen *Slc30a8*-Locus, umfassend das mutierte *Slc30a8*-Gen*,* umfasst;
(b) Selektieren des genetisch veränderten Nagetier-Embryos im Ein-Zell-Stadium, der den endogenen *Slc30a8*-Locus, umfassend das mutierte *Slc30a8*-Gen*,* umfasst; und
(c) Austragen des genetisch veränderten Nagetierembryos im Ein-Zell-Stadium in einer Nagetier-Leihmutter.

## Revendications

1. Rongeur dont le génome comprend un locus *Slc30a8* endogène comprenant un gène *Slc30a8* muté, dans lequel le gène *Slc30a8* muté comprend une mutation non-sens à l'extrémité 3' du troisième exon du gène *Slc30a8* et a pour résultat que le rongeur a une capacité accrue de sécrétion d'insuline par rapport à un rongeur sans la mutation.

2. Rongeur selon la revendication 1, dans lequel la capacité accrue de sécrétion d'insuline est en réponse à une hyperglycémie,
facultativement dans lequel le rongeur présente une sécrétion d'insuline accrue en réponse à une hyperglycémie induite par une inhibition de récepteur de l'insuline par rapport au rongeur sans la mutation, et
facultativement dans lequel l'augmentation de la sécrétion d'insuline en réponse à l'hyperglycémie induite par l'inhibition de récepteur de l'insuline n'est pas associée à une prolifération des cellules bêta ou à une masse de cellules bêta accrue par rapport au rongeur sans la mutation.

3. Rongeur selon l'une quelconque des revendications précédentes, dans lequel la capacité accrue de sécrétion d'insuline est liée à un régime riche en graisses,
facultativement dans lequel le rongeur a une sécrétion d'insuline accrue par rapport au rongeur sans la mutation lorsqu'il est alimenté avec un régime riche en graisses, dans lequel la sécrétion d'insuline accrue est associée à une prolifération de cellules bêta ou à une masse de cellules bêta accrue par rapport au rongeur sans la mutation, et
facultativement dans lequel la prolifération de cellules bêta accrue est dépendante du récepteur de l'insuline.

4. Rongeur selon l'une quelconque des revendications précédentes, dans lequel :
(I) la mutation non-sens est dans un codon correspondant au codon codant pour R138 dans SEQ ID NO : 14 lorsque la protéine SLC30A8 codée par le gène *Slc30a8* muté est alignée de manière optimale avec SEQ ID NO : 14 ; et/ou
(II) la mutation non-sens est à une position correspondant au résidu 412 dans la SEQ ID NO : 21 lorsque la séquence codante du gène *Slc30a8* muté est alignée de manière optimale avec SEQ ID NO : 21.

5. Rongeur selon l'une quelconque des revendications précédentes, dans lequel le gène *Slc30a8* muté est endogène au rongeur.

6. Rongeur selon l'une quelconque des revendications précédentes, dans lequel le rongeur est un rat ou une souris.

7. Rongeur selon la revendication 6, dans lequel le rongeur est la souris.

8. Rongeur selon la revendication 7, dans lequel le gène *Slc30a8* muté code pour une protéine SLC30A8 comprenant la séquence indiquée dans SEQ ID NO : 13 et/ou dans lequel le gène *Slc30a8* muté comprend la séquence codante indiquée dans SEQ ID NO : 22.

9. Rongeur selon l'une quelconque des revendications précédentes, dans lequel :
(I) le rongeur a une expression génique mitochondriale réduite par rapport au rongeur sans la mutation ; et/ou
(II) le rongeur a une expression de *Hvcn1* accrue par rapport au rongeur sans la mutation ; et/ou
(III) le rongeur présente une homéostasie du glucose et une sécrétion d'insuline induite par le glucose normales souss un régime alimentaire témoin par rapport au rongeur sans la mutation ; et/ou
(IV) le rongeur a un phénotype métabolique normal sous un régime alimentaire témoin par rapport au rongeur sans la mutation ; et/ou
(V) les niveaux d'expression d'ARNm de *Slc30a8* dans les îlots du rongeur sont d'au moins 25 % des niveaux d'expression d'ARNm de *Slc30a8* dans les îlots du rongeur sans la mutation.

10. Rongeur selon l'une quelconque des revendications précédentes, dans lequel le rongeur a une ou plusieurs ou toutes les caractéristiques suivantes par rapport au rongeur sans la mutation :
(a) sécrétion d'insuline induite par le glucose accrue lorsqu'alimenté avec le régime riche en graisses ;
(b) prolifération de cellules bêta pancréatiques accrue lorsqu'alimenté avec le régime riche en graisses ;
(c) nombre accru de cellules bêta pancréatiques lorsqu'alimenté avec le régime riche en graisses ; et
(d) niveaux d'insuline plasmatique accrus après blocage du récepteur de l'insuline.

11. Rongeur selon l'une quelconque des revendications précédentes, dans lequel le rongeur a une ou plusieurs ou toutes les caractéristiques suivantes par rapport au rongeur sans la mutation :
(a) niveaux d'insuline circulante accrus après 20 semaines d'alimentation avec le régime riche en graisses ;
(b) nombre de cellules bêta pancréatiques accru après 20 semaines d'alimentation avec le régime riche en graisses ;
(c) rapport proinsuline/insuline réduit lorsqu'alimenté avec le régime riche en graisses ; et
(d) niveaux d'insuline plasmatique après alimentation accrus après blocage du récepteur de l'insuline.

12. Rongeur selon l'une quelconque des revendications précédentes, dans lequel le rongeur est hétérozygote pour la mutation.

13. Rongeur selon l'une quelconque des revendications 1 à 11, dans lequel le rongeur est homozygote pour la mutation.

14. Rongeur selon l'une quelconque des revendications précédentes, dans lequel le rongeur est un mâle.

15. Rongeur selon l'une quelconque des revendications 1 à 13, dans lequel le rongeur est une femelle.

16. Procédé de criblage d'un composé pour une activité pour améliorer ou exacerber les diabètes de type 2, comprenant :
(a) mettre en contact un rongeur sujet selon l'une quelconque des revendications 1 à 15 avec le composé ; et
(b)
(I) mesurer un ou plusieurs des éléments suivants chez le rongeur sujet par rapport à un rongeur témoin non mis en contact avec le composé, dans lequel le rongeur témoin comprend la même *mutation Slc30a8* que le rongeur sujet : (1) sécrétion d'insuline induite par le glucose lorsqu'alimenté avec un régime riche en graisses ; (2) niveaux de prolifération de cellules bêta pancréatiques lorsqu'alimenté avec le régime riche en graisses ; (3) nombre de cellules bêta pancréatiques lorsqu'alimenté avec le régime riche en graisses ; et (4) niveaux d'insuline plasmatique après alimentation, après blocage du récepteur de l'insuline,
dans lequel l'activité pour améliorer les diabètes de type 2 est identifiée par un ou plusieurs des éléments suivants chez le rongeur sujet par rapport au rongeur témoin : (1) sécrétion d'insuline induite par le glucose accrue lorsqu'alimenté avec le régime riche en graisses ; (2) prolifération de cellules bêta pancréatiques accrue lorsqu'alimenté avec le régime riche en graisses ; (3) nombre accru de cellules bêta pancréatiques lorsqu'alimenté avec le régime riche en graisses ; et (4) niveaux d'insuline plasmatique après alimentation accrus après blocage du récepteur de l'insuline, et
dans lequel l'activité pour exacerber les diabètes de type 2 est identifiée par un ou plusieurs des éléments suivants chez le rongeur sujet par rapport au rongeur témoin : (1) sécrétion d'insuline induite par le glucose diminuée lorsqu'alimenté avec le régime riche en graisses ; (2) prolifération de cellules bêta pancréatiques diminuée lorsqu'alimenté avec le régime riche en graisses ; (3) nombre de cellules bêta pancréatiques diminué lorsqu'alimenté avec le régime riche en graisses ; et (4) niveaux d'insuline plasmatique après alimentation diminués après blocage du récepteur de l'insuline ; ou
(II) mesurer un ou plusieurs des éléments suivants chez le rongeur sujet par rapport à un rongeur témoin non mis en contact avec le composé, dans lequel le rongeur témoin comprend la même mutation *Slc30a8* que le rongeur sujet : (1) capacité à sécréter de l'insuline en réponse à l'hyperglycémie ; (2) clairance de l'insuline ; (3) expression génique mitochondriale ; et (4) expression de *Hvcn1,*
dans lequel l'activité pour améliorer les diabètes de type 2 est identifiée par un ou plusieurs des éléments suivants chez le rongeur sujet par rapport au rongeur témoin : (1) augmentation de la capacité à sécréter de l'insuline en réponse à l'hyperglycémie ; (2) augmentation de la clairance de l'insuline ; (3) diminution de l'expression génique mitochondriale ; et (4) augmentation de l'expression de *Hvcnl,* et
dans lequel l'activité pour exacerber les diabètes de type 2 est identifiée par un ou plusieurs des éléments suivants chez le rongeur sujet par rapport au rongeur témoin : (1) diminution de la capacité à sécréter de l'insuline en réponse à l'hyperglycémie ; (2) diminution de la clairance de l'insuline ; (3) augmentation de l'expression génique mitochondriale ; et (4) diminution de l'expression de *Hvcnl.*

17. Cellule de rongeur dont le génome comprend un locus *Slc30a8* endogène comprenant un gène *Slc30a8* muté, dans laquelle le gène *Slc30a8* muté comprend une mutation non-sens à l'extrémité 3' du troisième exon du gène *Slc30a8,* et dans laquelle un rongeur comprenant le gène *Slc30a8* muté a une capacité accrue de sécrétion d'insuline par rapport à un rongeur sans la mutation.

18. Procédé de production du rongeur selon l'une quelconque des revendications 1 à 15, comprenant :
(I)
(a) modifier le génome d'une cellule de rongeur pluripotente pour comprendre le locus *Slc30a8* endogène comprenant le gène *Slc30a8* muté comprenant la mutation non-sens à l'extrémité 3' du troisième exon du gène *Slc30a8* ;
(b) identifier ou sélectionner la cellule de rongeur pluripotente génétiquement modifiée comprenant le locus *Slc30a8* endogène comprenant le gène *Slc30a8* muté ;
(c) introduire la cellule de rongeur pluripotente génétiquement modifiée dans un embryon hôte de rongeur ; et
(d) permettre une gestation de l'embryon hôte de rongeur chez une mère porteuse de rongeur ; ou
(II)
(a) modifier le génome d'un embryon de stade unicellulaire de rongeur pour comprendre le locus *Slc30a8* endogène comprenant le gène *Slc30a8* muté ;
(b) sélectionner l'embryon de stade unicellulaire de rongeur génétiquement modifié comprenant le *locus Slc30a8* endogène comprenant le gène *Slc30a8* muté; et
(c) permettre une gestation de l'embryon de rongeur génétiquement modifié à un stade cellulaire dans une mère porteuse de rongeur.


### SEQUENCE LISTING

<110> Regeneron Pharmaceuticals, Inc.
<120> NON-HUMAN ANIMALS COMPRISING SLC30A8 MUTATION AND METHODS OF USE
<130> 57766-523060
<150> US 62/584,228
   <151> 2017-11-10
<150> US 62/666,337
   <151> 2018-05-03
<150> US 62/689,945
   <151> 2018-06-26
<160> 27
<170> PatentIn version 3.5
<210> 1
   <211> 16
   <212> DNA
   <213> Artificial Sequence
<220>
   <223> Synthetic
<400> 1
   cgaggccccc ttccaa 16
<210> 2
   <211> 17
   <212> DNA
   <213> Artificial Sequence
<220>
   <223> Synthetic
<400> 2
   catttgggtg gtatcga 17
<210> 3
   <211> 22
   <212> DNA
   <213> Artificial Sequence
<220>
   <223> Synthetic
<400> 3
   tgcgcaatca gagaatgtta cc 22
<210> 4
   <211> 17
   <212> DNA
   <213> Artificial Sequence
<220>
   <223> Synthetic
<400> 4
   catttgggtg gtattga 17
<210> 5
   <211> 22
   <212> DNA
   <213> Artificial Sequence
<220>
   <223> Synthetic
<400> 5
   tgtgggaatg tgagctctca ac 22
<210> 6
   <211> 29
   <212> DNA
   <213> Artificial Sequence
<220>
   <223> Synthetic
<400> 6
   cagctgacag caagattaat ggaagtacc 29
<210> 7
   <211> 21
   <212> DNA
   <213> Artificial Sequence
<220>
   <223> Synthetic
<400> 7
   gaccctggag acagaaatgt c 21
<210> 8
   <211> 229
   <212> DNA
   <213> Artificial Sequence
<220>
   <223> Synthetic
<220>
   <221> misc_feature
   <222> (1)..(51)
   <223> Slc30a8 intron 2
<220>
   <221> misc_feature
   <222> (52)..(198)
   <223> Slc30a8 exon3 pArg137X
<220>
   <221> misc_feature
   <222> (199)..(229)
   <223> Slc30a8 intron 3
<400> 8
   cctaattgca ttcccagcct ttaacccagt gtgatattgt gcatctcaca ggtggacacg       60
   ttgctgggag tctggctatc ctcactgatg cggctcatct cttaattgac ctgactagtt      120
   tcctgctcag tctcttttct ttgtggttgt catcgaggcc cccttccaag cggctgacat      180
   ttgggtggta ttgagcaggt aacattctct gattgcgcaa tagtgaatg                  229
<210> 9
   <211> 224
   <212> DNA
   <213> Artificial Sequence
<220>
   <223> Synthetic
<220>
   <221> misc_feature
   <222> (1)..(47)
   <223> Slc30a8 intron 3
<220>
   <221> misc_feature
   <222> (48)..(53)
   <223> XhoI
<220>
   <221> misc_feature
   <222> (54)..(87)
   <223> loxP
<220>
   <221> misc_feature
   <222> (54)..(224)
   <223> Self-deleting neomycin selection cassette
<400> 9
   ctcaggataa aataaccatg tgggaatgtg agctctcaac attaattctc gagataactt       60
   cgtataatgt atgctatacg aagttatatg catgccagta gcagcaccca cgtccacctt      120
   ctgtctagta atgtccaaca cctccctcag tccaaacact gctctgcatc catgtggctc      180
   ccatttatac ctgaagcact tgatggggcc tcaatgtttt acta                       224
<210> 10
   <211> 273
   <212> DNA
   <213> Artificial Sequence
<220>
   <223> Synthetic
<220>
   <221> misc_feature
   <222> (1)..(129)
   <223> Self-deleting neomycin selection cassette
<220>
   <221> misc_feature
   <222> (90)..(123)
   <223> loxP
<220>
   <221> misc_feature
   <222> (130)..(155)
   <223> ICEUI
<220>
   <221> misc_feature
   <222> (156)..(161)
   <223> NheI
<220>
   <221> misc_feature
   <222> (162)..(273)
   <223> Slc30a8 intron 3
<400> 10
   gatcagcagc ctctgttcca catacacttc attctcagta ttgttttgcc aagttctaat        60
   tccatcagac ctcgacctgc agcccctaga taacttcgta taatgtatgc tatacgaagt       120
   tatgctaggt aactataacg gtcctaaggt agcgagctag ctcctctact atgggacaac       180
   agaaatagct gtatcgtgat gccagacatt tctgtctcca gggtctagga gatgattctt       240
   tcggttactc tacatccttc gaagaaagga tga                                    273
<210> 11
   <211> 424
   <212> DNA
   <213> Artificial Sequence
<220>
   <223> Synthetic
<220>
   <221> misc_feature
   <222> (1)..(137)
   <223> Slc30a8 intron 3
<220>
   <221> misc_feature
   <222> (138)..(143)
   <223> XhoI
<220>
   <221> misc_feature
   <222> (144)..(177)
   <223> loxP
<220>
   <221> misc_feature
   <222> (184)..(209)
   <223> ICEUI
<220>
   <221> misc_feature
   <222> (210)..(215)
   <223> NheI
<220>
   <221> misc_feature
   <222> (216)..(424)
   <223> Slc30a8 intron 3
<400> 11
   atgatataga ttcaaacaga gcttgaaaaa aagggggaag gtaaagagaa agccttgccc      60
   aataattccc tctgggctac aagcctgctg ctcaggataa aataaccatg tgggaatgtg     120
   agctctcaac attaattctc gagataactt cgtataatgt atgctatacg aagttatgct     180
   aggtaactat aacggtccta aggtagcgag ctagctcctc tactatggga caacagaaat     240
   agctgtatcg tgatgccaga catttctgtc tccagggtct aggagatgat tctttcggtt     300
   actctacatc cttcgaagaa aggatgaagg cagagaatct agggtttagt gattgtccct     360
   agggagttag gcataaaata tggactttct taatcacaca catagtcatt cctacgatct     420
   gaat                                                                  424
<210> 12
   <211> 367
   <212> PRT
   <213> Mus musculus
<400> 12
   Met Glu Phe Leu Glu Arg Thr Tyr Leu Val Asn Asp Gln Ala Thr Lys
   1               5                   10                  15
   Met Tyr Ala Phe Pro Leu Asp Arg Glu Leu Arg Gln Lys Pro Val Asn
               20                  25                  30
   Lys Asp Gln Cys Pro Gly Asp Arg Pro Glu His Pro Glu Ala Gly Gly
           35                  40                  45
   Ile Tyr His Cys His Asn Ser Ala Lys Ala Thr Gly Asn Arg Ser Ser
       50                  55                  60
   Lys Gln Ala His Ala Lys Trp Arg Leu Cys Ala Ala Ser Ala Ile Cys
   65                  70                  75                  80
   Phe Ile Phe Met Val Ala Glu Val Val Gly Gly His Val Ala Gly Ser
                   85                  90                  95
   Leu Ala Ile Leu Thr Asp Ala Ala His Leu Leu Ile Asp Leu Thr Ser
               100                 105                 110
   Phe Leu Leu Ser Leu Phe Ser Leu Trp Leu Ser Ser Arg Pro Pro Ser
           115                 120                 125
   Lys Arg Leu Thr Phe Gly Trp Tyr Arg Ala Glu Ile Leu Gly Ala Leu
       130                 135                 140
   Leu Ser Val Leu Cys Ile Trp Val Val Thr Gly Val Leu Leu Tyr Leu
   145                 150                 155                 160
   Ala Cys Glu Arg Leu Leu Tyr Pro Asp Tyr Gln Ile Gln Ala Gly Ile
                   165                 170                 175
   Met Ile Thr Val Ser Gly Cys Ala Val Ala Ala Asn Ile Val Leu Thr
               180                 185                 190
   Met Ile Leu His Gln Arg Asn Phe Gly Tyr Asn His Lys Asp Val Gln
           195                 200                 205
   Ala Asn Ala Ser Val Arg Ala Ala Phe Val His Ala Leu Gly Asp Val
       210                 215                 220
   Phe Gln Ser Ile Ser Val Leu Ile Ser Ala Leu Ile Ile Tyr Phe Lys
   225                 230                 235                 240
   Pro Asp Tyr Lys Ile Ala Asp Pro Val Cys Thr Phe Ile Phe Ser Ile
                   245                 250                 255
   Leu Val Leu Ala Ser Thr Val Met Ile Leu Lys Asp Phe Ser Ile Leu
               260                 265                 270
   Leu Met Glu Gly Val Pro Lys Gly Leu Ser Tyr Asn Ser Val Lys Glu
           275                 280                 285
   Ile Ile Leu Ala Val Asp Gly Val Ile Ser Val His Ser Leu His Ile
       290                 295                 300
   Trp Ser Leu Thr Val Asn Gln Val Ile Leu Ser Val His Val Ala Thr
   305                 310                 315                 320
   Ala Ala Ser Gln Asp Ser Gln Ser Val Arg Thr Gly Ile Ala Gln Ala
                   325                 330                 335
   Leu Ser Ser Phe Asp Leu His Ser Leu Thr Ile Gln Ile Glu Ser Ala
               340                 345                 350
   Ala Asp Gln Asp Pro Ser Cys Leu Leu Cys Glu Asp Pro Gln Asp
           355                 360                 365
<210> 13
   <211> 136
   <212> PRT
   <213> Artificial Sequence
<220>
   <223> Synthetic
<400> 13
   Met Glu Phe Leu Glu Arg Thr Tyr Leu Val Asn Asp Gln Ala Thr Lys
   1               5                   10                  15
   Met Tyr Ala Phe Pro Leu Asp Arg Glu Leu Arg Gln Lys Pro Val Asn
               20                  25                  30
   Lys Asp Gln Cys Pro Gly Asp Arg Pro Glu His Pro Glu Ala Gly Gly
           35                  40                  45
   Ile Tyr His Cys His Asn Ser Ala Lys Ala Thr Gly Asn Arg Ser Ser
       50                  55                  60
   Lys Gln Ala His Ala Lys Trp Arg Leu Cys Ala Ala Ser Ala Ile Cys
   65                  70                  75                  80
   Phe Ile Phe Met Val Ala Glu Val Val Gly Gly His Val Ala Gly Ser
                   85                  90                  95
   Leu Ala Ile Leu Thr Asp Ala Ala His Leu Leu Ile Asp Leu Thr Ser
               100                 105                 110
   Phe Leu Leu Ser Leu Phe Ser Leu Trp Leu Ser Ser Arg Pro Pro Ser
           115                 120                 125
   Lys Arg Leu Thr Phe Gly Trp Tyr
       130                 135
<210> 14
   <211> 369
   <212> PRT
   <213> Homo sapiens
<400> 14
   Met Glu Phe Leu Glu Arg Thr Tyr Leu Val Asn Asp Lys Ala Ala Lys
   1               5                   10                  15
   Met Tyr Ala Phe Thr Leu Glu Ser Val Glu Leu Gln Gln Lys Pro Val
               20                  25                  30
   Asn Lys Asp Gln Cys Pro Arg Glu Arg Pro Glu Glu Leu Glu Ser Gly
   35 40 45
   Gly Met Tyr His Cys His Ser Gly Ser Lys Pro Thr Glu Lys Gly Ala
   50 55 60
   Asn Glu Tyr Ala Tyr Ala Lys Trp Lys Leu Cys Ser Ala Ser Ala Ile
   65 70 75 80
   Cys Phe Ile Phe Met Ile Ala Glu Val Val Gly Gly His Ile Ala Gly
   85 90 95
   Ser Leu Ala Val Val Thr Asp Ala Ala His Leu Leu Ile Asp Leu Thr
   100 105 110
   Ser Phe Leu Leu Ser Leu Phe Ser Leu Trp Leu Ser Ser Lys Pro Pro
   115 120 125
   Ser Lys Arg Leu Thr Phe Gly Trp His Arg Ala Glu Ile Leu Gly Ala
   130 135 140
   Leu Leu Ser Ile Leu Cys Ile Trp Val Val Thr Gly Val Leu Val Tyr
   145 150 155 160
   Leu Ala Cys Glu Arg Leu Leu Tyr Pro Asp Tyr Gln Ile Gln Ala Thr
   165 170 175
   Val Met Ile Ile Val Ser Ser Cys Ala Val Ala Ala Asn Ile Val Leu
   180 185 190
   Thr Val Val Leu His Gln Arg Cys Leu Gly His Asn His Lys Glu Val
   195 200 205
   Gln Ala Asn Ala Ser Val Arg Ala Ala Phe Val His Ala Leu Gly Asp
   210 215 220
   Leu Phe Gln Ser Ile Ser Val Leu Ile Ser Ala Leu Ile Ile Tyr Phe
   225 230 235 240
   Lys Pro Glu Tyr Lys Ile Ala Asp Pro Ile Cys Thr Phe Ile Phe Ser
   245 250 255
   Ile Leu Val Leu Ala Ser Thr Ile Thr Ile Leu Lys Asp Phe Ser Ile
   260 265 270
   Leu Leu Met Glu Gly Val Pro Lys Ser Leu Asn Tyr Ser Gly Val Lys
   275 280 285
   Glu Leu Ile Leu Ala Val Asp Gly Val Leu Ser Val His Ser Leu His
   290 295 300
   Ile Trp Ser Leu Thr Met Asn Gln Val Ile Leu Ser Ala His Val Ala
   305 310 315 320
   Thr Ala Ala Ser Arg Asp Ser Gln Val Val Arg Arg Glu Ile Ala Lys
   325 330 335
   Ala Leu Ser Lys Ser Phe Thr Met His Ser Leu Thr Ile Gln Met Glu
   340 345 350
   Ser Pro Val Asp Gln Asp Pro Asp Cys Leu Phe Cys Glu Asp Pro Cys
   355 360 365
   Asp
<210> 15
   <211> 137
   <212> PRT
   <213> Homo sapiens
<400> 15
   Met Glu Phe Leu Glu Arg Thr Tyr Leu Val Asn Asp Lys Ala Ala Lys
   1 5 10 15
   Met Tyr Ala Phe Thr Leu Glu Ser Val Glu Leu Gln Gln Lys Pro Val
   20 25 30
   Asn Lys Asp Gln Cys Pro Arg Glu Arg Pro Glu Glu Leu Glu Ser Gly
   35 40 45
   Gly Met Tyr His Cys His Ser Gly Ser Lys Pro Thr Glu Lys Gly Ala
   50 55 60
   Asn Glu Tyr Ala Tyr Ala Lys Trp Lys Leu Cys Ser Ala Ser Ala Ile
   65 70 75 80
   Cys Phe Ile Phe Met Ile Ala Glu Val Val Gly Gly His Ile Ala Gly
   85 90 95
   Ser Leu Ala Val Val Thr Asp Ala Ala His Leu Leu Ile Asp Leu Thr
   100 105 110
   Ser Phe Leu Leu Ser Leu Phe Ser Leu Trp Leu Ser Ser Lys Pro Pro
   115 120 125
   Ser Lys Arg Leu Thr Phe Gly Trp His
       130                 135
<210> 16
   <211> 1906
   <212> DNA
   <213> Mus musculus
<400> 16
   atgtgcgcgc acgcgcgcgc gcacacacac acacacacac acacacacac acacacacac      60
   acacttatta caattgtacc tttgagtctc ccagaaaagc agtttctgtg agtgatagaa     120
   tgagtctagg tgattttcca aggagactgt taattttcac agatttaccc acaacactga     180
   tataggaggc ctcattaact caagtactgg aagaattttt ctaatttctt aggaagttgt     240
   gtgaatataa taatatcagt gcttctttac ttccaaaact ggacagcgca tcaaacatca     300
   gaaacaacag tatcagctcc tgtcccaact accatggagt ttcttgagag aacttatctt     360
   gtgaatgatc aagccaccaa gatgtacgcc ttccctctag acagagaact tcgacagaag     420
   cctgtgaata aagatcagtg tcctggagac aggccagagc atccagaggc aggaggcatc     480
   tatcactgcc acaacagcgc caaggccaca gggaacaggt cgagcaagca agcgcatgcc     540
   aagtggagac tctgtgctgc ttcagcaata tgcttcatct ttatggtggc agaggtggtg     600
   ggtggacacg ttgctgggag tctggctatc ctcactgatg cggctcatct cttaattgac     660
   ctgactagtt tcctgctcag tctcttttct ttgtggttgt catcgaggcc cccttccaag     720
   cggctgacat ttgggtggta tcgagcagag atcctcggtg ccctgctgtc tgtcctttgc     780
   atctgggtgg tgactggtgt gctgctgtac cttgcctgtg agcgcctttt gtatcctgat     840
   taccagatcc aagcaggtat catgatcact gtttcaggct gtgcagtggc agccaacatt     900
   gtactaacta tgattttgca ccaacggaac tttggctaca accacaagga tgtacaagct     960
   aatgccagtg tccgagcagc ctttgtgcat gccctggggg atgtatttca gagcatcagt    1020
   gtgctaatta gtgctctcat tatctacttt aagcctgact acaaaattgc tgatccagtg    1080
   tgcacattta tcttttccat cctggttttg gccagcaccg tcatgatctt aaaagacttc    1140
   tccatcttac tcatggaagg tgttccaaag ggcctgagtt acaacagtgt gaaagagatc    1200
   atcctcgcag ttgatggcgt gatctccgtg cacagtctac acatctggtc actgactgtg    1260
   aaccaagtga ttctctctgt tcatgttgct acagctgcca gccaggacag ccagtctgtg    1320
   cggacaggaa ttgctcaagc cctcagcagc tttgatcttc actctcttac cattcagata    1380
   gaatctgcag cagaccagga ccccagctgc cttctctgcg aagaccctca ggactagctc    1440
   ggtcacactg tcagcttcct gtgtttccta ggccatgata agatgcagca aagtttctgc    1500
   aatgcacaat gaggcagccg tcggaataga tttgagaaag tcatgatgat gcaatgtgca    1560
   cactcttcct ttgtatttat ctctatccac catgaacgag gatgcatggg atttgtcggc     1620
   ttcttggatt atacactaat cagtagttgt gctcaattgt agtatatata gattattcct     1680
   aactggagct gaaataacag atgtttgcaa tcataggtaa tgaatgattc acttgcctac     1740
   aatagtgggt atagttttac tcggaaatgc ctttctagga atccacagca tgagaaacaa     1800
   acatttgaag agaatttgag gccttagaat ttgattctgc caccatactc aatgagatct     1860
   ttattttttg tgaaacagta aaacttccct cttttgacct tgcatg                    1906
<210> 17
   <211> 5373
   <212> DNA
   <213> Homo sapiens
<400> 17
   agcagttttt gtaggtgaaa acaatgaagc caggtaatat tgcaaggagg ctgtaatttt      60
   agcagaccta ccaacaacac tgatgtagga agctcattat tttaatttct ggagcctttt     120
   aatttttctc ttagaaagtg tataaataat tgcagtgctg ctttgcttcc aaaactgggc     180
   agtgagttca acaacaacga caacaacagc cgcagctcat cctggccgtc atggagtttc     240
   ttgaaagaac gtatcttgtg aatgataaag ctgccaagat gtatgctttc acactagaaa     300
   gtgtggaact ccaacagaaa ccggtgaata aagatcagtg tcccagagag agaccagagg     360
   agctggagtc aggaggcatg taccactgcc acagtggctc caagcccaca gaaaaggggg     420
   cgaatgagta cgcctatgcc aagtggaaac tctgttctgc ttcagcaata tgcttcattt     480
   tcatgattgc agaggtcgtg ggtgggcaca ttgctgggag tcttgctgtt gtcacagatg     540
   ctgcccacct cttaattgac ctgaccagtt tcctgctcag tctcttctcc ctgtggttgt     600
   catcgaagcc tccctctaag cggctgacat ttggatggca ccgagcagag atccttggtg     660
   ccctgctctc catcctgtgc atctgggtgg tgactggcgt gctagtgtac ctggcatgtg     720
   agcgcctgct gtatcctgat taccagatcc aggcgactgt gatgatcatc gtttccagct     780
   gcgcagtggc ggccaacatt gtactaactg tggttttgca ccagagatgc cttggccaca     840
   atcacaagga agtacaagcc aatgccagcg tcagagctgc ttttgtgcat gcccttggag     900
   atctatttca gagtatcagt gtgctaatta gtgcacttat tatctacttt aagccagagt     960
   ataaaatagc cgacccaatc tgcacattca tcttttccat cctggtcttg gccagcacca    1020
   tcactatctt aaaggacttc tccatcttac tcatggaagg tgtgccaaag agcctgaatt    1080
   acagtggtgt gaaagagctt attttagcag tcgacggggt gctgtctgtg cacagcctgc    1140
   acatctggtc tctaacaatg aatcaagtaa ttctctcagc tcatgttgct acagcagcca    1200
   gccgggacag ccaagtggtt cggagagaaa ttgctaaagc ccttagcaaa agctttacga    1260
   tgcactcact caccattcag atggaatctc cagttgacca ggaccccgac tgccttttct    1320
   gtgaagaccc ctgtgactag ctcagtcaca ccgtcagttt cccaaatttg acaggccacc     1380
   ttcaaacatg ctgctatgca gtttctgcat catagaaaat aaggaaccaa aggaagaaat     1440
   tcatgtcatg gtgcaatgca cattttatct atttatttag ttccattcac catgaaggaa     1500
   gaggcactga gatccatcaa tcaattggat tatatactga tcagtagctg tgttcaattg     1560
   caggaatgtg tatatagatt attcctgagt ggagccgaag taacagctgt ttgtaactat     1620
   cggcaatacc aaattcatct cccttccaat aatgcatctt gagaacacat aggtaaattt     1680
   gaactcagga aagtcttact agaaatcagt ggaagggaca aatagtcaca aaattttacc     1740
   aaaacattag aaacaaaaaa taaggagagc caagtcagga ataaaagtga ctctgtatgc     1800
   taacgccaca ttagaacttg gttctctcac caagctgtaa tgtgattttt ttttctactc     1860
   tgaattggaa atatgtatga atatacagag aagtgcttac aactaatttt tatttacttg     1920
   tcacattttg gcaataaatc cctcttattt ctaaattcta acttgtttat ttcaaaactt     1980
   tatataatca ctgttcaaaa ggaaatattt tcacctacca gagtgcttaa acactggcac     2040
   cagccaaaga atgtggttgt agagacccag aagtcttcaa gaacagccga caaaaacatt     2100
   cgagttgacc ccaccaagtt gttgccacag ataatttaga tatttacctg caagaaggaa     2160
   taaagcagat gcaaccaatt cattcagtcc acgagcatga tgtgagcact gctttgtgct     2220
   agacattggg cttagcattg aaactataaa gaggaatcag acgcagcaag tgcttctgtg     2280
   ttctggtagc aactcaacac tatctgtgga gagtaaactg aagatgtgca ggccaacatt     2340
   ctggaaatcc tatgtcaatg ggtttggttt ggaacctgga cttctgcatt tttaaaagtt     2400
   acccagagat gcttctaaag atgagccata gtctagaaga ttgtcaacca caggagttca     2460
   ttgagtggga cagctagaca catacattgg cagctacaat agtatcatga attgcaatga     2520
   tgtagtgggg tataaaagga aagcgatgga tattgccgga tgggcatggc cagtgatgtt     2580
   tcacgtcatt gaggtgacag ctctgctgga ctttgaatta catatggagg ctctccagga     2640
   agacgaagaa gagaaggaca ttctaggcaa aaagaagact aggcacaagg cacacttatg     2700
   tttgtctgtt agcttttagt tgaaaaagca aaatacatga tgcaaagaaa cctctccacg     2760
   ctgtgatttt taaaactaca tactttttgc aactttatgg ttatgagtat tgtagagaac     2820
   aggagatagg tcttagatga tttttatgtt gttgtcagac tctagcaagg tactagaaac     2880
   ctagcaggca ttaataattg ttgaggcaat gactctgagg ctatatctgg gccttgtcat     2940
   tatttatcat ttatatttgt atttttttct gaaatttgag ggccaagaaa acattgactt    3000
   tgactgagga ggtcacatct gtgccatctc tgcaaatcaa tcagcaccac tgaaataact     3060
   acttagcatt ctgctgagct ttccctgctc agtagagaca aatatactca tcccccacct     3120
   cagtgagctt gtttaggcaa ccaggattag agctgctcag gttcccaacg tctcctgcca     3180
   catcgggttc tcaaaatgga aagaatggtt tatgccaaat cacttttcct gtctgaagga     3240
   ccactgaatg gttttgtttt tccatatttt gcataggacg ccctaaagac taggtgactt 3300
   ggcaaacaca caagtgttag tataattctt tgcttctgct tctttttgaa aatcatgttt 3360
   agatttgatt ttaagtcaga aattcactga atgtcaggta atcattatgg agggagattt 3420
   gtgtgtcaac caaagtaatt gtcccatggc cccagggtat ttctgttgtt tccctgaaat 3480
   tctgcttttt tagtcagcta gattgaaaac tctgaacagt agatgtttat atggcaaaat 3540
   gcaagacaat ctacaaggga gattttaagg attttgagat gaaaaaacag atgctactca 3600
   ggggctttat gaaccatcca tcaattctga agttctgact ctcccattac cctttccctg 3660
   gtgtggtcag aactccaggt cactggaagt tagtggaatc atgtagttga attctttact 3720
   tcaagacatt gtattctctc cagctatcaa aacattaatg atcttttatg tctttttttt 3780
   gttattgtta tactttaagt tctggggtac atgtgcggaa catgtaggtt tgttacatag 3840
   gtatacatgt gccatggtgg tttgctgcac tcatcaacct gtcatctaca ttcttttatg 3900
   tctgtctttc aaagcaacac tctgttcttc tgagtagtga aatcaggtca actttaccac 3960
   cagcctccat ttttaatatg cttcaccatc atccagcacc tacttaagat ttatctaggg 4020
   ctctgtggtg atgttaggac ccataaaaga aatttatgcc ttccatatgt ttggttacag 4080
   atgggaaatg ggaatgttga aggacatgaa agaaaggatg tttacacatt aagcatcagt 4140
   tctgaagcta gattgtctga gtttgaatct tagctcttcc ctttattagc tctgtgacct 4200
   cgagctagtt acttaaatgc tctgatcctc tatttcctga tcagtgaaac ctccctattc 4260
   aaatgtgtga gagtttaata aattaggaca cttaaaaatg ttggagcagt gcatagcatg 4320
   tagtgttcag tacatgttaa atgttgtttt ttattatgta caaacatgag tgggcacaga 4380
   attttaaatc atctcaactt ttgagaaatt ttgagttatc aacaccgttc ccacaagaca 4440
   gtggcaaaat tattggtgag aattaaacag ctgtttctca gaggaagcaa tggaggcttg 4500
   ctgggataaa ggcatttact gagaggctgt tacctagtga gagtgatgaa ttaattaaaa 4560
   tagtcgaatc cctttctgac tgtctctgaa agcttccgct tttatctttg aagagcagaa 4620
   ttgtcactcc aaggacattt attaataaaa agaacaactg tccagtgcaa tgaaggcaaa 4680
   gtcataggtc tcccaagtct taccccattc ctgtgaaata tcaagttctt ggcttttctc 4740
   tgtcatgtag cctcaacttt ctctgaccgg gtgcatttct ttctctggtt tctaaattgc 4800
   cagtggcaaa tttggatcac ttacttaata tctgttaaat tttgtgaccc aacaaagtct 4860
   tttagcactg tggtgtcaaa aagaaaaaca cctcccaggc atatacattt tatagattcc 4920
   tggagaatgt tgctctccag ctccatcccc acccaatgaa atatgatcca gagagtcttg 4980
   caaagagaca agcctcattt tccacaatta gctctaaagt gcctccagga aatgattttc 5040
   tcagctcatc tctctgtatt ccctgttttg gatcacaggg caatctgttt aaatgactaa 5100
   ttacagaaat cattaaaggc accaagcaaa tgtcatctct gaatacacac atcccaagct 5160
   ttacaaatcc tgcctggctt gacagtgatg aggccactta acagtccagc gcaggcggat 5220
   gttaaaaaaa ataaaaaggt gaccatctgc ggtttagttt tttaactttc tgatttcaca 5280
   cttaacgtct gtcattctgt tactgggcac ctgtttaaat tctattttaa aatgttaatg 5340
   tgtgttgttt aaaataaaat caagaaagag aga 5373
<210> 18
   <211> 1906
   <212> DNA
   <213> Artificial Sequence
<220>
   <223> Synthetic
<400> 18
   atgtgcgcgc acgcgcgcgc gcacacacac acacacacac acacacacac acacacacac      60
   acacttatta caattgtacc tttgagtctc ccagaaaagc agtttctgtg agtgatagaa     120
   tgagtctagg tgattttcca aggagactgt taattttcac agatttaccc acaacactga     180
   tataggaggc ctcattaact caagtactgg aagaattttt ctaatttctt aggaagttgt     240
   gtgaatataa taatatcagt gcttctttac ttccaaaact ggacagcgca tcaaacatca     300
   gaaacaacag tatcagctcc tgtcccaact accatggagt ttcttgagag aacttatctt     360
   gtgaatgatc aagccaccaa gatgtacgcc ttccctctag acagagaact tcgacagaag     420
   cctgtgaata aagatcagtg tcctggagac aggccagagc atccagaggc aggaggcatc     480
   tatcactgcc acaacagcgc caaggccaca gggaacaggt cgagcaagca agcgcatgcc     540
   aagtggagac tctgtgctgc ttcagcaata tgcttcatct ttatggtggc agaggtggtg     600
   ggtggacacg ttgctgggag tctggctatc ctcactgatg cggctcatct cttaattgac     660
   ctgactagtt tcctgctcag tctcttttct ttgtggttgt catcgaggcc cccttccaag     720
   cggctgacat ttgggtggta ttgagcagag atcctcggtg ccctgctgtc tgtcctttgc     780
   atctgggtgg tgactggtgt gctgctgtac cttgcctgtg agcgcctttt gtatcctgat     840
   taccagatcc aagcaggtat catgatcact gtttcaggct gtgcagtggc agccaacatt     900
   gtactaacta tgattttgca ccaacggaac tttggctaca accacaagga tgtacaagct     960
   aatgccagtg tccgagcagc ctttgtgcat gccctggggg atgtatttca gagcatcagt    1020
   gtgctaatta gtgctctcat tatctacttt aagcctgact acaaaattgc tgatccagtg    1080
   tgcacattta tcttttccat cctggttttg gccagcaccg tcatgatctt aaaagacttc    1140
   tccatcttac tcatggaagg tgttccaaag ggcctgagtt acaacagtgt gaaagagatc    1200
   atcctcgcag ttgatggcgt gatctccgtg cacagtctac acatctggtc actgactgtg    1260
   aaccaagtga ttctctctgt tcatgttgct acagctgcca gccaggacag ccagtctgtg    1320
   cggacaggaa ttgctcaagc cctcagcagc tttgatcttc actctcttac cattcagata 1380
   gaatctgcag cagaccagga ccccagctgc cttctctgcg aagaccctca ggactagctc 1440
   ggtcacactg tcagcttcct gtgtttccta ggccatgata agatgcagca aagtttctgc 1500
   aatgcacaat gaggcagccg tcggaataga tttgagaaag tcatgatgat gcaatgtgca 1560
   cactcttcct ttgtatttat ctctatccac catgaacgag gatgcatggg atttgtcggc 1620
   ttcttggatt atacactaat cagtagttgt gctcaattgt agtatatata gattattcct 1680
   aactggagct gaaataacag atgtttgcaa tcataggtaa tgaatgattc acttgcctac 1740
   aatagtgggt atagttttac tcggaaatgc ctttctagga atccacagca tgagaaacaa 1800
   acatttgaag agaatttgag gccttagaat ttgattctgc caccatactc aatgagatct 1860
   ttattttttg tgaaacagta aaacttccct cttttgacct tgcatg 1906
<210> 19
   <211> 5373
   <212> DNA
   <213> Homo sapiens
<400> 19
   agcagttttt gtaggtgaaa acaatgaagc caggtaatat tgcaaggagg ctgtaatttt      60
   agcagaccta ccaacaacac tgatgtagga agctcattat tttaatttct ggagcctttt     120
   aatttttctt ttagaaagtg tataaataat tgcagtgctg ctttgcttcc aaaactgggc      180
   agtgagttca acaacaacga caacaacagc cgcagctcat cctggccgtc atggagtttc     240
   ttgaaagaac gtatcttgtg aatgataaag ctgccaagat gtatgctttc acactagaaa     300
   gtgtggaact ccaacagaaa ccggtgaata aagatcagtg tcccagagag agaccagagg     360
   agctggagtc aggaggcatg taccactgcc acagtggctc caagcccaca gaaaaggggg     420
   cgaatgagta cgcctatgcc aagtggaaac tctgttctgc ttcagcaata tgcttcattt     480
   tcatgattgc agaggtcgtg ggtgggcaca ttgctgggag tcttgctgtt gtcacagatg     540
   ctgcccacct cttaattgac ctgaccagtt tcctgctcag tctcttctcc ctgtggttgt     600
   catcgaagcc tccctctaag cggctgacat ttggatggca ctgagcagag atccttggtg     660
   ccctgctctc catcctgtgc atctgggtgg tgactggcgt gctagtgtac ctggcatgtg     720
   agcgcctgct gtatcctgat taccagatcc aggcgactgt gatgatcatc gtttccagct     780
   gcgcagtggc ggccaacatt gtactaactg tggttttgca ccagagatgc cttggccaca     840
   atcacaagga agtacaagcc aatgccagcg tcagagctgc ttttgtgcat gcccttggag     900
   atctatttca gagtatcagt gtgctaatta gtgcacttat tatctacttt aagccagagt     960
   ataaaatagc cgacccaatc tgcacattca tcttttccat cctggtcttg gccagcacca    1020
   tcactatctt aaaggacttc tccatcttac tcatggaagg tgtgccaaag agcctgaatt    1080
   acagtggtgt gaaagagctt attttagcag tcgacggggt gctgtctgtg cacagcctgc 1140
   acatctggtc tctaacaatg aatcaagtaa ttctctcagc tcatgttgct acagcagcca 1200
   gccgggacag ccaagtggtt cggagagaaa ttgctaaagc ccttagcaaa agctttacga 1260
   tgcactcact caccattcag atggaatctc cagttgacca ggaccccgac tgccttttct 1320
   gtgaagaccc ctgtgactag ctcagtcaca ccgtcagttt cccaaatttg acaggccacc 1380
   ttcaaacatg ctgctatgca gtttctgcat catagaaaat aaggaaccaa aggaagaaat 1440
   tcatgtcatg gtgcaatgca cattttatct atttatttag ttccattcac catgaaggaa 1500
   gaggcactga gatccatcaa tcaattggat tatatactga tcagtagctg tgttcaattg 1560
   caggaatgtg tatatagatt attcctgagt ggagccgaag taacagctgt ttgtaactat 1620
   cggcaatacc aaattcatct cccttccaat aatgcatctt gagaacacat aggtaaattt 1680
   gaactcagga aagtcttact agaaatcagt ggaagggaca aatagtcaca aaattttacc 1740
   aaaacattag aaacaaaaaa taaggagagc caagtcagga ataaaagtga ctctgtatgc 1800
   taacgccaca ttagaacttg gttctctcac caagctgtaa tgtgattttt ttttctactc 1860
   tgaattggaa atatgtatga atatacagag aagtgcttac aactaatttt tatttacttg 1920
   tcacattttg gcaataaatc cctcttattt ctaaattcta acttgtttat ttcaaaactt 1980
   tatataatca ctgttcaaaa ggaaatattt tcacctacca gagtgcttaa acactggcac 2040
   cagccaaaga atgtggttgt agagacccag aagtcttcaa gaacagccga caaaaacatt 2100
   cgagttgacc ccaccaagtt gttgccacag ataatttaga tatttacctg caagaaggaa 2160
   taaagcagat gcaaccaatt cattcagtcc acgagcatga tgtgagcact gctttgtgct 2220
   agacattggg cttagcattg aaactataaa gaggaatcag acgcagcaag tgcttctgtg 2280
   ttctggtagc aactcaacac tatctgtgga gagtaaactg aagatgtgca ggccaacatt 2340
   ctggaaatcc tatgtcaatg ggtttggttt ggaacctgga cttctgcatt tttaaaagtt 2400
   acccagagat gcttctaaag atgagccata gtctagaaga ttgtcaacca caggagttca 2460
   ttgagtggga cagctagaca catacattgg cagctacaat agtatcatga attgcaatga 2520
   tgtagtgggg tataaaagga aagcgatgga tattgccgga tgggcatggc cagtgatgtt 2580
   tcacgtcatt gaggtgacag ctctgctgga ctttgaatta catatggagg ctctccagga 2640
   agacgaagaa gagaaggaca ttctaggcaa aaagaagact aggcacaagg cacacttatg 2700
   tttgtctgtt agcttttagt tgaaaaagca aaatacatga tgcaaagaaa cctctccacg 2760
   ctgtgatttt taaaactaca tactttttgc aactttatgg ttatgagtat tgtagagaac 2820
   aggagatagg tcttagatga tttttatgtt gttgtcagac tctagcaagg tactagaaac 2880
   ctagcaggca ttaataattg ttgaggcaat gactctgagg ctatatctgg gccttgtcat 2940
   tatttatcat ttatatttgt atttttttct gaaatttgag ggccaagaaa acattgactt 3000
   tgactgagga ggtcacatct gtgccatctc tgcaaatcaa tcagcaccac tgaaataact 3060
   acttagcatt ctgctgagct ttccctgctc agtagagaca aatatactca tcccccacct 3120
   cagtgagctt gtttaggcaa ccaggattag agctgctcag gttcccaacg tctcctgcca 3180
   catcgggttc tcaaaatgga aagaatggtt tatgccaaat cacttttcct gtctgaagga 3240
   ccactgaatg gttttgtttt tccatatttt gcataggacg ccctaaagac taggtgactt 3300
   ggcaaacaca caagtgttag tataattctt tgcttctgct tctttttgaa aatcatgttt 3360
   agatttgatt ttaagtcaga aattcactga atgtcaggta atcattatgg agggagattt 3420
   gtgtgtcaac caaagtaatt gtcccatggc cccagggtat ttctgttgtt tccctgaaat 3480
   tctgcttttt tagtcagcta gattgaaaac tctgaacagt agatgtttat atggcaaaat 3540
   gcaagacaat ctacaaggga gattttaagg attttgagat gaaaaaacag atgctactca 3600
   ggggctttat gaaccatcca tcaattctga agttctgact ctcccattac cctttccctg 3660
   gtgtggtcag aactccaggt cactggaagt tagtggaatc atgtagttga attctttact 3720
   tcaagacatt gtattctctc cagctatcaa aacattaatg atcttttatg tctttttttt 3780
   gttattgtta tactttaagt tctggggtac atgtgcggaa catgtaggtt tgttacatag 3840
   gtatacatgt gccatggtgg tttgctgcac tcatcaacct gtcatctaca ttcttttatg 3900
   tctgtctttc aaagcaacac tctgttcttc tgagtagtga aatcaggtca actttaccac 3960
   cagcctccat ttttaatatg cttcaccatc atccagcacc tacttaagat ttatctaggg 4020
   ctctgtggtg atgttaggac ccataaaaga aatttatgcc ttccatatgt ttggttacag 4080
   atgggaaatg ggaatgttga aggacatgaa agaaaggatg tttacacatt aagcatcagt 4140
   tctgaagcta gattgtctga gtttgaatct tagctcttcc ctttattagc tctgtgacct 4200
   cgagctagtt acttaaatgc tctgatcctc tatttcctga tcagtgaaac ctccctattc 4260
   aaatgtgtga gagtttaata aattaggaca cttaaaaatg ttggagcagt gcatagcatg 4320
   tagtgttcag tacatgttaa atgttgtttt ttattatgta caaacatgag tgggcacaga 4380
   attttaaatc atctcaactt ttgagaaatt ttgagttatc aacaccgttc ccacaagaca 4440
   gtggcaaaat tattggtgag aattaaacag ctgtttctca gaggaagcaa tggaggcttg 4500
   ctgggataaa ggcatttact gagaggctgt tacctagtga gagtgatgaa ttaattaaaa 4560
   tagtcgaatc cctttctgac tgtctctgaa agcttccgct tttatctttg aagagcagaa 4620
   ttgtcactcc aaggacattt attaataaaa agaacaactg tccagtgcaa tgaaggcaaa 4680
   gtcataggtc tcccaagtct taccccattc ctgtgaaata tcaagttctt ggcttttctc 4740
   tgtcatgtag cctcaacttt ctctgaccgg gtgcatttct ttctctggtt tctaaattgc 4800
   cagtggcaaa tttggatcac ttacttaata tctgttaaat tttgtgaccc aacaaagtct 4860
   tttagcactg tggtgtcaaa aagaaaaaca cctcccaggc atatacattt tatagattcc 4920
   tggagaatgt tgctctccag ctccatcccc acccaatgaa atatgatcca gagagtcttg 4980
   caaagagaca agcctcattt tccacaatta gctctaaagt gcctccagga aatgattttc 5040
   tcagctcatc tctctgtatt ccctgttttg gatcacaggg caatctgttt aaatgactaa 5100
   ttacagaaat cattaaaggc accaagcaaa tgtcatctct gaatacacac atcccaagct 5160
   ttacaaatcc tgcctggctt gacagtgatg aggccactta acagtccagc gcaggcggat 5220
   gttaaaaaaa ataaaaaggt gaccatctgc ggtttagttt tttaactttc tgatttcaca 5280
   cttaacgtct gtcattctgt tactgggcac ctgtttaaat tctattttaa aatgttaatg 5340
   tgtgttgttt aaaataaaat caagaaagag aga 5373
<210> 20
   <211> 1104
   <212> DNA
   <213> Mus musculus
<400> 20
   atggagtttc ttgagagaac ttatcttgtg aatgatcaag ccaccaagat gtacgccttc      60
   cctctagaca gagaacttcg acagaagcct gtgaataaag atcagtgtcc tggagacagg     120
   ccagagcatc cagaggcagg aggcatctat cactgccaca acagcgccaa ggccacaggg     180
   aacaggtcga gcaagcaagc gcatgccaag tggagactct gtgctgcttc agcaatatgc     240
   ttcatcttta tggtggcaga ggtggtgggt ggacacgttg ctgggagtct ggctatcctc     300
   actgatgcgg ctcatctctt aattgacctg actagtttcc tgctcagtct cttttctttg     360
   tggttgtcat cgaggccccc ttccaagcgg ctgacatttg ggtggtatcg agcagagatc     420
   ctcggtgccc tgctgtctgt cctttgcatc tgggtggtga ctggtgtgct gctgtacctt     480
   gcctgtgagc gccttttgta tcctgattac cagatccaag caggtatcat gatcactgtt     540
   tcaggctgtg cagtggcagc caacattgta ctaactatga ttttgcacca acggaacttt     600
   ggctacaacc acaaggatgt acaagctaat gccagtgtcc gagcagcctt tgtgcatgcc     660
   ctgggggatg tatttcagag catcagtgtg ctaattagtg ctctcattat ctactttaag     720
   cctgactaca aaattgctga tccagtgtgc acatttatct tttccatcct ggttttggcc     780
   agcaccgtca tgatcttaaa agacttctcc atcttactca tggaaggtgt tccaaagggc     840
   ctgagttaca acagtgtgaa agagatcatc ctcgcagttg atggcgtgat ctccgtgcac     900
   agtctacaca tctggtcact gactgtgaac caagtgattc tctctgttca tgttgctaca     960
   gctgccagcc aggacagcca gtctgtgcgg acaggaattg ctcaagccct cagcagcttt    1020
   gatcttcact ctcttaccat tcagatagaa tctgcagcag accaggaccc cagctgcctt    1080
   ctctgcgaag accctcagga ctag                                           1104
<210> 21
   <211> 1110
   <212> DNA
   <213> Homo sapiens
<400> 21
   atggagtttc ttgaaagaac gtatcttgtg aatgataaag ctgccaagat gtatgctttc 60
   acactagaaa gtgtggaact ccaacagaaa ccggtgaata aagatcagtg tcccagagag 120
   agaccagagg agctggagtc aggaggcatg taccactgcc acagtggctc caagcccaca 180
   gaaaaggggg cgaatgagta cgcctatgcc aagtggaaac tctgttctgc ttcagcaata 240
   tgcttcattt tcatgattgc agaggtcgtg ggtgggcaca ttgctgggag tcttgctgtt 300
   gtcacagatg ctgcccacct cttaattgac ctgaccagtt tcctgctcag tctcttctcc 360
   ctgtggttgt catcgaagcc tccctctaag cggctgacat ttggatggca ccgagcagag 420
   atccttggtg ccctgctctc catcctgtgc atctgggtgg tgactggcgt gctagtgtac 480
   ctggcatgtg agcgcctgct gtatcctgat taccagatcc aggcgactgt gatgatcatc 540
   gtttccagct gcgcagtggc ggccaacatt gtactaactg tggttttgca ccagagatgc 600
   cttggccaca atcacaagga agtacaagcc aatgccagcg tcagagctgc ttttgtgcat 660
   gcccttggag atctatttca gagtatcagt gtgctaatta gtgcacttat tatctacttt 720
   aagccagagt ataaaatagc cgacccaatc tgcacattca tcttttccat cctggtcttg 780
   gccagcacca tcactatctt aaaggacttc tccatcttac tcatggaagg tgtgccaaag 840
   agcctgaatt acagtggtgt gaaagagctt attttagcag tcgacggggt gctgtctgtg 900
   cacagcctgc acatctggtc tctaacaatg aatcaagtaa ttctctcagc tcatgttgct 960
   acagcagcca gccgggacag ccaagtggtt cggagagaaa ttgctaaagc ccttagcaaa 1020
   agctttacga tgcactcact caccattcag atggaatctc cagttgacca ggaccccgac 1080
   tgccttttct gtgaagaccc ctgtgactag 1110
<210> 22
   <211> 1104
   <212> DNA
   <213> Artificial Sequence
<220>
   <223> Synthetic
<400> 22
   atggagtttc ttgagagaac ttatcttgtg aatgatcaag ccaccaagat gtacgccttc 60
   cctctagaca gagaacttcg acagaagcct gtgaataaag atcagtgtcc tggagacagg 120
   ccagagcatc cagaggcagg aggcatctat cactgccaca acagcgccaa ggccacaggg 180
   aacaggtcga gcaagcaagc gcatgccaag tggagactct gtgctgcttc agcaatatgc 240
   ttcatcttta tggtggcaga ggtggtgggt ggacacgttg ctgggagtct ggctatcctc      300
   actgatgcgg ctcatctctt aattgacctg actagtttcc tgctcagtct cttttctttg      360
   tggttgtcat cgaggccccc ttccaagcgg ctgacatttg ggtggtattg agcagagatc      420
   ctcggtgccc tgctgtctgt cctttgcatc tgggtggtga ctggtgtgct gctgtacctt      480
   gcctgtgagc gccttttgta tcctgattac cagatccaag caggtatcat gatcactgtt      540
   tcaggctgtg cagtggcagc caacattgta ctaactatga ttttgcacca acggaacttt      600
   ggctacaacc acaaggatgt acaagctaat gccagtgtcc gagcagcctt tgtgcatgcc      660
   ctgggggatg tatttcagag catcagtgtg ctaattagtg ctctcattat ctactttaag      720
   cctgactaca aaattgctga tccagtgtgc acatttatct tttccatcct ggttttggcc      780
   agcaccgtca tgatcttaaa agacttctcc atcttactca tggaaggtgt tccaaagggc      840
   ctgagttaca acagtgtgaa agagatcatc ctcgcagttg atggcgtgat ctccgtgcac      900
   agtctacaca tctggtcact gactgtgaac caagtgattc tctctgttca tgttgctaca      960
   gctgccagcc aggacagcca gtctgtgcgg acaggaattg ctcaagccct cagcagcttt     1020
   gatcttcact ctcttaccat tcagatagaa tctgcagcag accaggaccc cagctgcctt     1080
   ctctgcgaag accctcagga ctag                                            1104
<210> 23
   <211> 1110
   <212> DNA
   <213> Homo sapiens
<400> 23
   atggagtttc ttgaaagaac gtatcttgtg aatgataaag ctgccaagat gtatgctttc      60
   acactagaaa gtgtggaact ccaacagaaa ccggtgaata aagatcagtg tcccagagag     120
   agaccagagg agctggagtc aggaggcatg taccactgcc acagtggctc caagcccaca     180
   gaaaaggggg cgaatgagta cgcctatgcc aagtggaaac tctgttctgc ttcagcaata     240
   tgcttcattt tcatgattgc agaggtcgtg ggtgggcaca ttgctgggag tcttgctgtt     300
   gtcacagatg ctgcccacct cttaattgac ctgaccagtt tcctgctcag tctcttctcc     360
   ctgtggttgt catcgaagcc tccctctaag cggctgacat ttggatggca ctgagcagag     420
   atccttggtg ccctgctctc catcctgtgc atctgggtgg tgactggcgt gctagtgtac     480
   ctggcatgtg agcgcctgct gtatcctgat taccagatcc aggcgactgt gatgatcatc     540
   gtttccagct gcgcagtggc ggccaacatt gtactaactg tggttttgca ccagagatgc     600
   cttggccaca atcacaagga agtacaagcc aatgccagcg tcagagctgc ttttgtgcat     660
   gcccttggag atctatttca gagtatcagt gtgctaatta gtgcacttat tatctacttt     720
   aagccagagt ataaaatagc cgacccaatc tgcacattca tcttttccat cctggtcttg     780
   gccagcacca tcactatctt aaaggacttc tccatcttac tcatggaagg tgtgccaaag     840
   agcctgaatt acagtggtgt gaaagagctt attttagcag tcgacggggt gctgtctgtg     900
   cacagcctgc acatctggtc tctaacaatg aatcaagtaa ttctctcagc tcatgttgct     960
   acagcagcca gccgggacag ccaagtggtt cggagagaaa ttgctaaagc ccttagcaaa    1020
   agctttacga tgcactcact caccattcag atggaatctc cagttgacca ggaccccgac    1080
   tgccttttct gtgaagaccc ctgtgactag                                    1110
<210> 24
   <211> 29
   <212> DNA
   <213> Mus musculus
<400> 24
   cagctgacag caagattaat ggaagtacc 29
<210> 25
   <211> 26
   <212> DNA
   <213> Artificial Sequence
<220>
   <223> Synthetic
<400> 25
   tccaaaactg ggcagtgagt tcaaca 26
<210> 26
   <211> 19
   <212> DNA
   <213> Artificial Sequence
<220>
   <223> Synthetic
<400> 26
   aattgcagtg ctgctttgc 19
<210> 27
   <211> 19
   <212> DNA
   <213> Artificial Sequence
<220>
   <223> Synthetic
<400> 27
   agctgcggct gttgttgtc 19